# EUROPEAN PATENT SPECIFICATION

(11) **EP 1 633 702 B1**
(45) Date of publication and mention of the grant of the patent: **02.07.2008**
(21) Application number: 04734996.4
(22) Date of filing: 27.05.2004
(51) Int. Cl.: C07C 281/06, C07D 295/14, C07D 307/52, C07D 209/16, C07D 317/58, C07D 333/20, C07D 211/56, C07D 207/08, C07D 207/26, C07K 5/06, A61K 31/175, A61P 29/00, A61K 31/38, A61K 31/40, A61K 31/435

(54) **PROTEASE INHIBITORS**
PROTEASEHEMMER
INHIBITEURS DE PROTEASE

(30) Priority: 30.05.2003 DK 200300810; 30.05.2003 US 474424 P; 19.09.2003 DK 200301365
(43) Date of publication of application: 15.03.2006
(73) Proprietor: Prozymex A/S, 2970 Hoersholm (DK)
(72) Inventor: BONDEBJERG, Jon, DK-2100 Copenhagen (DK); FUGLSANG, Henrik, DK-2800 Lyngby (DK); NAERUM, Lars, DK-2900 Hellerup (DK)
(74) Representative: Inspicos A/S
(86) International application number: PCT/DK2004/000371
(87) International publication number: WO 2004/106289

(56) References cited:
- EP-A- 0 076 557
- EP-A- 0 083 849
- EP-A- 0 496 393
- WO-A-02/20804
- WO-A-97/16433
- WO-A-98/04277
- WO-A-99/59570
- WO-A-03/022871
- DE-A- 2 715 803
- GB-A- 992 961
- US-A- 4 407 794
- US-A- 5 602 102
- FROCHOT C ET AL: "A SOLID-PHASE SYNTHESIS OF THREE AZA-, IMINOAZA- AND REDUCED AZA-PEPTIDES FROM THE SAME PRECURSOR" LETTERS IN PEPTIDE SCIENCE, ESCOM SCIENCE PUBLISHERS, NL, vol. 4, no. 4-6, 1997, pages 219-225, XP001037193 ISSN: 0929-5666
- HYUNSOO H ET AL: "Investigations of azapeptides as mimetics of Leu-enkephalin" BIOORGANIC & MEDICINAL CHEMISTRY LETTERS, OXFORD, GB, vol. 8, no. 1-6, 6 January 1998 (1998-01-06), pages 117-120, XP004136634 ISSN: 0960-894X
- GRAY, C. J. ET AL: "Synthesis of azaalanine peptides using the solid phase method" BIOMEDICAL PEPTIDES, PROTEINS & NUCLEIC ACIDS , 2(1), 13-18 CODEN: BPPAFS; ISSN: 1353-8616, 1996, XP008033349
- PAYNTER, H. ET AL: "Synthesis of deltorphin I analogs and preliminary characterization of .mu.-receptor binding" BIOCHEMICAL SOCIETY TRANSACTIONS , 21(3), 262S CODEN: BCSTB5; ISSN: 0300-5127, 1993, XP008033355
- DUTTA, ANAND S. ET AL: "Polypeptides. Part 15. Synthesis and biological activity of .alpha.-aza analogs of luliberin modified in positions 6 and 10" JOURNAL OF THE CHEMICAL SOCIETY, PERKIN TRANSACTIONS 1: ORGANIC AND BIO-ORGANIC CHEMISTRY (1972-1999) , (2), 379-88 CODEN: JCPRB4; ISSN: 0300-922X, 1979, XP001194638
- NIEDRICH, HARTMUT ET AL: "Hydrazine compounds as hetero-components in peptides. XIX. Eledoisin peptides containing the N2-methylcarbazoyl radical (azaalanine)" JOURNAL FUER PRAKTISCHE CHEMIE (LEIPZIG) , 316(5), 741-9 CODEN: JPCEAO; ISSN: 0021-8383, 1974, XP008033357
- NIEDRICH, HARTMUT ET AL: "Hydrazine compounds as hetero-components in peptides. XV. Synthesis of eledoisin octapeptides with the carbazyl residues azaglycine and .alpha.-azaasparagine instead of glycine and asparagine" JOURNAL FUER PRAKTISCHE CHEMIE (LEIPZIG) , 314(5-6), 759-68 CODEN: JPCEAO; ISSN: 0021-8383, 1972, XP008033358
- NEUMANN, U. ET AL: "Reaction of dipeptidylpeptidase IV with substrate-analogous azapeptides" JOURNAL OF ENZYME INHIBITION , 4(3), 213-26 CODEN: ENINEG; ISSN: 8755-5093, 1991, XP008033354
- DATABASE CHEMCATS Chemical Abstracts Service, Columbus, Ohio, US; XP002263984 & "INTERCHIM INTERMEDIATES" 9 July 2002 (2002-07-09), INTERCHIM , MONTLUCON, CEDEX, 03103, FRANCE
- TIEN, N. B. ET AL: "Tuberculostatic N-arylglycines and derivatives" JOURNAL OF ORGANIC CHEMISTRY, 23, 186-8, 1958, XP002263983
- MISRA, VINAY S. ET AL: "Possible antituberculous compounds. XV. N-2-Thiazolylglycines, 1-acyl-4-arylsemicarbazides, 1-acyl-4-arylthiosemicarbazides, and N1-(N-arylglycyl)-N2-(arylidane or alkylidene) hydrazines" J. INDIAN CHEM. SOC., 40(9), 799-802, 1963, XP008025483

## Description

### FIELD OF THE INVENTION

The present invention relates to novel protease inhibitors, more specifically to inhibitors of cysteine and/or serine proteases useful in the treatment/prevention of inflammation, diabetes and similar diseases in which proteases are involved, especially mast cell inflammatory mediated diseases. More specifically the invention relates to monoacyl semicarbazides capable of inhibiting dipeptidyl-peptidase I (DPP-I); also known as cathepsin C, an enzyme that cleaves a dipeptide from the N terminus of a polypeptide chain.

### BACKGROUND OF THE INVENTION

Dipeptidyl peptidase-I (DPP-I; EC 3.4.14.1) also known as cathepsin C is a lysosomal cysteine protease belonging to the papain family. The enzyme is constitutively expressed in many tissues with highest levels in lung, kidney, liver and spleen. The cDNAs encoding rat, human and murine DPP-I have been cloned and sequenced and showed that the enzyme is highly conserved. DPP-I is synthesized as an inactive precursor (Zymogen), and is activated by a non-autocatalytic excision of an internal activation peptide within the N-terminal propeptide. Once activated, DPP-I catalyzes the removal of dipeptides from the N-terminal end of polypeptide substrates with broad specificity. The pH optimum lies in the region of 5-7 using humanè111.3 DPP-I. Furthermore, DPP-I is oligomeric with little amino acid sequence homology compared to the exopeptidases cathepsin B, H, L, O and S which in addition are monomeric. Recent data suggests that, beside of being an important enzyme in lysosomal protein degradation, DPP-I also functions as a key enzyme in the activation of granule serine peptidases in cytotoxic T lymphocytes and natural killer cells (granzymes A and B), mast cells (chymase and tryptase), and neutrophils (cathepsin G and elastase).

Mast cells are found in many tissues, but are present in greater numbers along the epithelial linings of the body, such as the skin, respiratory tract and gastrointestinal tract. Mast cells are also located in the perivascular tissue surrounding small blood vessels. In humans, two types of mast cells have been identified. The T-type, which expresses only tryptase, and the MC-type, which expresses both tryptase and chymase. In humans, the T-type mast cells are located primarily in alveolar tissue and intestinal mucose while the TC-type cells predominate in skin and conjuctiva. Mast cells can release a range of potent inflammatory mediators including cytokines, leukotrienes, prostaglandins, histamine and proteoglycans, but among the most abundant products of mast cell activation are the serine proteases of the chymotrypsin family; tryptase and chymase. These proteases are situated in the mast cell lysosomes as fully active enzymes. The exact site of tryptase and chymase activation from zymogen precursors is not known, but the Golgi apparatus might play a role in that regard. DPP-I, which is particular abundant in mast cells, seems to be the key enzyme responsible for activation of chymase and tryptase. Moreover, tryptase and chymase are emerging as important mediators of allergic diseases. After secretion from activated mast cells, there is evidence that these proteases are heavily involved in processes of inflammation, tissue remodelling, bronchoconstriction and mucus secretion, which have made these proteases attractive for therapeutic intervention. Mast cells seem also to play a role in angiogenesis since these cells accumulate in many angiogenesis-dependent situations. Moreover, several mast cell mediators (e.g. histamine, chymase, VEGF and bFGF) are found to be angiogenic and regulate endothelial cell proliferation and function.

Neutrophils cause considerable damage in a number of pathological conditions. When activated, neutrophils secrete destructive granular enzymes including elastase and cathepsin G and undergo oxidative bursts to release reactive oxygen intermediates. Numerous studies have been conducted on each of these activating agents in isolation. Pulmonary emphysema, cystic fibrosis and rheumatoid arthritis are just some examples of pathological conditions associated with the potent enzymes elastase and cathepsin G.

The strong evidence associating tryptase and chymase with a number of mast cell mediated allergic, immunological and inflammatory diseases, and the fact that cathepsin G and elastase also seem to play important roles in these types of diseases points out DPP-I as an attractive target enzyme for therapeutic intervention against the above mentioned diseases, due to its central role in activating these proteases.

US 5,602,102 to The University of Texas discloses inhibitors of dipeptidyl peptidase I.

WO03/022871A2 to Probiodrug discloses inhibitors of dipeptidyl peptidase I.

### SUMMARY OF THE INVENTION

The present invention relates to compounds of the general formula (I) 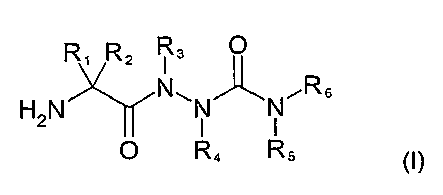 wherein R₁, R₂, R₃, R₄, R₅ and R₆ are as defined in the detailed part of this description, or a pharmaceutically acceptable salt or ester thereof.

It is contemplated that the compounds of the invention are useful for the treatment of inflammation or type 2 diabetes, particularly for treatment of prevention of mast cell inflammatory mediated diseases such as asthma, severe influenza, respiratory syncytial virus infection, CD8 T cell inhibition, inflammatory bowel diseases, psoriasis, atopic dermatitis, Papillon Lefevre syndrome, Haim Munk syndrome, gum disease, periodontitis, rheumatoid arthritis, Huntington's disease, Chagas' disease, Alzheimer's disease or sepsis. The compounds of the present invention are especially applicable in target cell apoptosis.

In accordance with the present invention there is provided compounds, which are contemplated as being useful as in vitro and in vivo diagnostic tools.

It is an object of the invention to provide novel compounds having pharmacological activity as inhibitors of proteases, particularly serine and/or cysteine proteases, more particularly cysteine proteases, even more particularly cysteine proteases of the papain superfamily, yet more particularly dipeptidyl-peptidase I.

Further objects will become apparent from the following description.

### DETAILED DESCRIPTION OF THE INVENTION

### Definitions

The term "DPP-I" or "DPPI" as used herein is intended to mean dipeptidyl-peptidase I (EC 3.4.14.1) also known as cathepsin C, cathepsin J, dipeptidyl aminopeptidase I and dipeptidyl transferase. DPPI cleaves a dipeptide Xaa-Xbb from the N terminus of a polypeptide chain Xaa-Xbb-Xcc-[Xxx]ₙ, except when Xaa is Arg or Lys, or when Xbb or Xcc is Pro.

The term "treatment" is defined as the management and care of a patient for the purpose of combating the disease, condition, or disorder and includes the administration of a compound of the present invention to prevent the onset of the symptoms or the complications, or alleviating the symptoms or the complications, or eliminating the disease, condition, or disorder.

As used herein, alone or in combination, the term "C₁₋₆ alkyl" denotes a straight or branched, saturated hydrocarbon chain having from one to six carbon atoms. C₁₋₆alkyl groups include, but are not limited to, methyl, ethyl, n-propyl, iso-propyl, n-butyl, iso-butyl, sec-butyl, tert-butyl, n-pentyl, iso-pentyl, 2-methylbutyl, 3-methylbutyl, n-hexyl, iso-hexyl, 4-methylpentyl, neopentyl, 2,2-dimethylpropyl and the like.

As used herein, alone or in combination, the term "C₂₋₆ alkenyl" denotes a straight or branched, unsaturated hydrocarbon chain having from two to six carbon atoms and at least one double bond. C₂₋₆ alkenyl groups include, but are not limited to, vinyl, 1-propenyl, allyl, iso-propenyl, n-butenyl, n-pentenyl, n-hexenyl and the like.

As used herein, alone or in combination, the term "C₂₋₆ alkynyl" denotes a straight or branched, unsaturated hydrocarbon chain having from two to six carbon atoms and at least one triple bond. C₂₋₆alkynyl groups include, but are not limited to, -C≡H,-C≡CCH₃, -CH₂C≡CH, -CH₂-CH₂C≡CH, -CH(CH₃)C≡CH and the like.

The term "C₁₋₆ alkoxy" in the present context designates a group O-C₁₋₆ alkyl used alone or in combination, wherein C₁₋₆ alkyl is as defined above. Examples of straight alkoxy groups are methoxy, ethoxy, propoxy, butoxy, pentoxy and hexoxy. Examples of branched alkoxy are iso-propoxy, sec-butoxy, tert-butoxy, iso-pentoxy and iso-hexoxy. Examples of cyclic alkoxy are cyclopropyloxy, cyclobutyloxy, cyclopentyloxy and cyclohexyloxy.

The term " C₁₋₆alkylthio" in the present context designates a group -S-C₁₋₆ alkyl wherein C₁₋₆ alkyl is as defined above. Representative examples include, but are not limited to, methylthio, ethylthio, n-propylthio, isopropylthio, butylthio, isobutylthio, sec-butylthio, tert-butylthio, n-pentylthio, isopentylthio, neopentylthio, tert-pentylthio, n-hexylthio, isohexylthio and the like.

The term "C₁₋₆ alkylcarbonyl" in the present context designates a group -(CO)-C₁₋₆ alkyl wherein C₁₋₆ alkyl is as defined above. Representative examples include, but are not limited to, methylcarbonyl, ethylcarbonyl, n-propylcarbonyl, isopropylcarbonyl, butylcarbonyl, isobutylcarbonyl, sec-butylcarbonyl, tert-butylcarbonyl, n-pentylcarbonyl, isopentylcarbonyl, neopentylcarbonyl, tert-pentylcarbonyl, n-hexylcarbonyl, isohexylcarbonyl and the like.

The term "C₁₋₆ N-alkylamide" in the present context designates a group -(CO)NH-C₁₋₆ alkyl, wherein C₁₋₆ alkyl is as defined above. Representative examples include, but are not limited to, N-methylamide, N-ethylamide, N-propylamide, N-butylamide, N-pentylamide and N-hexylamide.

The term "dialkylamino C₁₋₆ alkyl" as used herein designates a group di-C₁₋₄alkyl-N-C₁₋₆alkyl, wherein C₁₋₆ alkyl is as defined above. Representative examples include, but are not limited to, dimethylaminomethyl.

The term "C₃₋₁₀ cycloalkyl" as used herein denotes a radical of one or more saturated mono-, bi-, tri- or spirocyclic hydrocarbon having from three to ten carbon atoms. Examples include, but are not limited to, cyclopropyl, cyclobutyl, cyclopentyl, cyclohexyl, cycloheptyl, cyclooctyl, cyclononyl, cyclodecyl, bicyclo[3.2.1]octyl, spiro[4.5]decyl, norpinyl, norbonyl, norcaryl, adamantyl and the like.

The term "C₅₋₁₀ cycloalkenyl" as used herein denotes a radical of one or more saturated cyclic hydrocarbon having from five to ten carbon atoms and at least one double bond. Examples include, but are not limited to, cyclopentenyl and cyclohexenyl and the like.

The term "C₃₋₇ heterocycloalkyl" as used herein denotes a radical of a totally saturated heterocycle like a cyclic hydrocarbon containing one or more heteroatoms selected from nitrogen, oxygen and sulphur independently in the cycle. Examples of heterocycles include, but are not limited to, pyrrolidine (1-pyrrolidine, 2-pyrrolidine, 3-pyrrolidine, 4-pyrrolidine, 5-pyrrolidine), pyrazolidine (1-pyrazolidine, 2-pyrazolidine, 3-pyrazolidine, 4-pyrazolidine, 5-pyrazolidine), imidazolidine (1-imidazolidine, 2-imidazolidine, 3-imidazolidine, 4-imidazolidine, 5-imidazolidine), thiazolidine (2-thiazolidine, 3-thiazolidine, 4-thiazolidine, 5-thiazolidine), piperidine (1-piperidine, 2-piperidine, 3-piperidine, 4-piperidine, 5-piperidine, 6-piperidine), piperazine (1-piperazine, 2-piperazine, 3-piperazine, 4-piperazine, 5-piperazine, 6-piperazine), morpholine (2-morpholine, 3-morpholine, 4-morpholine, 5-morpholine, 6-morpholine), thiomorpholine (2-thiomorpholine, 3-thiomorpholine, 4-thiomorpholine, 5-thiomorpholine, 6- thiomorpholine), 1,2-oxathiolane (3-(1,2-oxathiolane), 4-(1,2-oxathiolane), 5-(1,2-oxathiolane)), 1,3-dioxolane (2-(1,3-dioxolane), 3-(1,3-dioxolane), 4-(1,3-dioxolane)), tetrahydropyrane (2- tetrahydropyrane, 3- tetrahydropyrane, 4-tetrahydropyrane, 5- tetrahydropyrane, 6- tetrahydropyrane), hexahydropyradizine, (1-(hexahydropyradizine), 2-(hexahydropyradizine), 3-(hexahydropyradizine), 4-(hexahydropyradizine), 5-(hexahydropyradizine), 6-(hexahydropyradizine)).

The term "aryl" as used herein is intended to include carbocyclic aromatic ring systems. Aryl is also intended to include the partially hydrogenated derivatives of the carbocyclic systems enumerated below.

The term "heteroaryl" as used herein includes heterocyclic aromatic ring systems containing one or more heteroatoms selected from nitrogen, oxygen and sulphur such as furyl, thienyl, pyrrolyl, and is also intended to include the partially hydrogenated derivatives of the heterocyclic systems enumerated below.
Examples of "aryl" and "heteroaryl" includes, but are not limited to, phenyl, biphenyl, indenyl, naphthyl (1-naphthyl, 2-naphthyl), N-hydroxytetrazolyl, N-hydroxytriazolyl, N-hydroxyimidazolyl, anthracenyl (1-anthracenyl, 2-anthracenyl, 3-anthracenyl), phenanthrenyl, fluorenyl, pentalenyl, azulenyl, biphenylenyl, thiophenyl (1-thienyl, 2-thienyl), furyl (1-furyl, 2-furyl), furanyl, thiophenyl, isoxazolyl, isothiazolyl, 1,2,3-triazolyl, 1,2,4-triazolyl, pyranyl, pyridazinyl, pyrazinyl, 1,2,3-triazinyl, 1,2,4-triazinyl, 1,3,5-triazinyl, 1,2,3-oxadiazolyl, 1,2,4-oxadiazolyl, 1,2,5-oxadiazolyl, 1,3,4-oxadiazolyl, 1,2,3-thiadiazolyl, 1,2,4-thiadiazolyl, 1,2,5-thiadiazolyl, 1 ,3,4-thiadiazolyl, tetrazolyl, thiadiazinyl, indolyl, isoindolyl, benzofuranyl, benzothiophenyl (thianaphthenyl), indolyl, oxadiazolyl, isoxazolyl, quinazolinyl, fluorenyl, xanthenyl, isoindanyl, benzhydryl, acridinyl, benzisoxazolyl, purinyl, quinazolinyl, quinolizinyl, quinolinyl, isoquinolinyl, quinoxalinyl, naphthyridinyl, phteridinyl, azepinyl, diazepinyl, pyrrolyl (2-pyrrolyl), pyrazolyl (3-pyrazolyl), imidazolyl (1-imidazolyl, 2-imidazolyl, 4-imidazolyl, 5-imidazolyl), triazolyl (1,2,3-triazol-1-yl, 1,2,3-triazol-2-yl, 1,2,3-triazol-4-yl, 1,2,4-triazol-3-yl), oxazolyl (2-oxazolyl, 4-oxazolyl, 5-oxazolyl), thiazolyl (2-thiazolyl, 4-thiazolyl, 5-thiazolyl), pyridyl (2-pyridyl, 3-pyridyl, 4-pyridyl), pyrimidinyl (2-pyrimidinyl, 4-pyrimidinyl, 5-pyrimidinyl, 6-pyrimidinyl), pyrazinyl, pyridazinyl (3-pyridazinyl, 4-pyridazinyl, 5-pyridazinyl), isoquinolyl (1-isoquinolyl, 3-isoquinolyl, 4-isoquinolyl, 5-isoquinolyl, 6-isoquinolyl, 7-isoquinolyl, 8-isoquinolyl), quinolyl (2-quinolyl, 3-quinolyl, 4-quinolyl, 5-quinolyl, 6-quinolyl, 7-quinolyl, 8-quinolyl), benzo[b]furanyl (2-benzo[b]furanyl, 3-benzo[b]furanyl, 4-benzo[b]furanyl, 5-benzo[b]furanyl, 6-benzo[b]furanyl, 7-benzo[b]furanyl), 2,3-dihydro-benzo[b]furanyl (2-(2,3-dihydrobenzo[b]furanyl), 3-(2,3-dihydro-benzo[b]furanyl), 4-(2,3-dihydro-benzo[b]furanyl), 5-(2,3-dihydro-benzo[b]furanyl), 6-(2,3-dihydro-benzo[b]furanyl), 7-(2,3-dihydrobenzo[b]furanyl)), benzo[b]thiophenyl (2-benzo[b]thiophenyl, 3-benzo[b]thiophenyl, 4-benzo[b]thiophenyl, 5-benzo[b]thiophenyl, 6-benzo[b]thiophenyl, 7-benzo[b]thiophenyl), 2,3-dihydro-benzo[b]thiophenyl (2-(2,3-dihydro-benzo[b]thiophenyl), 3-(2,3-dihydrobenzo[b]thiophenyl), 4-(2,3-dihydro-benzo[b]thiophenyl), 5-(2,3-dihydrobenzo[b]thiophenyl), 6-(2,3-dihydro-benzo[b]thiophenyl), 7-(2,3-dihydrobenzo[b]thiophenyl)), indolyl (1-indolyl, 2-indolyl, 3-indolyl, 4-indolyl, 5-indolyl, 6-indolyl, 7-indolyl), indazolyl (1-indazolyl, 2-indazolyl, 3-indazolyl, 4-indazolyl, 5-indazolyl, 6-indazolyl, 7-indazolyl), benzimidazolyl, (1-benzimidazolyl, 2-benzimidazolyl, 4-benzimidazolyl, 5-benzimidazolyl, 6-benzimidazolyl, 7-benzimidazolyl, 8-benzimidazolyl), benzoxazolyl (1-benzoxazolyl, 2-benzoxazolyl), benzothiazolyl (1-benzothiazolyl, 2-benzothiazolyl, 4-benzothiazolyl, 5-benzothiazolyl, 6-benzothiazolyl, 7-benzothiazolyl), carbazolyl (1-carbazolyl, 2-carbazolyl, 3-carbazolyl, 4-carbazolyl). Non-limiting examples of partially hydrogenated derivatives are 1,2,3,4-tetrahydronaphthyl, 1,4-dihydronaphthyl, pyrrolinyl, pyrazolinyl, indolinyl, oxazolidinyl, oxazolinyl, oxazepinyl and the like.

The term "aryl-C₁₋₅alkyl" as used herein refers to a C₁₋₅-alkyl group as defined above having one, two, three, four or five carbon atoms attached through an aryl group as defined above.

The term "heteroaryl-C₁₋₅alkyl" as used herein refers to a C₁₋₅-alkyl is as defined above having one, two, three, four or five carbon atoms attached through a heteroaryl group as defined above.

The term "C₁₋₆-alkylaryl" as used herein refers to an aryl group as defined above attached through a C₁₋₆alkyl group as defined above having one, two, three, four, five or six carbon atoms.

The term "C₁₋₆-alkylheteroaryl" as used herein refers to a heteroaryl group as defined above attached through a C₁₋₆alkyl group as defined above having one, two, three, four, five or six carbon atoms.

The term "aroyl" as used herein represents a group -(CO)-aryl wherein aryl is as defined above.

The term "C₃₋₇cyloalkyl-C₁₋₅alkyl" as used herein refers to a cycloalkyl group as defined above attached through an alkyl group as defined above having the indicated number of carbon atoms.

The term "C₃₋₇heterocycloalkyl-C₁₋₅alkyl" as used herein refers to a heterocycloalkyl group as defined above attached through an alkyl group as defined above having the indicated number of carbon atoms.

"Halogen" designates an atom selected from the group consisting of F, Cl, Br and I.

The terms "unsubstituted" or "substituted" as used herein means that the groups in question are optionally unsubstituted or substituted with one, two or three substituents independently of each other selected from C₁₋₆alkyl, C₁₋₆alkylthio, C₁₋₆alkylcarbonyl, C₁₋₆ -N-alkylamide, C₁₋₆alkoxy, dialkylamino-C₁₋₆alkyl, amide, hydroxy, carboxy, amino, halogen, trifluoromethyl, trifluoromethoxy, trifluoromethylthio and cyano. When the groups in question are substituted with more than one substituent the substituents may be the same or different.

The terms "amino acid", "amino acid residue", "natural amino acid" and "natural amino acid residue" as used herein all refer to the D- or L- isomers of the 20 standard amino acid residues: alanine (Ala), arginine (Arg), asparagine (Asn), aspartic acid (Asp), cysteine (Cys), glutamine (Gln), glutamic acid (Glu), glycine (Gly), histidine (His), isoleucine (Ile), leucine (Leu), lysine (Lys), methionine (Met), phenylalanine (Phe), proline (Pro), serine (Ser), threonine (Thr), tryptophan (Trp), tyrosine (Tyr) and valine (Val).

The terms "unnatural amino acid" and "non-natural amino acid residue" as used herein refer to non-standard or modified or unnatural amino acid residues. Examples of non-standard amino acid residues are 4-hydroxyproline, 6-*N*-methyl lysine, 2-aminoisobutyric acid, isovaline, and alpha-methyl serine. Examples of unnatural amino acid residues are pipecolic acid, thiazolidine carboxylic acid, dehydroproline, 3- and 4-methylproline, and 3,3-dimethylproline.

Certain of the above defined terms may occur more than once in the structural formulae, and upon such occurrence each term shall be defined independently of the other.

As used herein, the phrase "a functional group which can be converted to hydrogen in vivo" is intended to include any group, which upon administering the present compounds to the subjects in need thereof can be converted to hydrogen enzymatically or by the acidic environment in the stomach. Non-limiting examples of such groups are acyl, carbamoyl, monoalkylated carbamoyl, dialkylated carbamoyl, alkoxycarbonyl including C₁₋₆-alkoxycarbonyl, alkoxyalkyl groups including C₁₋₆-alkoxy- C₁₋₆-alkyl and the like such as C₁₋₆-alkylcarbonyl, aroyl, C₁₋₆-alkylcarbamoyl (C₁₋₆-N-alkylamide) and di-C₁₋₆ alkyl-alkylcarbamoyl.

As used herein, the phrase "diseases and disorders related to dipeptidyl-peptidase I" is intended to include any disease or disorder in which an effect, preferably an inhibiting effect, on the dipeptidyl-peptidase I enzyme is beneficial.

The term "IC₅₀" as used herein denotes the concentration required for 50% inhibition of DPP-I in a binding assay described herein (DPP-I assay).

Abbreviations and symbols commonly used in the peptide and chemical arts are used herein to describe the compounds of the present invention. In general, the amino acid abbreviations follow the IUPAC-IUB Joint Commission on Biochemical Nomenclature as described in Eur. J. Biochem., 158, 9 (1984).

Certain radical groups are abbreviated herein. t-Bu refers to the tertiary butyl radical, Boc refers to the t-butyloxycarbonyl radical, Fmoc refers to the fluorenylmethoxycarbonyl radical, Ph refers to the phenyl radical, Cbz refers to the benzyloxycarbonyl radical.

### The compounds

The present invention relates to a compound of formula (I) 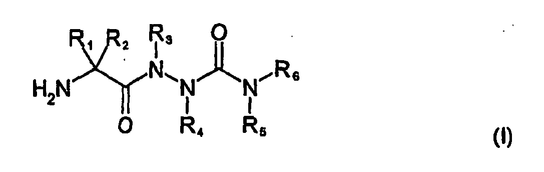 or a pharmaceutically acceptable salt or ester thereof,
wherein
R₁ and R₂ independently of each other are hydrogen, C₁₋₆alkyl, C₂₋₆alkenyl, C₂₋₆alkynyl, an unsubstituted or substituted C₃₋₁₀cycloalkyl group, an unsubstituted or substituted C₆₋₁₀cycloalkenyl group, an unsubstituted or substituted C₃₋₇heterocycloalkyl group, an unsubstituted or substituted C₁₋₆alkylaryl group but not benzyl when R₈ are phenyl, an unsubstituted or substituted C₁₋₆alkylheteroaryl group, an unsubstituted or substituted aryl group, an unsubstituted or substituted heteroaryl group, an unsubstituted or substituted aryl-C₁₋₆alkyl group, an unsubstituted or substituted heteroaryl-C₁₋₆alkyl group, an unsubstituted or substituted C₃₋₇cycloalkyl-C₁₋₆alkyl group, an unsubstituted or substituted C₃₋₇heterocycloalkyl-C₁₋₆alkyl group, or R₁ and R₂ together form an unsubstituted or substituted C₃₋₁₀cycloalkyl group or an unsubstituted or substituted C₃₋₇heterocycloalkyl group;
R₃ is hydrogen, C₁₋₆alkyl, or R₃ and R₁ together form an unsubstituted or substituted C₃₋₁₀ cycloalkyl group or an unsubstituted or substituted C₃₋₇heterocycloalkyl group, or R₃ and R₂ together form an unsubstituted or substituted C₃₋₁₀cycloalkyl group or an unsubstituted or substituted C₃₋₇heterocycloalkyl group;
R₄ and R₅ independently of each other are hydrogen or C₁₋₆alkyl;
R₈ is C₁₋₆alkyl, -CH(R₇)CONH(R₈), C₂₋₆alkenyl, C₂₋₆alkynyl, an unsubstituted or substituted C₃₋₁₀cycloalkyl group, an unsubstituted or substituted C₆₋₁₀cycloalkenyl group, an unsubstituted or substituted C₃₋₇heterocycloalkyl group, an unsubstituted or substituted C₃₋₇cycloalkyl-C₁₋₆alkyl group, an unsubstituted or substituted C₃₋₇heterocycloalkyl-C₁₋₆alkyl group, an unsubstituted or substituted C₁₋₆alkylaryl group, an unsubstituted or substituted C₁₋₆alkyl-heteroaryl group, an unsubstituted or substituted aryl group, an unsubstituted or substituted heteroaryl group, an unsubstituted or substituted aryl-C₁-₆alkyl group, an unsubstituted or substituted heteroaryl-C₁₋₆alkyl group;
R₇ is hydrogen, an unsubstituted or substituted C₁₋₆alkyl group, an unsubstituted or substituted C₃₋₁₀cycloalkyl group, an unsubstituted or substituted C₁₋₆alkylaryl group, an unsubstituted or substituted C₁₋₆alkylheteroaryl group or a side chain of a natural or non-natural amino add residue;
R₈ is hydrogen, an unsubstituted or substituted C₁₋₆alkyl group, an unsubstituted or substituted C₃₋₁₀cycloalkyl group, an unsubstituted or substituted C₁₋₆alkylaryl group, an unsubstituted or substituted C₁₋₆alkylheteroaryl group, a side chain of a natural or non-natural amino add residue or a group -CH(R₇)CONH(R₉);
R₉ is hydrogen, an unsubstituted or substituted C₁₋₆alkyl group, an unsubstituted or substituted C₃₋₁₀cycloalkyl group, an unsubstituted or substituted C₁₋₆alkylaryl group or an unsubstituted or substituted C₁₋₆alkylheteroaryl group; wherein a substituted group is substituted with one, two or three substituents independently selected from the group consisting of C₁₋₆alkyl, C₁₋₆alkylthio, C₁₋₆alkylcarbonyl, C₁₋₆-N-alkylamide, C₁₋₆alkoxy, dialkylamino-C₁₋₆alkyl, amide, hydroxy, carboxy, amino, halogen, trifluoromethyl, trifluoromethoxy, trifluoromethylthio and cyano, with the proviso that either; the compound is neither Ile-Gly^{a}-Leu-Met-NH₂ nor Tyr-AzAla-Gly-Phe-Leu, wherein in Gly^{a} is an α-axa-amino acid; or with the proviso that for use in medicine, the compound is not Ile-Gly^{a}-Leu-Met-NH₂, wherein in Gly^{a} is an α-axa-amino acid; or with the proviso that for use in the preparation of a medicament, the compound may be Ile-Gly^{a}-Leu-Met-NH₂ or Tyr-AzAla-Gly-Phe-Leu, wherein in Gly^{a} is an α-axa-amino acid.

R₁ and R₂ may, independently of each other, be hydrogen, C₁₋₆alkyl, an unsubstituted or substituted phenyl group, an unsubstituted or substituted C₁₋₆alkylaryl group or an unsubstituted or substituted C₁₋₆alkylheteroaryl group; or R₁ and R₂ may, independently of each other, be methyl, ethyl, propyl, butyl, iso-butyl, benzyl, 1-naphthylmethyl, 2-naphthylmethyl or (4-pyridyl)methyl.

In a specific embodiment R₁ is C₁₋₆alkyl and R₂ is H.

In other embodiments, R₁ and R₂ together form an unsubstituted or substituted C₃₋₁₀cycloalkyl group or an unsubstituted or substituted C₃₋₇-heterocycloalkyl group such as an unsubstituted or substituted cyclohexyl group.

R₃ may be hydrogen or methyl.

R₁ and R₃ may together form an unsubstituted or substituted C₃₋₁₀cycloalkyl group or an unsubstituted or substituted C₃₋₇heterocycloalkyl group.

Also, R₂ and R₃ may together form an unsubstituted or substituted C₃₋₁₀cycloalkyl group or an unsubstituted or substituted C₃₋₇-heterocycloalkyl group.

R₄ and/or R₅ may be hydrogen or methyl.

In an interesting embodiment, at least one of R₃ and R₄ and R₅ is hydrogen, and in particular all of R₃ and R₄ and R₅ is hydrogen.

R₆ may be -CH(R₇)CONH(R₈), C₁₋₆alkyl, an unsubstituted or substituted C₁₋₆alkylaryl group or an unsubstituted or substituted C₁₋₆alkylheteroaryl group. Thus, R₆ may be an unsubstituted or substituted benzyl group.

In another embodiment R₆ may be the group -CH(R₇)CONH(R₈).

In particular R₆ may be selected from 2S-3-phenylpropan-2-yl-amide, 2S-N-[2S-3-(m-fluorophenyl)propan-2-yl-amide]-3-cyclohexylpropan-2-yl-amide, 2S-N-[2S-3-(m-fluorophenyl)propan-2-yl-amide]-4-phenylbutan-2-yl-amide, 2S-N-[2S-3-(indol-3-yl)propan-2-yl-amide]-3-phenylpropan-2-yl-amide, 2S-N-[2S-3-(m-fluorophenyl)propan-2-yl-amide]-3-methylbutan-2-yl-amide, 2S-N-[2S-3-(indol-3-yl)propan-2-yl-amide]-butan-2-yl-amide, 2S-N-[2S-3-(indol-3-yl)propan-2-yl-amide]-2-phenylethan-2-yl-amide, 2S-N-[2S-3-(indol-3-yl)propan-2-yl-amide]-4-phenylbutan-2-yl-amide, 2S-N-[2S-3-(m-fluorophenyl)propan-2-yl-amide]-3-(indol-3-yl)propan-2-yl-amide, 2S-N-[2S-3-(m-methoxyphenyl)propan-2-yl-amide]-3-(p-chlorophenyl)propan-2-yl-amide, 2S-N-[2S-3-(1,1'-biphenyl-4-yl)propan-2-yl-amide]-3-phenylpropan-2-yl-amide, 2S-N-[2S-3-(m-methoxyphenyl)propan-2-yl-amide]-3-(1,1'-biphenyl-4-yl)propan2-yl-amide, 2S-N-[2S-3-(1,1'-biphenyl-4-yl)propan-2-yl-amide]-3-(p-hydroxyphenyl)propan-2-yl-amide, 2S-N-[2S-4-methylpentan-2-yl-amide]-3, (benzo[b]thiophen-3-yl)propan-2-yl-amide, 2S-N-[2S-3-(2-naphthyl)propan-2-yl-amide]-4-phenylbutan-2-yl-amide, 2S-N-[2S-3-(m-methoxyphenyl)propan-2-yl-amide]-4-phenylbutan-2-yl-amide, 2S-N-[2S-3-(benzo[b]thiophen-3-yl)propan-2-yl-amide]-4-phenylbutan-2-yl-amide, 2S-N-[2S-3-(p-chlorophenyl)propan-2-yl-amide]-4-phenylbutan-2-yl-amide, 2S-N-[2S-3-(m-fluorophenyl)propan-2-yl-amide]-3-(2-naphthyl)propan-2-yl-amide, 2S-N-[2S-3-(m-fluorophenyl)propan-2-yl-amide]-3-(1,1'-biphenyl-4-yl)propan-2-yl-amide, 2-phenylethyl, 2-(p-hydroxyphenyl)ethyl, benzyl, o-chlorobenzyl, 2-furfuryl, 2-(3-indolyl)ethyl, 1-naphthylmethyl, 4-piperonyl, p-chlorobenzyl, 2-(thiophen-2-yl)ethyl, m-fluorobenzyl, o-fluorobenzyl, p-fluorobenzyl, o-trifluoromethylbenzyl, m-chlorobenzyl, o-methoxybenzyl, o-methylbenzyl, alpha-methylbenzyl, o-(o-hydroxymethylphenylthio)benzyl, o-bromobenzyl, 2-chloro-6-fluorobenzyl, o-ethoxybenzyl, [1,1'-biphenyl-4-yl]methyl, [1,1'-biphenyl-2-yl]methyl, p-[thiophen-2-yl]benzyl, o-[N-morpholinyl]benzyl, o-[N-piperidinyl]benzyl and o-methylthiobenzyl.

In an interesting embodiment R₆ is an unsubstituted or substituted [1,1'-biphenyl-2-yl]methyl group or an unsubstituted or substituted [1,1'-biphenyl-4-yl]methyl group.

R₇ may be hydrogen, an unsubstituted or substituted C₁₋₆alkyl group, an unsubstituted or substituted C₃₋₁₀cycloalkyl group, an unsubstituted or substituted C₁₋₆alkylaryl group, an unsubstituted or substituted C₁₋₆alkylheteroaryl group. Thus, R₇ may be 2-phenylethyl.

R₈ may be hydrogen, -CH(R₇)CONH(R₉) or an unsubstituted or substituted C₁₋₆alkyl group.

Thus, when R₈ is -CH(R₇)CONH(R₉), R₇ may be an unsubstituted or substituted C₁₋₆alkylaryl group or an unsubstituted or substituted C₁₋₆alkyl-heteroaryl group. In an interesting embodiment R₇ is (indol-3-yl)methyl or (m-fluorophenyl)methyl.

R₉ may be hydrogen or an unsubstituted or substituted C₁₋₆alkyl group. In particular, R₉ is hydrogen.

As shown in the examples herein a representative compound of the present invention is a selective inhibitor of DPP-I, especially the compound of Example 11 has been shown to selectively inhibit DPP-I, i.e. it does not inhibit Catepsin B, G, H, L, DPP-IV, neutrophil elastase or tryptase.

Preferred compounds of the invention are:
1-(2*S*-2-Aminopropionyl)-4-(2*S*-3-phenylpropan-2-yl-amide)semicarbazide;
1-(aminoacetyl)-4-(2*S*-3-phenylpropan-2-yl-amide)semicarbazide;
1-(2*S*-2-aminopropionyl)-1-methyl-4-(2*S*-3-phenylpropan-2-yl-amide)semicarbazide;
1-(1-aminocyclohexane-1-carbonyl)-4-[2*S-N*-[2S-3-(*m*-fluorophenyl)propan-2-yl-amide]-3-cyclohexylpropan-2-yl-amide]semicarbazide;
1-(1-aminocyclohexane-1-carbonyl)-4-[2*S*-*N*-[2S-3-(*m*-fluorophenyl)propan-2-yl-amide]-4-phenylbutan-2-yl-amide]semicarbazide;
1-(2*S*-2-aminobutanoyl)-4-[2*S-N*-[2*S*-3-(indol-3-yl)propan-2-yl-amide]-3-phenylpropan-2-yl-amide]semicarbazide;
1-(2*S*-2-aminopropanoyl)-4-[2*S*-*N*-[2*S*-3-(indol-3-yl)propan-2-yl-amide]-3-phenylpropan-2-yl-amide]semicarbazide;
1-(2*S-*3-naphthyl-2-propanoyl)-4-[2*S*-*N*-[2*S*-3-(*m*-fluorophenyl)propan-2-yl-amide]-3-methylbutan-2-yl-amide]semicarbazide;
1-[2*S*-2-amino-3-(4-pyridyl)propanoyl]-4-[2*S*-*N*-(2*S*-3-(indol-3-yl)propan-2-yl-amide)-butan-2-yl-amide]semicarbazide;
1-[2*S*-2-amino-3-(4-pyridyl)propanoyl]-4-[2*S*-N-(2S-3-(indol-3-yl)propan-2-yl-amide)-2-phenylethan-2-yl-amide]semicarbazide;
1-(2*S*-2-Aminobutanoyl)-4-[2*S*-*N*-[2S-3-(indol-3-yl)propan-2-yl-amide]-4-phenylbutan-2-yl-amide]semicarbazide;
1-(2*S*-2-Aminobutanoyl)-4-[2*S*-*N*-[2*S*-3-(*m*-fluorophenyl)propan-2-yl-amide]-3-(indol-3-yl)propan-2-yl-amide]semicarbazide;
1-(2*S*-2-Aminobutanoyl)-4-[2*S*-*N*-[2*S*-3-(*m*-methoxyphenyl)propan-2-yl-amide]-3-(*p-*chlorophenyl)propan-2-yl-amide]semicarbazide;
1-(2*S*-2-Aminopentanoyl)-4-[2*S*-*N*-[2*S*-3-(*m*-methoxyphenyl)propan-2-yl-amide]-3-(*p-*chlorophenyl)propan-2-yl-amide]semicarbazide;
1-(2*S*-2-amino-4-phenylbutanoyl)-4-[2*S*-*N*-[2*S*-3-(1,1'-biphenyl-4-yl)propan-2-yl-amide]-3-phenylpropan-2-yl-amide]semicarbazide;
1-(2*S*-2-aminopentanoyl)-4-[2*S*-*N*-[2*S*-3-(*m*-methoxyphenyl)propan-2-yl-amide]-3-(1,1'-biphenyl-4-yl)propan2-yl-amide]semicarbazide;
1-(2*S*-2-aminobutanoyl)-4-[2*S*-*N*-[2*S*-3-(1,1'-biphenyl-4-yl)propan-2-yl-amide]-3-(*p-*hydroxyphenyl)propan-2-yl-amide]semicarbazide;
1-(2*S*-2-aminobutanoyl)-4-[2*S*-*N*-[2*S*-4-methylpentan-2-yl-amide]-3-(benzo[b]thiophen-3-yl)propan-2-yl-amide]semicarbazide;
1-(2*S*-2-Aminobutanoyl)-4-[2*S*-*N*-[2*S*-3-(2-naphthyl)propan-2-yl-amide]-4-phenylbutan-2-yl-amide]semicarbazide;
1-(2*S*-2-Aminobutanoyl)-4-[2*S*-*N*-[2*S*-3-(*m*-methoxyphenyl)propan-2-yl-amide]-4-phenylbutan-2-yl-amide]semicarbazide;
1-(2*S*-2-Aminobutanoyl)-4-[2*S-N*-[2*S*-3-(benzo[b]thiophen-3-yl)propan-2-yl-amide]-4-phenylbutan-2-yl-amide]semicarbazide;
1-(2*S*-2-Aminobutanoyl)-4-[2*S*-*N*-[2*S*-3-(*p*-chlorophenyl)propan-2-yl-amide]-4-phenylbutan-2-yl-amide]semicarbazide;
1-(2*S*-2-Aminopentanoyl)-4-[2*S*-*N*-[2*S*-3-(2-naphthyl)propan-2-yl-amide]-4-phenylbutan-2-yl-amide]semicarbazide;
1-(2*S*-2-Aminobutanoyl)-4-[2*S*-*N*-[2*S*-3-(*m*-fluorophenyl)propan-2-yl-amide]-3-(2-naphthyl)propan-2-yl-amide]semicarbazide;
1-(2*S*-2-Aminobutanoyl)-4-[2*S*-*N*-[2*S*-3-(*m*-fluorophenyl)propan-2-yl-amide]-3-(1,1'-biphenyl-4-yl)propan-2-yl-amide]semicarbazide;
1-(2*S*-2-aminopropionyl)-4-(2-phenylethyl)semicarbazide;
1-(2*S*-2-aminopropionyl)-4-(2-phenylethyl)-4-methylsemicarbazide;
1-(2*S*-2-aminopropionyl)-4-[2-(*p*-hydroxyphenyl)ethyl]semicarbazide;
1-(2*S*-2-aminopropionyl)-4-benzylsemicarbazide;
1-(2*S*-2-aminopropionyl)-4-(*o*-chlorobenzyl)semicarbazide;
1-(2*S*-2-aminopropionyl)-4-(2-furfuryl)semicarbazide;
1-(2*S*-2-aminopropionyl)-4-[2-(3-indolyl)ethyl]semicarbazide;
1-(2*S*-2-aminopropionyl)-4-(1-naphthylmethyl)semicarbazide;
1-(2*S*-2-aminopropionyl)-4-piperonylsemicarbazide;
1-(2*S*-2-aminopropionyl)-4-(*p*-chlorobenzyl)semicarbazide;
1-(2*S*-2-aminopropionyl)-4-[2-(thiophen-2-yl)ethyl]semicarbazide;
1-(2*S*-2-aminopropionyl)-4-(*m*-fluorobenzyl)semicarbazide;
1-(2*S*-2-aminopropionyl)-4-(*o*-fluorobenzyl)semicarbazide;
1-(2*S*-2-aminopropionyl)-4-(*p*-fluorobenzyl)semicarbazide;
1-(2*S*-2-aminopropionyl)-4-(*o*-trifluoromethylbenzyl)semicarbazide;
1-(2*S*-2-aminopropionyl)-4-(*m*-chlorobenzyl)semicarbazide;
1-(2*S*-2-aminopropionyl)-4-(*o*-methoxybenzyl)semicarbazide;
1-(2*S*-2-aminopropionyl)-4-(*o*-methylbenzyl)semicarbazide;
1-(2*S-*2-aminopropionyl)-4-(alpha-methylbenzyl)semicarbazide;
1-(2*S*-2-aminobutanoyl)-4-(*o*-chlorobenzyl)semicarbazide;
1-(2*S*-2-aminovaleryl)-4-(*o*-chlorobenzyl)semicarbazide;
1-(2*S*-2-aminohexanoyl)-4-(*o*-chlorobenzyl)semicarbazide;
1-(2*S*-2-aminoisocaproyl)-4-(*o*-chlorobenzyl)semicarbazide;
1-(1-aminocyclohexane-1-carbonyl)-4-(*o*-chlorobenzyl)semicarbazide;
1-(2*S*-2-amino-3-phenyl-propionyl)-4-(*o*-chlorobenzyl)semicarbazide;
1,1-(2*S*-1,4-*cyclo*-aminobutanoyl)-4-(*o*-chlorobenzyl)semicarbazide;
1,1-(2*R*-1,4-*cyclo*-aminobutanoyl)-4-(*o*-chlorobenzyl)semicarbazide;
1-(2*S*-2-Aminobutanoyl)-4-[2*S*-*N*-[2*S*-3-(*m*-fluorophenyl)propan-2-yl-amide]-4-phenylbutan-2-yl-amide]semicarbazide;
1-(2*S*-2-Aminobutanoyl)-4-[*o*-(*o*-hydroxymethylphenylthio)benzyl]semicarbazide;
1-(2*S*-2-Aminobutanoyl)-4-(*o*-bromobenzyl)semicarbazide;
1-(2*S*-2-Aminobutanoyl)-4-(2-chloro-6-fluorobenzyl)semicarbazide;
1-(2*S*-2-Aminobutanoyl)-4-(*o*-ethoxybenzyl)semicarbazide;
1-(2*S*-2-Aminobutanoyl)-4-([1,1'-biphenyl-4-yl]methyl)semicarbazide;
1-(2*S*-2-Aminobutanoyl)-4-([1,1'-biphenyl-2-yl]methyl)semicarbazide;
1-(2*S*-2-Aminobutanoyl)-4-(*p*-[thiophen-2-yl]benzyl)semicarbazide;
1-(2*S*-2-Aminobutanoyl)-4-(*o*-[*N*-morpholinyl]benzyl)semicarbazide;
1-(2*S*-2-Aminobutanoyl)-4-(1-naphthylmethyl)semicarbazide;
1-(2*S*-2-Aminobutanoyl)-4-(*o*-[*N*-piperidinyl]benzyl)semicarbazide;
1-(2*S*-2-Aminobutanoyl)-4-(*o*-methylthiobenzyl)semicarbazide;
1-(2*R*-2-aminopropionyl)-4-(2*S*-3-phenylpropan-2-yl-amide)semicarbazide;
1-(2*R*-2-aminopropionyl)-1-methyl-4-(2*S*-3-phenylpropan-2-yl-amide)semicarbazide;
1-(2*R*-2-aminobutanoyl)-4-[2*S-N*-[2S-3-(indol-3-yl)propan-2-yl-amide]-3-phenylpropan-2-yl-amide]semicarbazide;
1-(2*R*-2-aminopropanoyl)-4-[2*S-N-*[2*S*-3-(indol-3-yl)propan-2-yl-amide]-3-phenylpropan-2-yl-amide]semicarbazide;
1-(2*R*-3-naphthyl-2-propanoyl)-4-[2*S*-N-[2*S*-3-(*m*-fluorophenyl)propan-2-yl-amide]-3-methylbutan-2-yl-amide]semicarbazide;
1-[2*R*-2-amino-3-(4-pyridyl)propanoyl]-4-[2*S*-*N*-(2*S*-3-(indol-3-yl)propan-2-yl-amide)-butan-2-yl-amide]semicarbazide;
1-[2*R*-2-amino-3-(4-pyridyl)propanoyl]-4-[2*S*-*N*-(2*S*-3-(indol-3-yl)propan-2-yl-amide)-2-phenylethan-2-yl-amide]semicarbazide;
1-(2*R*-2-Aminobutanoyl)-4-[2*S*-*N*-[2*S*-3-(indol-3-yl)propan-2-yl-amide]-4-phenylbutan-2-yl-amide]semicarbazide;
1-(2*R*-2-Aminobutanoyl)-4-[2*S*-*N*-[2*S*-3-(*m*-fluorophenyl)propan-2-yl-amide]-3-(indol-3-yl)propan-2-yl-amide]semicarbazide;
1-(2*R*-2-Aminobutanoyl)-4-[2*S*-*N*-[2*S*-3-(*m*-methoxyphenyl)propan-2-yl-amide]-3-(*p-*chlorophenyl)propan-2-yl-amide]semicarbazide;
1-(2*R*-2-Aminopentanoyl)-4-[2*S*-*N*-[2*S*-3-(*m*-methoxyphenyl)propan-2-yl-amide]-3-(*p-*chlorophenyl)propan-2-yl-amide]semicarbazide;
1-(2*R*-2-amino-4-phenylbutanoyl)-4-[2*S-N*-[2*S*-3-(1,1'-biphenyl-4-yl)propan-2-yl-amide]-3-phenylpropan-2-yl-amide]semicarbazide;
1-(2*R*-2-aminopentanoyl)-4-[2*S*-*N*-[2*S-*3-(*m*-methoxyphenyl)propan-2-yl-amide]-3-(1,1'-biphenyl-4-yl)propan2-yl-amide]semicarbazide;
1-(2*R*-2-aminobutanoyl)-4-[2*S-N*-[2*S*-3-(1,1'-biphenyl-4-yl)propan-2-yl-amide]-3-(*p-*hydroxyphenyl)propan-2-yl-amide]semicarbazide;
1-(2*R*-2-aminobutanoyl)-4-[2*S*-*N*-[2*S*-*N*-methylpentan-2-yl-amide]-3-(benzo[b]thiophen-3-yl)propan-2-yl-amide]semicarbazide;
1-(2*R*-2-Aminobutanoyl)-4-[2*S*-*N*-[2*S*-3-(2-naphthyl)propan-2-yl-amide]-4-phenylbutan-2-yl-amide]semicarbazide;
1-(2*R*-2-Aminobutanoyl)-4-[2*S*-*N*-[2*S*-3-(*m*-methoxyphenyl)propan-2-yl-amide]-4-phenylbutan-2-yl-amide]semicarbazide;
1-(2*R*-2-Aminobutanoyl)-4-[2*S*-*N*-[2*S*-3-(benzo[b]thiophen-3-yl)propan-2-yl-amide]-4-phenylbutan-2-yl-amide]semicarbazide;
1-(2*R*-2-Aminobutanoyl)-4-[2*S*-*N*-[2*S*-3-(*p*-chlorophenyl)propan-2-yl-amide]-4-phenylbutan-2-yl-amide]semicarbazide;
1-(2*R*-2-Aminopentanoyl)-4-[2*S-N-*[2*S*-3-(2-naphthyl)propan-2-yl-amide]-4-phenylbutan-2-yl-amide]semicarbazide;
1-(2*R*-2-Aminobutanoyl)-4-[2*S-N*-[2*S*-3-(*m*-fluorophenyl)propan-2-yl-amide]-3-(2-naphthyl)propan-2-yl-amide]semicarbazide;
1-(2*R*-2-Aminobutanoyl)-4-[2*S*-*N*-[2*S*-3-(*m*-fluorophenyl)propan-2-yl-amide]-3-(1,1'-biphenyl-4-yl)propan-2-yl-amide]semicarbazide;
1-(2*R*-2-aminopropionyl)-4-(2-phenylethyl)semicarbazide;
1-(2*R*-2-aminopropionyl)-4-(2-phenylethyl)-4-methylsemicarbazide;
1-(2*R*-2-aminopropionyl)-4-[2-(*p*-hydroxyphenyl)ethyl]semicarbazide;
1-(2*R*-2-aminopropionyl)-4-benzylsemicarbazide;
1-(2*R*-2-aminopropionyl)-4-(*o*-chlorobenzyl)semicarbazide;
1-(2*R*-2-aminopropionyl)-4-(2-furfuryl)semicarbazide;
1-(2*R*-2-aminopropionyl)-4-[2-(3-indolyl)ethyl]semicarbazide;
1-(2*R-*2-aminopropionyl)-4-(1-naphthylmethyl)semicarbazide;
1-(2*R*-2-aminopropionyl)-4-piperonylsemicarbazide;
1-(2*R*-2-aminopropionyl)-4-(*p*-chlorobenzyl)semicarbazide;
1-(2*R*-2-aminopropionyl)-4-[2-(thiophen-2-yl)ethyl]semicarbazide;
1-(2*R*-2-aminopropionyl)-4-(*m*-fluorobenzyl)semicarbazide;
1-(2*R*-2-aminopropionyl)-4-(*o*-fluorobenzyl)semicarbazide;
1-(2*R*-2-aminopropionyl)-4-(*p*-fluorobenzyl)semicarbazide;
1-(2*R*-2-aminopropionyl)-4-(*o*-trifluoromethylbenzyl)semicarbazide;
1-(2*R*-2-aminopropionyl)-4-(*m*-chlorobenzyl)semicarbazide;
1-(2*R*-2-aminopropionyl)-4-(*o*-methoxybenzyl)semicarbazide;
1-(2*R*-2-aminopropionyl)-4-(*o*-methylbenzyl)semicarbazide;
1-(2*R*-2-aminopropionyl)-4-(alpha-methylbenzyl)semicarbazide;
1-(2*R*-2-aminobutanoyl)-4-(*o*-chlorobenzyl)semicarbazide;
1-(2*R*-2-aminovaleryl)- 4-(*o*-chlorobenzyl)semicarbazide;
1-(2*R*-2-aminohexanoyl)- 4-(*o*-chlorobenzyl)semicarbazide;
1-(2*R*-2-aminoisocaproyl)- 4-(*o*-chlorobenzyl)semicarbazide;
1-(2*R*-2-amino-3-phenyl-propionyl)- 4-(*o*-chlorobenzyl)semicarbazide;
1-(2*R*-2-Aminobutanoyl)-4-[2*S*-*N*-[2S-3-(*m*-fluorophenyl)propan-2-yl-amide]-4-phenylbutan-2-yl-amide]semicarbazide;
1-(2*R*-2-Aminobutanoyl)-4-[*o*-(*o*-hydroxymethylphenylthio)benzyl]semicarbazide;
1-(2*R*-2-Aminobutanoyl)-4-(*o*-bromobenzyl)semicarbazide;
1-(2*R*-2-Aminobutanoyl)-4-(2-chloro-6-fluorobenzyl)semicarbazide;
1-(2*R*-2-Aminobutanoyl)-4-(*o*-ethoxybenzyl)semicarbazide;
1-(2*R*-2-Aminobutanoyl)-4-([1,1'-biphenyl-4-yl]methyl)semicarbazide;
1-(2*R*-2-Aminobutanoyl)-4-([1,1'-biphenyl-2-yl]methyl)semicarbazide;
1-(2*R*-2-Aminobutanoyl)-4-(*p*-[thiophen-2-yl]benzyl)semicarbazide;
1-(2*R*-2-Aminobutanoyl)-4-(*o*-[*N*-morpholinyl]benzyl)semicarbazide;
1-(2*R*-2-Aminobutanoyl)-4-(1-naphthylmethyl)semicarbazide;
1-(2*R*-2-Aminobutanoyl)-4-(*o*-[N-piperidinyl]benzyl)semicarbazide; and
1-(2*R*-2-Aminobutanoyl)-4-(*o*-methylthiobenzyl)semicarbazide.

The compounds of the invention may exist as geometric isomers or optical isomers or stereoisomers as well as tautomers. Accordingly, the invention includes all geometric isomers and tautomers including mixtures and racemic mixtures of these and a pharmaceutically acceptable salt thereof, especially all *R*- and *S*- isomers.

The present invention also encompasses pharmaceutically acceptable salts of the present compounds. Such salts include pharmaceutically acceptable acid addition salts, pharmaceutically acceptable metal salts, ammonium and alkylated ammonium salts. Acid addition salts include salts of inorganic acids as well as organic acids. Representative examples of suitable inorganic acids include hydrochloric, hydrobromic, hydroiodic, phosphoric, sulfuric, nitric acids and the like. Representative examples of suitable organic acids include formic, acetic, trichloroacetic, trifluoroacetic, propionic, benzoic, cinnamic, citric, fumaric, glycolic, lactic, maleic, malic, malonic, mandelic, oxalic, picric, pyruvic, salicylic, succinic, methanesulfonic, ethanesulfonic, tartaric, ascorbic, pamoic, bismethylene salicylic, ethanedisulfonic, gluconic, citraconic, aspartic, stearic, palmitic, EDTA, glycolic, p-aminobenzoic, glutamic, benzenesulfonic, p-toluenesulfonic acids and the like. Further examples of pharmaceutically acceptable inorganic or organic acid addition salts include the pharmaceutically acceptable salts listed in J. Pharm. Sci. 1977, 66, 2.
Examples of metal salts include lithium, sodium, potassium, magnesium salts and the like. Examples of ammonium and alkylated ammonium salts include ammonium, methylammonium, dimethylammonium, trimethylammonium, ethylammonium, hydroxyethylammonium, diethylammonium, butylammonium, tetramethylammonium salts and the like.

Also intended as pharmaceutically acceptable acid addition salts are the hydrates, which the present compounds are able to form.

The acid addition salts may be obtained as the direct products of compound synthesis. In the alternative, the free base may be dissolved in a suitable solvent containing the appropriate acid, and the salt isolated by evaporating the solvent or otherwise separating the salt and solvent.

The compounds of the present invention may form solvates with standard low molecular weight solvents using methods well known to the person skilled in the art. Such solvates are also contemplated as being within the scope of the present invention.

The invention also encompasses prodrugs of the present compounds, which on administration undergo chemical conversion by metabolic processes before becoming active pharmacological substances. In general, such prodrugs will be functional derivatives of the present compounds, which are readily convertible in vivo into the required compound of the Formula I. Prodrugs are any covalently bonded compounds, which release the active parent drug according to Formula I in vivo. If a chiral center or another form of an isomeric center is present in a compound of the present invention, all forms of such isomer or isomers, including enantiomers and diastereomers, are intended to be covered herein. Inventive compounds containing a chiral center may be used as a racemic mixture, an enantiomerically enriched mixture, or the racemic mixture may be separated using well-known techniques and an individual enantiomer may be used alone. In cases in which compounds have unsaturated carbon-carbon double bonds, both the cis (Z) and trans (E) isomers are within the scope of this invention. In cases wherein compounds may exist in tautomeric forms, such as keto-enol tautomers, each tautomeric form is contemplated as being included within this invention whether existing in equilibrium or predominantly in one form. Conventional procedures for the selection and preparation of suitable prodrug derivatives are described, for example, in "Design of Prodrugs", ed. H. Bundgaard, Elsevier, 1985.

The invention also encompasses active metabolites of the present compounds.

The present invention includes all complexes of the compounds of this invention.

The meaning of any substituent at any one occurrence in Formula I or any subformula thereof is independent of its meaning, or any other substituent's meaning, at any other occurrence, unless specified otherwise.

In a preferred embodiment of this invention, the compounds of Formula I exhibit an IC₅₀ value of less than 500 *µ*M, preferably less than 100 *µ*M, more preferably less than 50 *µ*M, even more preferably less than 1 *µ*M, especially less than 500 nM, particularly less than 100 nM, when subjected to a human dipeptidyl dipeptidase-I assay such as the assay disclosed herein.

### Methods of Preparation

The compounds of the present invention may be prepared by the methods set forth in the schemes 1-3 below.

A list of the used abbreviations is given below under Materials and Methods. 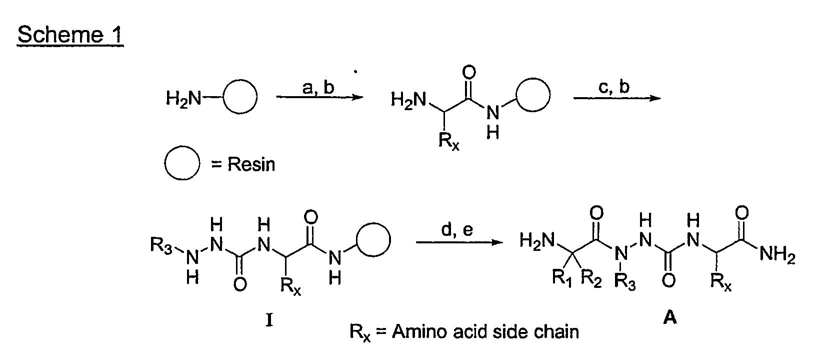 Reagents and conditions: a) Fmoc-AA-OPfp, DhbtOH, DMF; or Fmoc-AA-OH, TBTU, NEM, DMF; b) piperidine, DMF; c) Fmoc-NHNH-CO₂Pfp, DhbtOH, DMF; or Fmoc-N(R₃)NH₂, CDI, DMF; d) FmocNHC(R₁)R₂CO₂H or, BocNHC(R₁)R₂CO₂H TBTU, NEM, DMF; or, FmocNHC(R₁)R₂CO₂Pfp, DhbtOH, DMF; e) 2% TIPS in TFA.

The first Fmoc protected amino acid is loaded onto Rink derivatized resin by conventional peptide coupling procedures (such as by the use of Pfp esters or the TBTU coupling reagent), in an aprotic solvent (such as DMF). The semicarbazide (intermediate **I**) is formed by acylation with either Fmoc-NHNH-CO₂Pfp or Fmoc-N(R₃)NH-COIm in an aprotic solvent (such as DMF), with or without the use of DhbtOH additive. The N-terminal residue is introduced by acylation, such as with an activated Fmoc or Boc protected amino acid, in DCM or DMF, with or without the addition of base. The desired products **A** are obtained upon cleavage with a TFA/TIPS mixture (the Fmoc group, if any, is removed prior to cleavage).

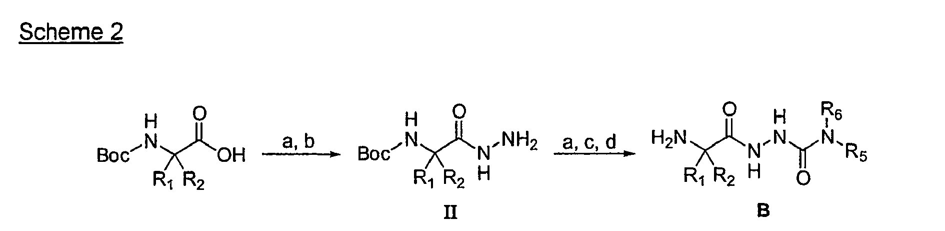

Reagents and conditions: a) CDI, THF; b) NH₂NH₂, THF; c) R₅R₆NH, THF; d) TFA.

Semicarbazides of formula **B** are prepared without the use of solid support. A Boc protected amino acid is converted to its hydrazide by activation with CDI, followed by reaction with hydrazine hydrate. The amino acid hydrazide II is then reacted with CDI again, and displacement with a range of different amines, followed by Boc deprotection gives the crude products, which are purified by HPLC.

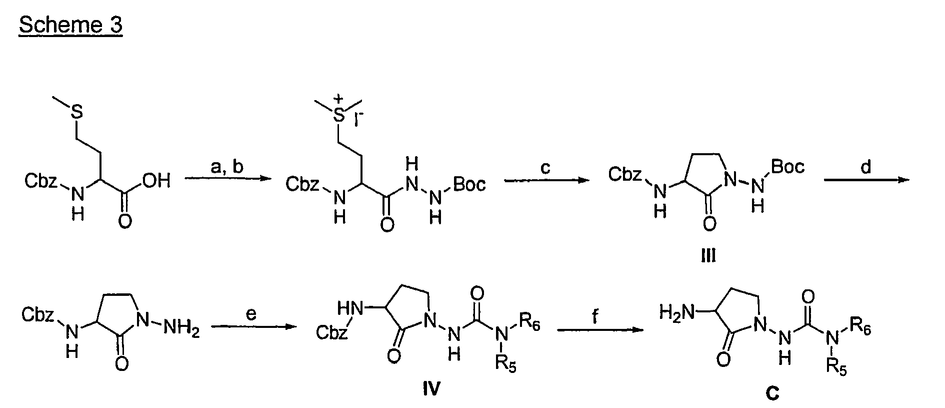

Reagents and conditions: a) CDI, THF; then BocNHNH₂; b) Mel; c) NaH, DMF, DCM; d) TFA, DCM; or HCl in dioxane; e) CDI, THF; then R₅R₆NH; f) TFA, TMSBr, PhSMe; or HBr in acetic acid

Semicarbazides of formula C are prepared starting from the 3-benzyloxycarbonylamino-1-*tert*-butoxycarbonylaminopyrrolidin-2-one III, which is prepared analogous to general procedures described by Freidinger et al. (J. Org. Chem., 1982, 47, 104-109) and Duffy et al. (Bioorg. Med. Chem. Lett., 1999, 9, 1907-1910). The Boc group is preferably removed with HCl in dioxane. Formation of the semicarbazide bond (intermediate **IV**) is achieved by reaction with CDI in THF followed by displacement with the appropriate amine. The Cbz group is either removed by TMSBr in TFA using thioanisole as scavenger, or by HBr in acetic acid. The desired products are obtained after HPLC purification.

The starting materials used herein are commercially available amino acids or are prepared by routine methods well known to those of ordinary skill in the art and can be found in standard reference books, such as the COMPENDIUM OF ORGANIC SYNTHETIC METHODS, Vol. I-VI (published by Wiley-Interscience).

Coupling methods to form amide bonds herein are generally well known to the art. The methods of peptide synthesis generally set forth by Bodansky et al., THE PRACTICE OF PEPTIDE SYNTHESIS, Springer-Verlag, Berlin, 1984; E. Gross and J. Meienhofer, THE PEPTIDES, Vol. 1, 1-284 (1979); and J. M. Stewart and J. D. Young, SOLID PHASE PEPTIDE SYNTHESIS, 2d Ed., Pierce Chemical Co., Rockford, III., 1984, are generally illustrative of the technique.

Synthetic methods to prepare the compounds of this invention frequently employ protective groups to mask a reactive functionality or minimize unwanted side reactions. Such protective groups are described generally in Green, T. W. PROTECTIVE GROUPS IN ORGANIC SYNTHESIS, John Wiley & Sons, New York (1981). The term "amino protecting groups" generally refers to the Boc, acetyl, benzoyl, Fmoc and Cbz groups and derivatives thereof as known to the art. Methods for protection and deprotection, and replacement of an amino protecting group with another moiety are well known.

Acid addition salts of the compounds of Formula I are prepared in a standard manner in a suitable solvent from the parent compound and an excess of an acid, such as hydrochloric, hydrobromic, hydrofluoric, sulfuric, phosphoric, acetic, trifluoroacetic, maleic, succinic or methanesulfonic. Certain of the compounds form inner salts or zwitterions that may be acceptable. Cationic salts are prepared by treating the parent compound with an excess of an alkaline reagent, such as a hydroxide, carbonate or alkoxide, containing the appropriate cation; or with an appropriate organic amine. Cations such as Li⁺, Na⁺, K⁺, Ca⁺⁺, Mg⁺⁺ and NH₄⁺ are specific examples of cations present in pharmaceutically acceptable salts. Halides, sulfate, phosphate, alkanoates (such as acetate and trifluoroacetate), benzoates, and sulfonates (such as mesylate) are examples of anions present in pharmaceutically acceptable salts.

### Pharmaceutical compositions

In one aspect of this invention, there is provided a pharmaceutical composition comprising, as an active ingredient, a compound of the present invention together with a pharmaceutically acceptable carrier or diluent. This composition may be in unit dosage form and may comprise from about 0.1µg to about 1000 mg such as, e.g., from about 1 µg to about 500 mg, from about 5 µg to about 250 mg, from about 50 µg to about 100 mg or from about 0.1 to about 50 mg, of the compound of the invention or a pharmaceutically acceptable salt or ester thereof. The composition of the invention may be used for oral, nasal, transdermal, pulmonal or parenteral administration. It is contemplated that the pharmaceutical composition of the invention is useful for treatment of inflammation, type2 diabetes, asthma, severe influenza, respiratory syncytial virus infection, CD8 T cell inhibition, inflammatory bowel diseases, psoriasis, atopic dermatitis, Papillon Lefevre syndrome, Haim Munk syndrome, gum disease, periodontitis, rheumatoid arthritis, Huntington's disease, Chagas' disease, Alzheimer's disease, sepsis and/or for application in target cell apoptosis.

The compounds of the invention may be administered alone or in combination with pharmaceutically acceptable carriers, diluents or excipients, in either single or multiple doses. Accordingly, the compounds of Formula I may be used in the manufacture of a medicament. The pharmaceutical compositions according to the invention may be formulated with pharmaceutically acceptable carriers or diluents as well as any other known adjuvants and excipients in accordance with conventional techniques such as those disclosed in Remington: The Science and Practice of Pharmacy, 19.sup.th Edition, Gennaro, Ed., Mack Publishing Co., Easton, Pa., 1995.

The pharmaceutical compositions may be specifically formulated for administration by any suitable route such as the oral, rectal, nasal, pulmonary, topical (including buccal and sublingual), transdermal, intracisternal, intraperitoneal, vaginal and parenteral (including subcutaneous, intramuscular, intrathecal, intravenous and intradermal) route, the oral route being preferred. It will be appreciated that the preferred route will depend on the general condition and age of the subject to be treated, the nature of the condition to be treated and the active ingredient chosen.

Pharmaceutical compositions for oral administration include solid dosage forms such as capsules, tablets, dragees, pills, lozenges, powders and granules. Where appropriate, they can be prepared with coatings such as enteric coatings or they can be formulated so as to provide controlled release of the active ingredient such as sustained or prolonged release according to methods well known in the art.

Liquid dosage forms for oral administration include solutions, emulsions, suspensions, syrups and elixirs.

Pharmaceutical compositions for parenteral administration include sterile aqueous and non-aqueous injectable solutions, dispersions, suspensions or emulsions as well as sterile powders to be reconstituted in sterile injectable solutions or dispersions prior to use. Depot injectable formulations are also contemplated as being within the scope of the present invention.

Other suitable administration forms include suppositories, sprays, ointments, cremes, gels, inhalants, dermal patches, implants etc.

A typical oral dosage may be in the range of from about 0.001 to about 100 mg/kg body weight per day, preferably from about 0.01 to about 50 mg/kg body weight per day, and more preferred from about 0.05 to about 10 mg/kg body weight per day administered in one or more dosages such as 1 to 3 dosages. The exact dosage will depend upon the frequency and mode of administration, the sex, age, weight and general condition of the subject treated, the nature and severity of the condition treated and any concomitant diseases to be treated and other factors evident to those skilled in the art. A person skilled in the art know how to adjust the dosage to obtain the desired effect.

The formulations may conveniently be presented in unit dosage form by methods known to those skilled in the art. A typical unit dosage form for oral administration one or more times per day such as 1 to 3 times per day may contain of from about 1 µg to about 1000 mg such as, e.g., from about 0.05 to about 1000 mg, preferably from about 0.1 to about 500 mg, and more preferred from about 0.5 mg to about 200 mg.

For parenteral routes, such as intravenous, intrathecal, intramuscular and similar administration, typically doses are in the order of about half the dose employed for oral administration.

The compounds of this invention are generally utilized as the free substance or as a pharmaceutically acceptable salt thereof. One example is an acid addition salt of a compound having the utility of a free base. When a compound of the Formula (I) contains a free base such salts are prepared in a conventional manner by treating a solution or suspension of a free base of the Formula (I) with a chemical equivalent of a pharmaceutically acceptable acid, for example, inorganic and organic acids. Representative examples are mentioned above. Physiologically acceptable salts of a compound with a hydroxy group include the anion of said compound in combination with a suitable cation such as sodium or ammonium ion.

For parenteral administration, solutions of the novel compounds of the Formula (I) in sterile aqueous solution, aqueous propylene glycol or sesame or peanut oil may be employed. Such aqueous solutions should be suitable buffered if necessary and the liquid diluent first rendered isotonic with sufficient saline or glucose. The aqueous solutions are particularly suitable for intravenous, intramuscular, subcutaneous and intraperitoneal administration. The sterile aqueous media employed are all readily available by standard techniques known to those skilled in the art.

Suitable pharmaceutical carriers include inert solid diluents or fillers, sterile aqueous solution and various organic solvents. Examples of solid carriers are lactose, terra alba, sucrose, cyclodextrin, talc, gelatine, agar, pectin, acacia, magnesium stearate, stearic acid or lower alkyl ethers of cellulose. Examples of liquid carriers are syrup, peanut oil, olive oil, phospholipids, fatty acids, fatty acid amines, polyoxyethylene or water. Similarly, the carrier or diluent may include any sustained release material known in the art, such as glyceryl monostearate or glyceryl distearate, alone or mixed with a wax. The pharmaceutical compositions formed by combining the novel compounds of the Formula (I) and the pharmaceutically acceptable carriers are then readily administered in a variety of dosage forms suitable for the disclosed routes of administration. The formulations may conveniently be presented in unit dosage form by methods known in the art of pharmacy.

Formulations of the present invention suitable for oral administration may be presented as discrete units such as capsules or tablets, each containing a predetermined amount of the active ingredient, and which may include a suitable excipient. These formulations may be in the form of powder or granules, as a solution or suspension in an aqueous or non-aqueous liquid, or as an oil-in-water or water-in-oil liquid emulsion.

If a solid carrier is used for oral administration, the preparation may be tabletted; placed in a hard gelatine capsule in powder or pellet form or it can be in the form of a troche or lozenge. The amount of solid carrier will vary widely but will usually be from about 25 mg to about 1 g. If a liquid carrier is used, the preparation may be in the form of a syrup, emulsion, soft gelatine capsule or sterile injectable liquid such as an aqueous or non-aqueous liquid suspension or solution.

A typical tablet, which may be prepared by conventional tabletting techniques, may contain:

| *Core:* | |
|---|---|
| Active compound (free compound or salt) | 5.0 mg |
| Lactosum Ph. Eur. | 67.8 mg |
| Cellulose, microcryst. (Avicel) | 31.4 mg |
| Amberlite | 1.0 mg |
| Magnesii stearas | q.s. |

| *Coating:* | |
|---|---|
| Hydroxypropyl methylcellulose approx. | 9 mg |
| Acylated monoglyceride approx. | 0.9 mg |

If desired, the pharmaceutical composition of the invention may comprise the compound of the Formula (I) in combination with further pharmacologically active substances such as those described in the foregoing.

### Use of the invention

The compounds of Formula I are useful as protease inhibitors, particularly as inhibitors of cysteine and serine proteases, more particularly as inhibitors of cysteine proteases, even more particularly as inhibitors of cysteine proteases of the papain superfamily, yet more particularly as inhibitors of DPP-I and selective inhibitors of DPP-I. The present invention provides useful compositions and formulations of said compounds, including pharmaceutical compositions and formulations of said compounds.

The compounds of the present invention may especially be useful for the treatment or prevention of diseases such as inflammation, type2 diabetes and similar diseases involving a protease. The present compounds are especially useful for treating diseases in which cysteine proteases are implicated and especially diseases in which dipeptidyl peptidase-I is implicated; most particularly mast cell inflammatory mediated diseases. Examples of diseases in which dipeptidyl peptidase-I is implicated are: inflammation, type2 diabetes, asthma, severe influenza, respiratory syncytial virus infection, CD8 T cell inhibition, inflammatory bowel diseases, psoriasis, atopic dermatitis, Papillon Lefevre syndrome, Haim Munk syndrome, gum disease, periodontitis, rheumatoid arthritis, Huntington's disease, Chagas' disease, Alzheimer's disease, sepsis as well as in target cell apoptosis.

It is contemplated that an effective amount of a compound or a composition of this invention corresponds to an a mount of active ingredient, i.e. active compound or a pharmaceutically acceptable salt or ester thereof, in the range of e.g. from about 0.05 to about 100 mg per day, preferably from about 0.1 to about 50 mg per day.

In yet another aspect, the present invention relates to use of a compound of this invention for the preparation of a medicament, preferably a medicament for treatment of inflammation, type2 diabetes, asthma, inflammatory bowel diseases, psoriasis, atopic dermatitis, Papillon Lefevre syndrome. Haim Munk syndrome, gum disease, periodontitis, arthritis, Huntington's disease, Chagas' disease, Alzheimer's disease, sepsis or for application in target cell apoptosis.

For acute therapy, parenteral administration of a compound of Formula I is preferred. An intravenous infusion of the compound in 5% dextrose in water or normal saline, or a similar formulation with suitable excipients, is most effective, although an intramuscular bone injection is also useful. Typically, the parenteral dose will be about 0.01 to about 100 mg/kg; preferably between 0.1 and 20 mg/kg, in a manner to maintain the concentration of drug in the plasma at a concentration effective to inhibit dipeptidyl dipeptidase-I (cathepsin C). The compounds may be administered one to four times daily at a level to achieve a total daily dose of about 0.4 to about 400 mg/kg/day. The precise amount of an inventive compound which is therapeutically effective, and the route by which such compound is best administered, is readily determined by one of ordinary skill in the art by comparing the blood level of the agent to the concentration required to have a therapeutic effect.

The compounds of this invention may also be administered orally to the patient, in a manner such that the concentration of drug is sufficient to inhibit bone resorption or to achieve any other therapeutic indication as disclosed herein. Typically, a pharmaceutical composition containing the compound is administered at an oral dose of between about 0.1 to about 50 mg/kg in a manner consistent with the condition of the patient. Preferably the oral dose would be about 0.5 to about 20 mg/kg.

No unacceptable toxicological effects are expected when compounds of the present invention are administered in accordance with the present invention.

The compounds of the present invention fully or partly inhibit dipeptidyl-peptidase I, and are thus useful for the treatment and/or prevention of a wide variety of conditions and disorders in which inhibition of DPP-I is beneficial. It is of specific interest that a selective inhibitory effect can be obtained by use of the compounds of the invention, i.e. side-effects relating to inhibition of other proteases can be markedly reduced or eliminated.

Accordingly, in another aspect the present invention relates to a compound of the general Formula (I) or any optical or geometric isomer or tautomeric form thereof including mixtures of these or a pharmaceutically acceptable salt thereof for use as a pharmaceutical composition.

The invention also relates to pharmaceutical compositions comprising, as an active ingredient, at least one compound of the Formula (I) or any optical or geometric isomer or tautomeric form thereof including mixtures of these or a pharmaceutically acceptable salt thereof together with one or more pharmaceutically acceptable carriers or diluents.

Furthermore, the invention also relates to the use of the compounds and compositions of the present invention to modulate DPPI levels in a subject (e.g., human) in need thereof in an amound effective to modulate DPPI levels. In a preferred embodiment, the compound or composition inhibits DPPI.

In the following synthetic examples, all of the starting materials were obtained from commercial sources unless otherwise indicated. Without further elaboration, it is believed that one skilled in the art can, using the preceding description, utilize the present invention to its fullest extent. These examples are given to illustrate the invention, not to limit its scope.

### EXAMPLES

### Materials and Methods

Temperature is in degrees Centigrade (°C). Experiments were conducted at room temperature (20 °C), unless otherwise noted. All solvents were HPLC grade. Anhydrous solvents were obtained by storing over 4 Å activated molecular sieves. Unless otherwise noted starting materials were purchased from commercial suppliers and used without further purification.

Solid phase reactions run at room temperature were performed in flat-bottom polyethylene syringes equipped with sintered Teflon filters (70 µm pores), Teflon tubing, Teflon valves for flow control, and suction to drain the syringes from below, or on a VacMaster parallel synthesis rack. Fmoc deprotection was performed with 20% (v/v) piperidine in DMF (2 + 10 min.). TBTU-couplings were performed by dissolving the acid (3 eq.) in DMF with NEM (4 eq.), followed by addition of TBTU (2.88 eq.). The resulting solution was preactivated for 10 min. before use. Pfp esters (3 eq.) were coupled with DhbtOH (1 eq.) present. The disappearance of the bright yellow color indicated complete capping of the resin-bound amino groups. Solid phase reactions were generally run in an amount of solvent that was enough to cover the resin. Resin loadings were determined by Fmoc cleavage and optical density measurements at 290 nm, using a calibration curve.

NMR data were acquired on a Bruker Advance DRX 250. CDCl₃ is deuteriochloroform, DMSO-d₆ is hexadeuteriodimethylsulfoxide, D₂O is deuteriooxide, and CD₃OD is tetradeuteriomethanol. Abbreviation for NMR data are as follows: s = singlet, d = doublet, t = triplet, q = quartet, m = multiplet, dd = doublet of doublets, dt =doublet of triplets, td = triplet of doublets, app = apparent, br = broad. Chemical shifts are reported in ppm, relative to internal solvent peaks (2.49 for DMSO-*d*₆, 7.25 for CDCl₃, 4.75 for D₂O, 3.35 for CD₃OD). Coupling constants *J* are reported in Hz. ES-MS spectra were obtained on a Micromass Quattro micro™ instrument in the positive mode unless otherwise noted.

Analytical HPLC was performed on a Gilson system (UV/VIS-155 detector at 215 and 254 nm, 402 syringe pump, 819 injection module, valvemate 35, 864 degasser, 233 XL on-line column switching module, and a Zorbax 300SB RP-18 column, 4.6 x 50 mm with a 322 pump). Eluents A (0.1 % TFA in water) and B (1% TFA in acetonitrile) were used in a linear gradient (0% B → 100% B in 7 min.). Purity (given in parentheses) is at 215 nm.

Preparative HPLC was performed on the same Gilson system, using a Zorbax 300SB RP-18, 21.2 mm x 25 cm column, with a flow of 15 mL/min.

### Abbreviations

- AA: Amino acid
- AFC: 7-Amino-trifluoromethyl coumarin
- CDI: 1,1'-Carbonyldiimidazole
- DCM: Dichloromethane
- DE: Diethylether
- DhbtOH: 3,4-Dihydro-3-hydroxy-4-oxo-1,2,3-benzotriazine
- DIC: *N,N'*-Diisopropylcarbodiimide
- DIPEA: *N,N-*Diisopropylethylamine
- DMAP: 4-Dimethylaminopyridine
- DMF: *N,N*-Dimethylformamide
- DMSO: Dimethyl sulfoxide
- EA: Ethyl acetate
- EDTA: Ethylenediaminetetraacetic acid
- Fmoc: 9-Fluorenylmethoxycarbonyl
- Im: 1-Imidazolyl
- NEM: *N*-Ethylmorpholine
- PE: Petroleum ether (bp. 60-80°)
- PEGA: Poly(ethylene glycol)-poly(acryl amide) copolymer
- Pfp: Pentafluorophenyl
- Rink amide linker: *p*-[(*R*,*S*)-α-[1-(9*H*-fluoren-9-yl)-methoxyformamido]-2,4-dimethoxybenzyl]-phenoxyacetic acid
- TBTU: *N*-[(1*H*-Benzotriazol-1-yl)-(dimethylamino)-methylene]-*N-*methylmethan-aminium tetrafluoroborate *N*-oxide
- TFA: Trifluoroacetic acid
- THF: Tetrahydrofurane
- TIPS: Triisopropylsilane
- TMSBr: Trimethylsilyl bromide

### Nomenclature

The compound class described in the following can be viewed as both azapeptides and derivatives of semicarbazide. In naming the compounds in the examples below the term semicarbazide has been used as the parent group, rather than the term azapeptide.

### EXAMPLE 1

### 1-(2S-2-Aminopropionyl)-4-(2S-3-phenylpropan-2-yl-amide)semicarbazide

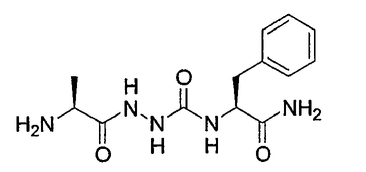

a) Preparation of Fmoc-NHNH₂: Fmoc-Cl (10 g, 38.7 mmol) dissolved in diethyl ether (180 mL) was added dropwise to a solution of hydrazine hydrate (3.8 mL, 77 mmol) in diethyl ether (100 mL) cooled in an ice-bath. White precipitation formed quickly. When all the Fmoc-Cl was added the resulting white suspension was warmed to rt. and stirred for 30 min. The white solid was isolated by filtration, washed with diethyl ether (x3) and water (x3) and dried *in vacuo* to give the desired product. Yield: 8 g (81 %); R_{f} = 0.2 (PE:EA 1:1 + a few drops of acetic acid); HPLC: Rₜ = 4.16 min. (>99%); ¹H-NMR (DMSO-d₆, 250 MHz) δ 8.34 (br, 1H), 7.90-7.88 (d, *J* = 7.3, 2H), 7.71-7.68 (d, *J* = 7.3, 2H), 7.45-7.29 (m, 4H), 4.30-4.18 (m, 3H), 4.08 (br, 2H); ¹³C (DMSO-d₆, 250 MHz) δ 158.2, 143.8, 140.7, 127.6, 127.0, 125.2, 120.0, 65.6, 46.7; ES-MS: mass calcd for C₁₅H₁₅N₂O₂ 255.1 (MH⁺). Found *mlz* 255.1.
b) Preparation of Fmoc-NHNH-CO₂Pfp: Fmoc-NHNH₂ (750 mg, 2.95 mmol) and bis(pentafluorophenyl)carbonate (1221 mg, 3.1 mmol) were stirred in dry THF (15 mL) at rt for 18 h. The reaction mixture was diluted with water (50 mL) and extracted with EA (x3). The combined organic fractions were washed with 1 M HCl (x1) and brine (x1), dried (MgSO₄) and concentrated to give a clear oil, which solidified *in vacuo.* Off-white solid. Yield: 1.32 g (96%). R_{f} = 0.4 (PE:EA 3:1). This compound was used crude, since it was partially unstable to purification by both recrystallization and silica chromatography.
c) Scheme 1: PEGA₈₀₀ resin derivatized with Rink amide linker (L = 0.3 mmol/g, 405 mg, 109 µmol) was Fmoc deprotected, and then washed with DMF (x5). Fmoc-Phe-OPfp (182 mg, 328 µmol) and DhbtOH (18 mg, 109 µmol) were dissolved in DMF (3 mL) and added to the resin. After disappearance of the yellow color the resin was washed with DMF (x5). Following Fmoc deprotection (as above) and wash with DMF (x5) the resin-bound amine was acylated with Fmoc-NHNH-CO₂Pfp (170 mg, 366 *µ*mol) in the presence of DhbtOH (18 mg, 109 *µ*mol) in DMF (3 mL) for 18 h. The resin was washed with DMF (x5), Fmoc deprotected and washed again with DMF (x5). Fmoc-Ala-OPfp (174 mg, 328 µmol) and DhbtOH (18 mg, 109 *µ*mol) were dissolved in DMF (3 mL) and added to the resin. After disappearance of the yellow color the resin was washed with DMF (x5), then Fmoc deprotected and finally washed with DMF (x5) and DCM (x3). Cleavage was achieved by 1 h treatment with 2% TIPS in TFA (drops of DCM added to obtain a homogeneous solution). The resin was washed several times (x6) with TFA solution and the combined washings were concentrated to give a clear oil. The crude product was purified by preparative HPLC. The title compound was obtained as a white solid. Yield: 24.5 mg (69%); HPLC: Rₜ = 2.77 min. (>98%); ¹H-NMR (D₂O, 250 MHz) δ 7.13-6.99 (m, 5H), 4.19-4.13 (dd, *J* = 6.1, 8.3, 1H), 3.87-3.79 (q, *J* = 7.1, 1H), 2.90-2.82 (dd, *J* = 6.1, 14.0, 1H), 2.75-2.66 (dd*, J* = 8.4, 14.0, 1H), 1.26-1.23 (d, *J* = 7.1, 3H); ES-MS: mass calcd for C₁₃H₂₀N₅O₃ 294.1 (MH⁺). Found *m*/*z* 294.2.

### EXAMPLE 2

### 1-(Aminoacetyl)-4-(2S-3-phenylpropan-2-yl-amide)semicarbazide

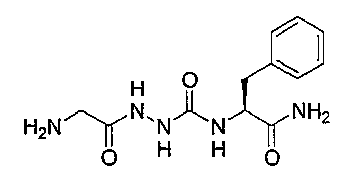

Scheme 1: Starting from 200 mg resin (0.06 mmol), following the general procedure of Example 1c, except substituting Fmoc-Ala-OPfp for Fmoc-Gly-OPfp. Formation of the semicarbazide bond was achieved by acylation with Fmoc-NHNHCOlm. This was prepared just prior to use by reaction of FmocNHNH₂ (0.18 mmol) and CDI (0.18 mmol) in dry DMF (1 mL) for 1h. The resulting solution was then added directly to the resin. Reaction time 16 h. The title compound was obtained as a white solid. Yield: 3.1 mg (19%); HPLC: Rₜ = 2.42 min. (>99%); ES-MS: mass calcd for C₁₂H₁₈N₅O₃ 280.1 (MH⁺). Found *m*/*z* 280.1

### EXAMPLE 3

### 1-(2S-2-Aminopropionyl)-1-methyl-4-(2S-3-phenylpropan-2-yl-amide)semicarbazide

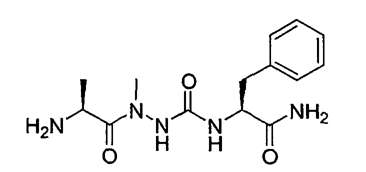

Starting from 315 mg resin (0.095 mmol), following the general procedure of Example 2, except substituting FmocNHNH₂ for FmocN(Me)NH₂ (prepared according to procedure in J. Org. Chem., 1999, 64, 7388-7394). Preactivation with CDI performed for 3 h instead of just 1h. White solid. Yield: 13.3 mg (46%); HPLC: Rₜ = 2.71 min. (>99%); ¹H-NMR (DMSO-*d*₆, 250 MHz) δ 8.64 (s, 1H), 8.10 (br, 2H), 7.59 (s, 1H), 7.31-7.14 (m, 6H), 6.88 (br, 1H), 4.43-4.35 (m, 1H), 3.50 (1H, hidden under water signal), 3.09-3.05 (m, 1H), 2.90 (s, 3H), 2.84-2.74 (m, 1H), 1.30-1.18 (br, 3H); ES-MS: mass calcd for C₁₄H₂₂N₅O₃ 308.2 (MH⁺). Found *m*/*z* 308.0.

Examples 4-26 were all prepared using TentaGel S-NH₂ resin (L = 0.41 mmol/g, 150 mg, 0.062 mmol) derivatized with the Rink linker. Coupling of individual amino acid derivatives were performed using TBTU or Pfp-ester chemistry, as described in Example 1 and in Materials and Methods. The semicarbazide bond was formed using Fmoc-NHNH-COlm, as described in Example 2. Wash, cleavage and purification by HPLC were performed as outlined in Example 1c.

### EXAMPLE 4

### 1-(1-Aminocyclohexane-1-carbonyl)-4-[2S-N-[2S-3-(m-fluorophenyl)propan-2-yl-amide]-3-cyclohexylpropan-2-yl-amide]semicarbazide

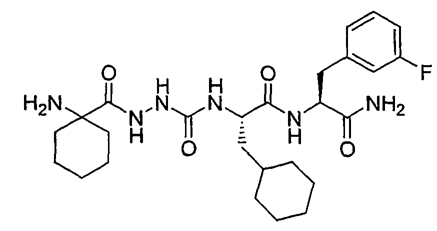

White solid. Yield: 19.9 mg (62%); HPLC: Rₜ = 4.10 min. (>99%); ¹H-NMR (DMSO-*d*₆/D₂O 10:1, 250 MHz) δ 7.32-7.23 (m, 1H), 7.05-6.94 (m, 3H), 4.46-4.37 (m, 1H), 4.07-4.01 (m, 1H), 3.06-2.98 (dd, *J* = 5.0, 13.8, 1H), 2.86-2.77 (dd, *J* = 9.2, 13.8, 1H), 2.05-1.96 (m, 2H), 1.74-1.15 (m, 19H), 1.07-0.74 (m, 2H); ES-MS: mass calcd for C₂₆H₄₀FN₆O₄ 519.3 (MH⁺). Found *m*/*z* 519.1.

### EXAMPLE 5

### 1-(1-Aminocyclohexane-1-carbonyl)-4-[2S-N-[2S-3-(m-fluorophenyl)propan-2-yl-amide]-4-phenylbutan-2-yl-amide]semicarbazide

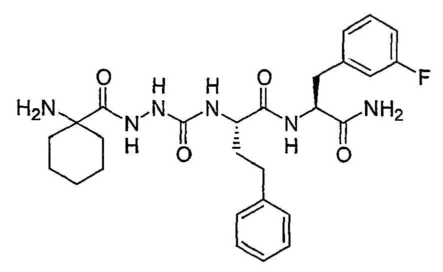

White solid. Yield: 16.9 mg (52%); HPLC: Rₜ = 4.21 min. (>99%); ¹H-NMR (DMSO-*d*₆/D₂O 10:1, 250 MHz) δ 7.33-6.95 (m, 9H), 4.50-4.44 (m, 1H), 4.13-4.07 (m, 1H), 3.09-3.02 (dd, *J* = 5.0, 13.8, 1H), 2.88-2.79 (dd, *J* = 9.7, 13.8, 1H), 2.53-2.44 (2H, partially under DMSO signal), 2.07-1.99 (m, 2H), 1.87-1.14 (m, 10H); ES-MS: mass calcd for C₂₇H₃₆FN₆O₄ 527.3 (MH⁺). Found *m*/*z* 527.0.

### EXAMPLE 6

### 1-(2S-2-Aminobutanoyl)-4-[2S-N-[2S-3-(indol-3-yl)propan-2-yl-amide]-3-phenylpropan-2-yl-amide]semicarbazide

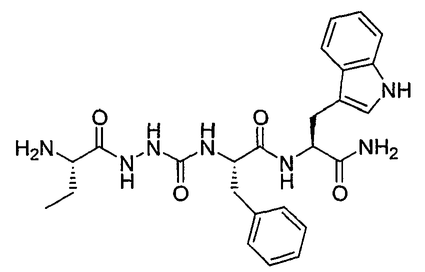

White solid. Yield: 13.3 mg (44%); HPLC: Rₜ = 3.64 min. (>99%); ¹H-NMR (D₂O, 250 MHz) δ 7.66-7.63 (d, *J* = 7.6, 1H), 7.52-7.49 (d, *J* = 8.0, 1H), 7.34-7.13 (m, 6H), 7.01-6.97 (m, 2H), 4.61-4.56 (m, 1H), 4.35-4.29 (t, *J* = 7.4, 1H), 4.02-3.96 (t, *J* = 6.6, 1H), 3.36-3.28 (dd, *J* = 5.9, 14.7, 1H), 3.19-3.10 (dd, *J* = 8.4, 14.7, 1 H), 2.88-2.77 (m, 2H), 1.99-1.87 (m, 2H), 1.04-0.98 (t, *J* = 7.6, 3H); ES-MS: mass calcd for C₂₅H₃₂N₇O₄ 494.2 (MH⁺). Found *m*/*z* 494.0.

### EXAMPLE 7

### 1-(2S-2-Aminopropanoyl)-4-[2S-N-[2S-3-(indol-3-yl)propan-2-yl-amide]-3-phenylpropan-2-yl-amide]semicarbazide

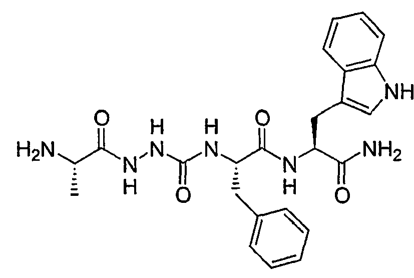

White solid. Yield: 13.1 mg (44%); HPLC: Rₜ = 3.56 min. (>99%); ¹H-NMR (D₂O, 250 MHz) δ 7.69-7.66 (d, *J* = 7.6, 1H), 7.42-7.39 (d, *J* = 7.9, 1H), 7.31-7.02 (m, 8H), 4.56-4.41 (m, 2H), 3.91-3.75 (1H, partially under water signal), 3.24-3.16 (dd, *J* = 5.3, 14.6, 1H), 3.09-2.99 (m, 2H), 2.86-2.77 (dd, *J* = 8.1, 13.8,1H), 1.44-1.41 (d, *J* = 7.0, 3H; minor isomeric signal observed); ES-MS: mass calcd for C₂₄H₃₀N₇O₄ 480.2 (MH⁺). Found *m*/*z* 480.1.

### EXAMPLE 8

### 1-(2S-3-Naphthyl-2-propanoyl)-4-[2S-N-[2S-3-(m-fluorophenyl)propan-2-yl-amide]3-methylbutan-2-yl-amide]semicarbazide

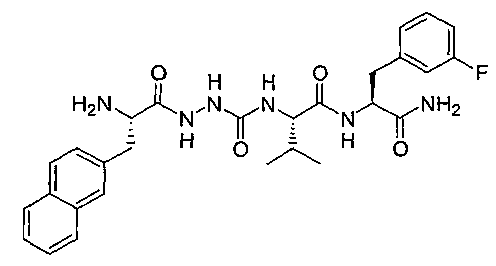

White solid. Yield: 20.5 mg (62%); HPLC: Rₜ = 4.11 min. (>95%); ¹H-NMR (DMSO-*d*₆/D₂O 10:1, 250 MHz) δ 7.92-7.81 (m, 4H), 7.34-7.31 (d, *J* = 7.9, 1H), 7.55-7.43 (m, 3H), 7.34-7.25 (m, 1H), 7.10-6.95 (m, 3H), 4.50-4.45 (m, 1H), 4.13-4.08 (m, 1H), 4.03-4.01 (d, *J* = 6.0, 1H), 3.36-3.28 (dd, *J* = 5.7, 14.4, 1H), 3.21-3.13 (dd, *J* = 7.5, 14.4, 1H), 3.06-2.95 (dd, *J* = 5.3, 13.8, 1H), 2.88-2.79 (dd, *J* = 9.4, 13.8, 1H), 1.99-1.86 (m, 1H), 0.80-0.71 (dd, *J* = 6.6, 6H); ES-MS: mass calcd for C₂₈H₃₄FN₆O₄ 537.3 (MH⁺). Found *m*/*z* 537.0.

### EXAMPLE 9

### 1-[2S-2-Amino-3-(4-pyridyl)propanoyl]-4-[2S-N-(2S-3-(indol-3-yl)propan-2-yl-amide)-butan-2-yl-amide]semicarbazide

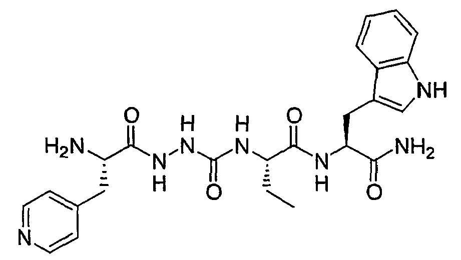

White solid. Yield: 13.8 mg (45%); HPLC: Rₜ = 2.91 min. (>99%); ¹H-NMR (DMSO-*d*₆/D₂O 10:1, 250 MHz) δ 8.79-8.76 (d, *J* = 6.6, 2H), 7.87-7.84 (d, *J* = 6.6, 2H), 7.62-7.59 (d, *J* = 7.5, 1H), 7.34-7.31 (d, *J* = 6.9, 1H), 7.13-6.95 (m, 3H), 4.51-4.45 (m, 1H), 4.20-4.14 (m, 1H), 4.09-4.04 (dd, *J* = 5.3, 7.5, 1H), 3.41-3.20 (m, 2H), 3.17-3.09 (dd, *J* = 5.3, 14.8, 1H), 3.01-2.92 (dd, *J* = 8.2, 14.8, 1H), 1.70-1.40 (m, 2H), 0.81-0.75 (t, *J* = 7.2, 3H); ES-MS: mass calcd for C₂₄H₃₀N₈O₄ 495.2 (MH⁺). Found *m*/*z* 495.0.

### EXAMPLE 10

### 1-[2S-2-Amino-3-(4-pyridyl)propanoyl]-4-[2S-N-(2S-3-(indol-3-yl)propan-2yl-amide)-2-phenylethan-2-yl-amide]semicarbazide

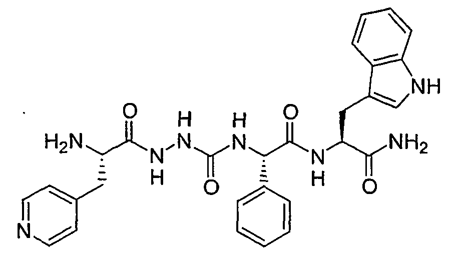

White solid. Yield: 18.9 mg (57%); HPLC: Rₜ = 3.26 min.(>36%)/3.36 min.(>63%); isobaric peaks; ¹H-NMR (DMSO-*d*₆/D₂O 10:1, 250 MHz) δ 8.80-8.73 (m, 2H), 7.89-7.84 (m, 2H), 7.62-7.54 (m, 1H), 7.36-6.88 (m, 9H), 5.38 (s, 0.5H), 5.32 (s, 0.5H), 4.53-4.37 (m, 1H), 4.21-4.15 (m, 1H), 3.42-2.86 (m, 4H); ES-MS: mass calcd for C₂₈H₃₁N₈O₄ 543.2 (MH⁺). Found *m*/*z* 543.0.

### EXAMPLE 11

### 1-(2S-2-Aminobutanoyl)-4-[2S-N-[2S-3-(m-fluorophenyl)propan-2-yl-amide]-4-phenylbutan-2-yl-amide]semicarbazide

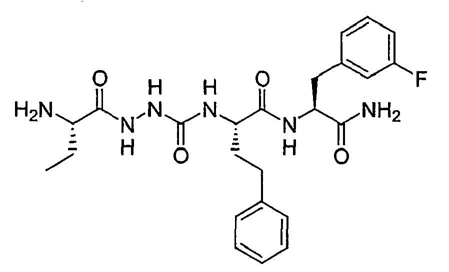

White solid. Yield: 21.6 mg (54%); HPLC: Rₜ = 4.17 min. (>99%); ¹H-NMR (D₂O, 250 MHz) δ 7.22-6.72 (m, 9H), 4.53-4.45 (m, 1H), 3.92-3.81 (m, 2H), 3.15-3.07 (dd, *J* = 5.3, 14.4, 1H), 2.85-2.75 (dd, *J* = 10.4, 14.4, 1H), 2.32-2.26 (t, *J* = 7.8, 2H), 1.88-1.59 (m, 4H), 0.92-0.81 (t, *J* = 7.5, 3H); ES-MS: mass calcd for C₂₄H₃₁FN₆O₄ 487.24 (MH⁺). Found *m*/*z* 487.2

### EXAMPLE 12

### 1-(2S-2-Aminobutanoyl)-4-[2S-N-[2S-3-(indol-3-yl)propan-2-yl-amide]-4-phenylbutan-2-yl-amide]semicarbazide

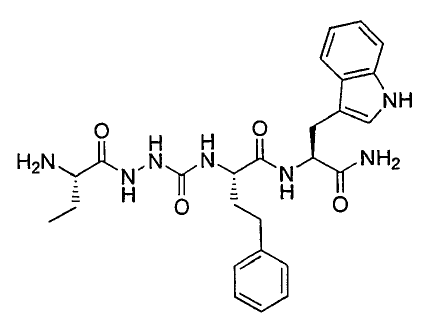

White solid. Yield: 14 mg (44%); HPLC: Rₜ = 4.03 min. (>98%); ¹H-NMR (CD₃OD, 250 MHz) δ 7.74-7.55 (d, *J* = 7.0, 1H), 7.33-7.00 (m, 8H), 4.79-4.70 (m, 1H), 4.17-4.12 (dd, *J* = 5.3, 8.2, 1H), 3.88-3.83 (t, *J* = 6.6, 1H), 3.43-3.32 (m, 1H, partially hidden under methanol signal), 3.22-3.12 (dd, *J* = 8.6, 14.7, 1H), 2.58-2.49 (t, *J* = 8.5, 2H), 2.05-1.69 (m, 4H), 1.14-1.08 (t, *J* = 7.5, 3H); ES-MS: mass calcd for C₂₆H₃₄N₇O₄ 508.3 (MH⁺). Found *m*/*z* 508.5.

### EXAMPLE 13

### 1-(2S-2-Aminobutanoyl)-4-[2S-N-[2S-3-(m-fluorophenyl)propan-2-yl-amide]-3-(indol-3-yl)propan-2-yl-amide]semicarbazide

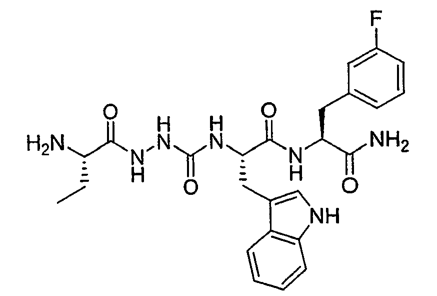

White solid. Yield: 13 mg (41%); HPLC: Rₜ = 3.97 min. (>99%); ¹H-NMR (CD₃OD, 250 MHz) δ 7.73-7.70 (d, *J* = 7.3, 1H), 7.49-7.03 (m, 8H), 4.75-4.68 (m, 1H), 4.63-4.58 (t, *J* = 7.0, 1H), 3.93-3.88 (m, 1H), 3.35-3.17 (m, 3H), 3.04-2.95 (dd, *J* = 8.4, 13.8, 1H), 2.11-1.92 (m, 2H), 1.23-1.15 (t, *J* = 7.5, 3H); ES-MS: mass calcd for C₂₅H₃₁FN₇O₄512.3 (MH⁺). Found *m*/*z* 512.4

### EXAMPLE 14

### 1-(2S-2-Aminobutanoyl)-4-[2S-N-[2S-3-(m-methoxyphenyl)propan-2-yl-amide]-3-(p-chlorophenyl)propan-2-yl-amide]semicarbazide

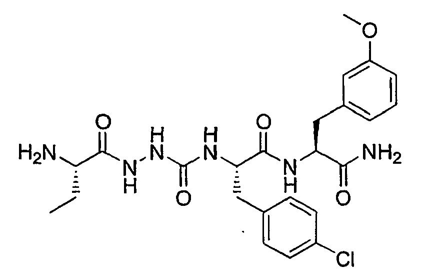

White solid. Yield: 12 mg (38%); HPLC: Rₜ = 4.14 min. (>96%); ¹H-NMR (CD₃OD, 250 MHz; one signal hidden under water peak) δ 7.41-7.18 (m, 5H), 6.98-6.89 (m, 4H), 4.78-4.70 (m, 1H), 4.62-4.44 (dd, *J* = 5.6, 7.8, 1H), 3.33-2.91 (m, 4H), 2.17-1.88 (m, 2H), 1.29-1.18 (t, *J* = 7.6, 3H); ES-MS: mass calcd for C₂₄H₃₂ClN₆O₅ 519.2 (MH⁺). Found *m*/*z* 519.4.

### EXAMPLE 15

### 1-(2S-2-Aminopentanoyl)-4-[2S-N-[2S-3-(m-methoxyphenyl)propan-2-yl-amide]-3-(p-chlorophenyl)propan-2-yl-amide]semicarbazide

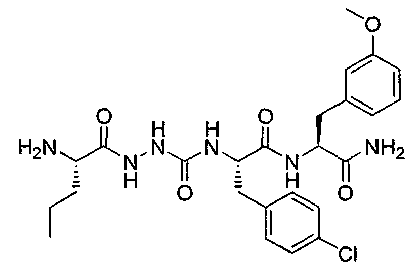

White solid. Yield: 18 mg (33%); HPLC: Rₜ = 4.21 min. (>98%); ES-MS: mass calcd for C₂₅H₃₄ClN₆O₅, 533.2 (MH⁺). Found *m*/*z* 533.4.

### EXAMPLE 16

### 1-(2S-2-amino-4-phenylbutanoyl)-4-[2S-N-[2S-3-(1,1'-biphenyl-4-yl)propan-2-yl-amide]-3-phenylpropan-2-yl-amide]semicarbazide

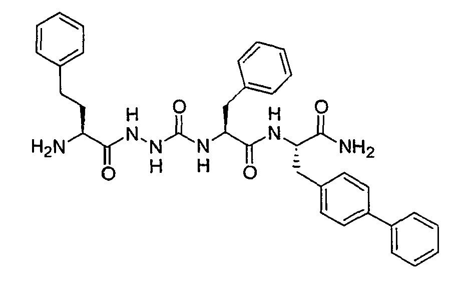

### EXAMPLE 17

### 1-(2S-2-aminopentanoyl)-4-[2S-N-[2S-3-(m-methoxyphenyl)propan-2-yl-amide]-3-(1,1'-biphenyl-4-yl)propan2-yl-amide]semicarbazide

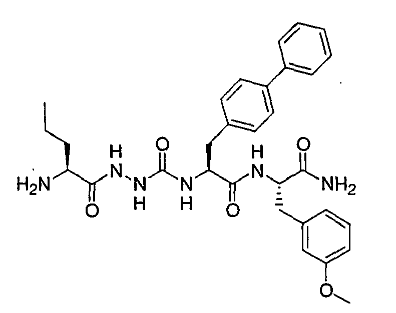

### EXAMPLE 18

### 1-(2S-2-aminobutanoyl)-4-[2S-N-[2S-3-(1,1'-biphenyl-4-yl)propan-2-yl-amide]-3-(p-hydroxyphenyl)propan-2-yl-amide]semicarbazide

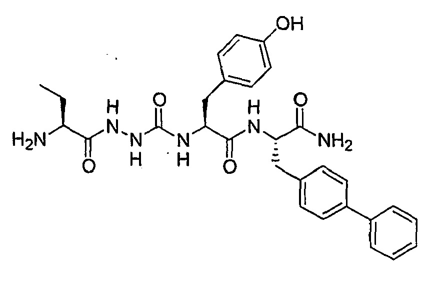

### EXAMPLE 19

### 1-(2S-2-aminobutanoyl)-4-[2S-N-[2S-4-methylpentan-2-yl-amide]-3-(benzo[b]thiophen-3-yl)propan-2-yl-amide]semicarbazide

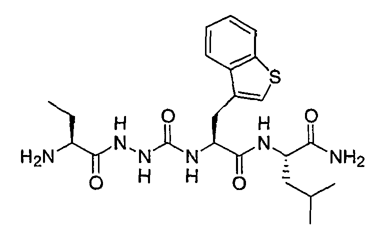

### EXAMPLE 20

### 1-(2S-2-Aminobutanoyl)-4-[2S-N-[2S-3-(2-naphthyl)propan-2-yl-amide]-4-phenylbutan-2-yl-amide]semicarbazide

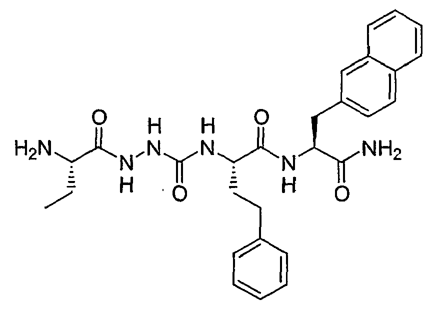

### EXAMPLE 21

### 1-(2S-2-Aminobutanoyl)-4-[2S-N-[2S-3-(m-methoxyphenyl)propan-2-yl-amide]-4-phenyl]butan-2-yl-amide]semicarbazide

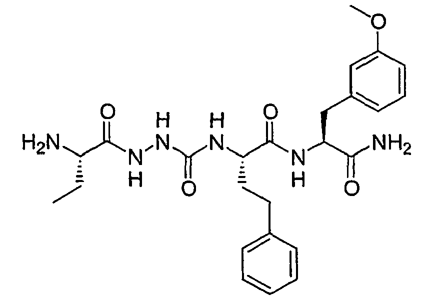

### EXAMPLE 22

### 1-(2S-2-Aminobutanoyl)-4-[2S-N-[2S-3-(benzo[b]thiophen-3-yl)propan-2-yl-amide]-4-phenylbutan-2-yl-amide]semicarbazide

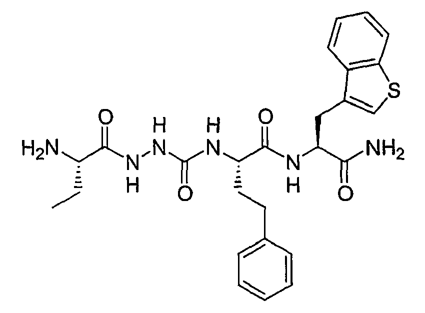

### EXAMPLE 23

### 1-(2S-2-Aminobutanoyl)-4-[2S-N-[2S-3-(p-chlorophenyl)propan-2-yl-amide]-4-phenylbutan-2-yl-amide]semicarbazide

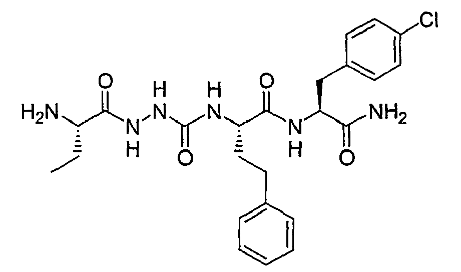

### EXAMPLE 24

### 1-(2S-2-Aminopentanoyl)-4[2S-N-[2S-3-(2-naphthyl)propan-2-yl-amide]-4-phenylbutan-2-yl-amide]semicarbazide

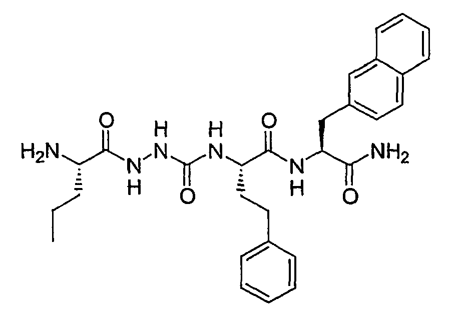

### EXAMPLE 25

### 1-(2S-2-Aminobutanoyl)-4-[2S-N-[2S-3-(m-fluorophenyl)propan-2-yl-amide]-3-(2-naphthyl)propan-2-yl-amide]semicarbazide

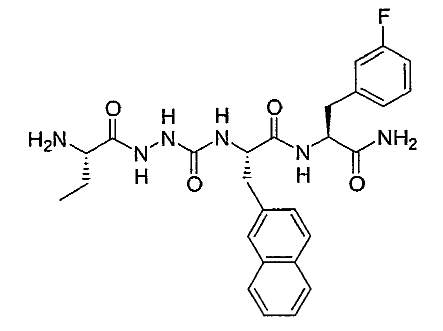

### EXAMPLE 26

### 1-(2S-2-Aminobutanoyl)-4-[2S-N-[2S-3-(m-fluorophenyl)propan-2-yl-amide]-3-(1,1'-biphenyl-4-yl)propan-2-yl-amide]semicarbazide

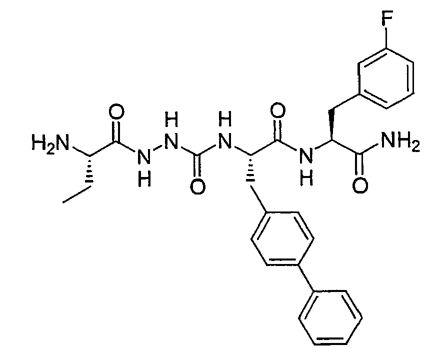

### EXAMPLE 27

### 1-(2S-2-Aminopropionyl)-4-(2-phenylethyl)semicarbazide

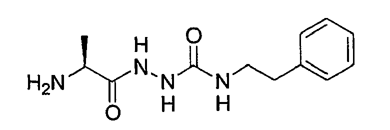

Scheme 2: a) Preparation of *N*^{α}*-*Boc-L-alaninehydrazide: CDI (4.71 g, 29.1 mmol) was added slowly to a solution of *N*^{α}-Boc-L-alanine (5.0 g, 26.4 mmol) in 50 mL dry THF. After addition the solution was stirred for 30 min. Then, hydrazine monohydrate (3.85 mL, 79.3 mmol) was added and the resulting solution was stirred overnight. The reaction mixture was concentrated and the white solid was dissolved in 1,4-dioxane and lyophilized overnight. The crude product was suspended in diethyl ether, cooled to -18 °C and filtered. The filtrate was dried *in vacuo* overnight yielding 5.6 g (104%) of crude product. The solid was a mixture of imidazole and desired product (imidazole:product 2:1, determined by ¹H-NMR) and was used without further purification. ¹H-NMR (DMSO-*d*₆ exchanged with D₂O, 250 MHz) δ 3.96-3.85 (m, 1 H), 1.39-1.31 (s, 6H), 1.18-1.10 (d, *J* = 7.2, 3H)
b) CDI (44 mg, 0.27 mmol) was added to a solution of *N*^{α}-Boc-L-alaninehydrazide (59.9 mg, 0.295 mmol) in 1.0 mL dry THF. The resulting solution was stirred overnight and 2-phenylethylamine (0.27 mmol) dissolved in 0.5 mL dry THF was added. The reaction mixture was stirred overnight, and then concentrated. The residue was treated with 1 mL 95% TFA/H₂O for 5 min, and concentrated followed by purification by preparative HPLC. The title compound was obtained as a white solid. Yield: 45 mg (66%); HPLC: Rₜ = 3.11 min. (>99%); ¹H-NMR (D₂O, 250 MHz) δ (rotamerism observed) 7.37-7.21 (m, 5H), 4.10-4.02 (q, *J* = 7.2, 1H), 3.40-3.31 (td, *J* = 6.9, 2.51, 2H), 2.79-2.72 (t, *J* = 6.9, 2H), 1.50-1.45 (d, *J* = 7.2, 3H); ES-MS: mass calcd for C₁₂H₁₈N₄O₂ 251.1 (MH⁺). Found *m*/*z* 251.0.

### EXAMPLE 28

### 1-(2S-2-Aminopropionyl)-4-(2-phenylethyl)-4-methylsemicarbazide

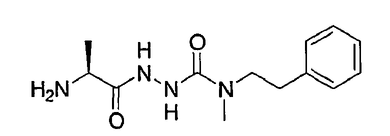

Scheme 2: Following the general procedure of Example 27, except substituting 2-phenylethylamine for *N*-methylphenethylamine. The title compound was obtained as a white solid. Yield: 29 mg (41%); HPLC: Rₜ= 3.17 min. (>99%); ¹H-NMR (D₂O, 250 MHz) δ (rotamerism observed) 7.37-7.20 (m, 5H), 4.15-4.04 (q, *J* = 7.2, 1H), 3.54-3.45 (m, 2H), 2.87-2.75 (m, 5H), 1.54-1.49 (d, *J* = 7.2, 3H); ES-MS: mass calcd for C₁₃H₂₁N₄O₂ 265.2 (MH⁺). Found *m*/*z* 265.0.

### EXAMPLE 29

### 1-(2S-2-Aminopropionyl)-4-[2-(p-hydroxyphenyl)ethyl]semicarbazide

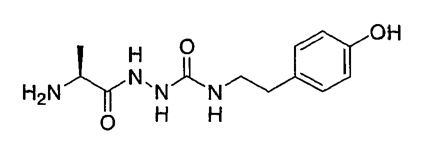

Scheme 2: Following the general procedure of Example 27, except substituting 2-phenylethylamine for tyramine (dissolved in DMF). The title compound was obtained as a white solid. Yield: 20 mg (28%); HPLC: Rₜ = 2.46 min. (>99%); ¹H-NMR (D₂O, 250 MHz) δ (rotamerism observed) 7.15-7.07 (m, 2H), 6.84-6.76 (m, 2H), 4.10-4.01 (q, *J* = 7.2, 1H), 3.35-3.25 (td, *J* = 6.9, 3.14, 2H), 2.71-2.63 (t, 6.9, 2H), 1.50-1.43 (d, *J* = 7.2, 3H); ES-MS: mass calcd for C₁₂H₁₉N₄O₃ 267.1 (MH⁺). Found *m*/*z* 267.0.

### EXAMPLE 30

### 1-(2S-2-Aminopropionyl)-4-benzylsemicarbazide

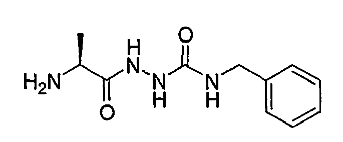

Scheme 2: Following the general procedure of Example 27, except substituting 2-phenylethylamine for benzylamine. The title compound was obtained as a white solid. Yield: 23 mg (36%); HPLC: Rₜ= 2.57 min. (>99%); ¹H-NMR (D₂O, 250 MHz) δ (rotamerism observed) 7.40-7.23 (m, 5H), 4.34-4.29 (s, 2H), 4.15-4.05 (q, *J* = 7.2, 1 H), 1.55-1.49 (d, *J* = 7.2, 3H); ES-MS: mass calcd for C₁₁H₁₇N₄O₂ 237.1 (MH⁺). Found *m*/*z* 237.0.

### EXAMPLE 31

### 1-(2S-2-Aminopropionyl)-4-(o-chlorobenzyl)semicarbazide

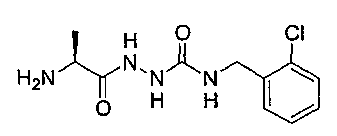

Scheme 2: Following the general procedure of Example 27, except substituting 2-phenylethylamine for *o*-chlorobenzylamine. The title compound was obtained as a white solid. Yield: 24 mg (33%); HPLC: Rₜ = 3.02 min. (>99%);¹H-NMR (D₂O, 250 MHz) δ (rotamerism observed) 7.23-7.01 (m, 4H), 4.20-4.15 (s, 2H), 3.94-3.84 (q, *J* = 7.2, 1H), 1.35-1.27 (d, *J* = 7.2, 3H); ES-MS: mass calcd for C₁₁H₁₆Cl₄O₂ 271.1 (MH⁺). Found *m*/*z* 271.0.

### EXAMPLE 32

### 1-(2S-2-Aminopropionyl)-4-(2-furfuryl)semicarbazide

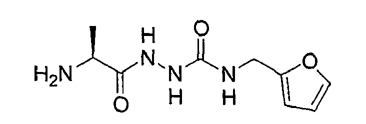

Scheme 2: Following the general procedure of Example 27, except substituting 2-phenylethylamine for furfurylamine. The title compound was obtained as a white solid. Yield: 6 mg (10%); HPLC (15 min. gradient): Rₜ= 1.80 min. (>99%); ¹H-NMR (D₂O, 250 MHz) δ (rotamerism observed) 7.44-7.37 (m, 1H), 6.39-6.34 (m, 1H), 6.26-6.22 (m, 1H), 4.30-4.26 (s, 2H), 4.16-4.05 (q, *J* = 7.2, 1H), 1.55-1.47 (d, *J* = 7.2, 3H); ES-MS: mass calcd for C₉H₁₅N₄O₃ 227.1 (MH⁺). Found *m*/*z* 227.0.

### EXAMPLE 33

### 1-(2S-2-Aminopropionyl)-4-[2-(3-indolyl)ethyl]semicarbazide

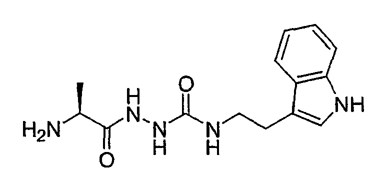

Scheme 2: Following the general procedure of Example 27, except substituting 2-phenylethylamine for tryptamine. The following changes were applied: CDI (22 mg, 0.14 mmol), *N*^{α}-Boc-L-alaninehydrazide (45 mg, 0.22 mmol) in 0.5 mL dry THF, tryptamine (22 mg, 0.14 mmol) in 100 µL DMF. The title compound was obtained as a white solid. Yield: 13 mg (17%); HPLC: Rₜ = 3.11 min. (>99%); ¹H-NMR (D₂O, 250 MHz) δ (rotamerism observed) 7.68-7.61 (m, 1H), 7.48-7.42 (m, 1H), 7.27-7.06 (m, 3H), 4.09-3.97 (q, *J* = 7.2, 1H), 3.46-3.37 (td, *J* = 6.9, 3.14, 2H), 2.95-2.87 (t, *J* = 6.9, 2H), 1.48-1.41 (d, *J* = 7.2, 3H); ES-MS: mass calcd for C₁₄H₂ON₅O₂ 290.2 (MH⁺). Found *m*/*z* 290.2.

### EXAMPLE 34

### 1-(2S-2-Aminopropionyl)-4-(1-naphthylmethyl)semicarbazide

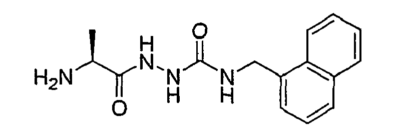

Scheme 2: Following the general procedure of Example 27, except substituting 2-phenylethylamine for 1-naphthylmethylamine. The title compound was obtained as a white solid. Yield: 42 mg (55%); HPLC: Rₜ = 3.50 min. (>97%);¹H-NMR (D₂O, 250 MHz) δ (rotamerism observed) 8.03-7.81 (m, 3H), 7.63-7.40 (m, 4H), 4.79-4.74 (s, 2H), 4.13-4.03 (q, *J* = 7.2, 1H), 1.52-1.45 (d, *J* = 7.2, 3H); ES-MS: mass calcd for C₁₅H₁₉N₄O₂ 287. (MH⁺). Found *m*/*z* 287.0.

### EXAMPLE 35

### 1-(2S-2-Aminopropionyl)-4-piperonylsemicarbazide

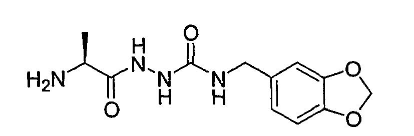

Scheme 2: Following the general procedure of Example 27, except substituting 2-phenylethylamine for piperonylamine. The title compound was obtained as a white solid. Yield: 14 mg (19%); HPLC: Rₜ = 2.70 min. (>95%); ¹H-NMR (D₂O, 250 MHz) δ (rotamerism observed) 6.85-6.72 (m, 3H), 5.91-5.88 (s, 2H), 4.23-4.20 (s, 2H), 4.16-4.05 (q, *J* = 7.2, 1H), 1.55-1.48 (d, *J* = 7.2, 3H); ES-MS: mass calcd for C₁₂H₁₇N₄O₄ 281.1 (MH⁺). Found *m*/*z* 280.9.

### EXAMPLE 36

### 1-(2S-2-Aminopropionyl)-4-(p-chlorobenzyl)semicarbazide

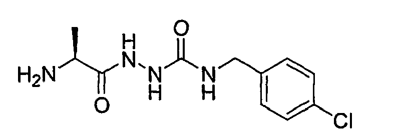

Scheme 2: Following the general procedure of Example 27, except substituting 2-phenylethylamine for p-chlorobenzylamine. The following changes were applied: CDI (44 mg, 0.27 mmol), *N*^{α}-Boc-L-alaninehydrazide (90 mg, 0.25 mmol), 4-chlorobenzylamine (35 µL, 0.44 mmol). The title compound was obtained as a white solid. Yield: 52 mg (71%); HPLC: Rₜ = 3.30 min. (>97%); ¹H-NMR (D₂O, 250 MHz) δ (rotamerism observed) 7.37-7.30 (m, 2H), 7.27-7.19 (m, 2H), 4.31-4.26 (s, 2H), 4.16-4.05 (q, *J* = 7.2, 1H), 1.55-1.49 (d, *J* = 7.2, 3H); ES-MS: mass calcd for C₁₁H₁₆ClN₄O₂ 271.1 (MH⁺). Found *m*/*z* 271.1.

### EXAMPLE 37

### 1-(2S-2-Aminopropionyl)-4-[2-(thiophen-2-yl)ethyl]semicarbazide

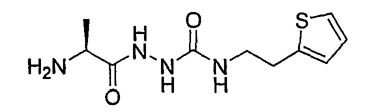

Scheme 2: Following the general procedure of Example 27, except substituting 2-phenylethylamine for 2-(thiophen-2-yl)ethylamine. The following changes were applied: CDI (44 mg, 0.27 mmol), *N*^{α}-Boc-L-alaninehydrazide (90 mg, 0.44 mmol), 2-thiopheneethylamine (35 µL, 0.27 mmol). The title compound was obtained as a white solid. Yield: 47 mg (67%); HPLC: Rₜ = 2.72 min. (>91%); ¹H-NMR (D₂O, 250 MHz) δ (rotamerism observed) 7.28-7.22 (m, 1H), 7.00-6.94 (m, 1H), 6.90-6.86 (m, 1H), 4.12-4.02 (q, *J* = 7.2, 1H), 3.45-3.32 (td, *J* = 6.5, 3.5, 2H), 3.03-2.93 (t, *J* = 6.6, 2H), 1.52-1.47 (d, *J* = 7.2, 3H); ES-MS: mass calcd for C₁₀H₁₇N₄O₂S 257.1 (MH⁺). Found *m*/*z* 257.2.

### EXAMPLE 38

### 1-(2S-2-Aminopropionyl)-4-(m-fluorobenzyl)semicarbazide

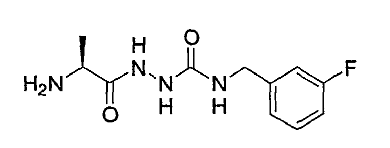

Scheme 2: Following the general procedure of example 27, except substituting 2-phenylethylamine for 3-fluorobenzylamine. The following changes were applied: CDI (44 mg, 0.27 mmol, 1.1 eq.), *N*^{α}-Boc-L-alanine (50 mg, 0.25 mmol, 1.0 eq.), 3-fluorobenzylamine (31 µL, 0.27 mmol, 1.1 eq.). Yield: 34 mg (55%); HPLC: Rₜ = 2.71 min. (>99%); ¹H-NMR (D₂O, 250 MHz) δ (rotamerism observed) 7.42-7.27 (m, 1H), 7.11-6.94 (m, 3H), 4.34-4.29 (s, 2H), 4.17-4.01 (m, 1H), 1.61-1.53 (d, *J* = 7.2, 3H); ES-MS: mass calcd for C₁₁H₁₅FN₄O₂ 255.1 (MH⁺). Found *m*/*z* 255.1.

### EXAMPLE 39

### 1-(2S-2-Aminopropionyl)-4-(o-fluorobenzyl)semicarbazide

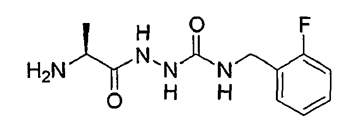

Scheme 2: Following the general procedure of example 27, except substituting 2-phenylethylamine for 2-fluorobenzylamine. The following changes were applied: CDI (44 mg, 0.27 mmol, 1.1 eq.), *N*^{α}-Boc-L-alanine (50 mg, 0.25 mmol, 1.0 eq.), 2-fluorobenzylamine (31 µL, 0.27 mmol, 1.1 eq.). Yield: 27 mg (44%); HPLC: Rₜ = 2.59 min. (>99%); ¹H-NMR (D₂O, 250 MHz) δ (rotamerism observed) 7.38-7.28 (m, 2H), 7.22-7.07 (m, 2H), 4.41-4.37 (s, 2H), 4.21-4.08 (m, 1H), 1.61-1.53 (d, *J* = 7.2, 3H); ES-MS: mass calcd for C₁₁H₁₅FN₄O₂ 255.1 (MH⁺). Found *m*/*z* 255.1.

### EXAMPLE 40

### 1-(2S-2-Aminopropionyl)-4-(p-fluorobenzyl)semicarbazide

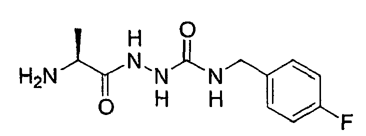

Scheme 2: Following the general procedure of example 27, except substituting 2-phenylethylamine for 4-fluorobenzylamine. The following changes were applied: CDI (44 mg, 0.27 mmol, 1.1 eq.), *N*^{α}-Boc-L-alanine (50 mg, 0.25 mmol, 1.0 eq.), 4-fluorobenzylamine (31 µL, 0.27 mmol, 1.1 eq.). Yield: 39 mg (62%); HPLC: Rₜ = 2.70 min. (>99%); ¹H-NMR (D₂O, 250 MHz) δ (rotamerism observed) 7.39-7.31 (m, 2H), 7.22-7.10 (m, 2H), 4.40-4.33 (s, 2H), 4.28-4.15 (m, 1H), 1.61-1.53 (d, *J* = 7.2, 3H); ES-MS: mass calcd for C₁₁H₁₅FN₄O₂ 255.1 (MH⁺). Found *m*/*z* 255.1.

### EXAMPLE 41

### 1-(2S-2-Aminopropionyl)-4-(o-trifluoromethylbenzyl)semicarbazide

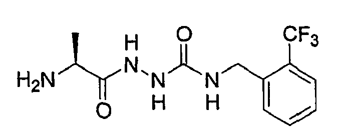

Scheme 2: Following the general procedure of example 27, except substituting 2-phenylethylamine for 2-trifluoromethylbenzylamine. The following changes were applied: CDI (44 mg, 0.27 mmol, 1.1 eq.), *N*^{α}-Boc-L-alanine (50 mg, 0.25 mmol, 1.0 eq.), 2-trifluoromethylbenzylamine (38 µL, 0.27 mmol, 1.1 eq.). Yield: 31 mg (41 %); HPLC: Rₜ = 3.37 min. (>98%); ¹H-NMR (D₂O, 250 MHz) δ (rotamerism observed) 7.85-7.46 (m, 4H), 4.54-4.52 (s, 2H), 4.22-4.08 (q, *J* = 7.2, 1H), 1.61-1.53 (d, *J* = 7.2, 3H); ES-MS: mass calcd for C₁₂H₁₅F₃N₄O₂ 305.1 (MH⁺). Found *m*/*z* 305.1.

### EXAMPLE 42

### 1-(2S-2-Aminopropionyl)-4-(m-chlorobenzyl)semicarbazide

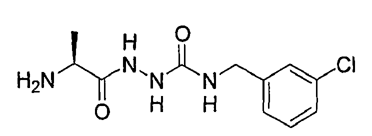

Scheme 2: Following the general procedure of example 27, except substituting 2-phenylethylamine for 3-chlorobenzylamine. The following changes were applied: CDI (44 mg, 0.27 mmol, 1.1 eq.), *N*^{α}-Boc-L-alanine (50 mg, 0.25 mmol, 1.0 eq.), 3-chlorobenzylamine (34 µL, 0.27 mmol, 1.1 eq.). Yield: 47 mg (70%); HPLC: Rₜ = 3.17 min. (>98%); ¹H-NMR (D₂O, 250 MHz) δ (rotamerism observed) 7.31-7.21 (m, 3H), 7.18-7.11 (m, 1H), 4.31-4.26 (s, 2H), 4.16-4.05 (m, *J* = 7.2, 1H), 1.61-1.53 (d, *J* = 7.2, 3H); ES-MS: mass calcd for C₁₁H₁₅ClN₄O₂ 271.1 (MH⁺). Found *m*/*z* 271.1.

### EXAMPLE 43

### 1-(2S-2-Aminopropionyl)-4-(o-methoxybenzyl)semicarbazide

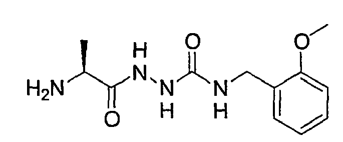

Scheme 2: Following the general procedure of example 27, except substituting 2-phenylethylamine for 2-methoxybenzylamine. The following changes were applied: CDI (44 mg, 0.27 mmol, 1.1 eq.), *N*^{α}-Boc-L-alanine (50 mg, 0.25 mmol, 1.0 eq.), 2-methoxybenzylamine (36 µL, 0.27 mmol, 1.1 eq.). Yield: 35 mg (53%); HPLC: Rₜ = 2.91 min. (>97%);¹H-NMR (D₂O, 250 MHz) δ (rotamerism observed) 7.33-7.21 (m, 2H), 7.09-6.99 (m, 2H), 4.31-4.28 (s, 2H), 4.16-4.05 (m, 1H), 1.61-1.53 (d, *J* = 7.2, 3H); ES-MS: mass calcd for C₁₂H₁₈N₄O₃ 267.1 (MH⁺). Found *m*/*z* 267.1.

### EXAMPLE 44

### 1-(2S-2-Aminopropionyl)-4-(o-methylbenzyl)semicarbazide

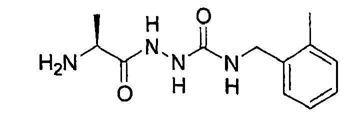

Scheme 2: Following the general procedure of example 27, except substituting 2-phenylethylamine for 2-methylbenzylamine. The following changes were applied: CDI (44 mg, 0.27 mmol, 1.1 eq.), *N*^{α}-Boc-L-alanine (50 mg, 0.25 mmol, 1.0 eq.), 2-methylbenzylamine (34 µL, 0.27 mmol, 1.1 eq.). Yield: 41 mg (66%); HPLC: Rₜ = 2.98 min. (>98%); ¹H-NMR (D₂O, 250 MHz) δ (rotamerism observed) 7.33-7.25 (s, 4H), 4.31-4.26 (s, 2H), 4.25-4.14 (m, 1H), 2.35-2.29 (s, 3H), 1.61-1.53 (d, *J* = 7.2, 3H); ES-MS: mass calcd for C₁₂H₁₈N₄O₂ 251.1 (MH⁺). Found *m*/*z* 251.2.

### EXAMPLE 45

### 1-(2S-2-Aminopropionyl)-4-(alpha-methylbenzyl)semicarbazide

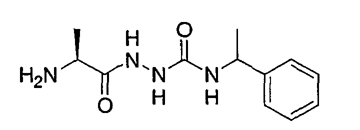

Scheme 2: Following the general procedure of example 27, except substituting 2-phenylethylamine for (+/-)-alpha-methylbenzylamine. The following changes were applied: CDI (44 mg, 0.27 mmol, 1.1 eq.), *N*^{α}-Boc-L-alanine (50 mg, 0.25 mmol, 1.0 eq.), (+/-)-alpha-methylbenzylamine (35 µL, 0.27 mmol, 1.1 eq.). Yield: 20 mg (33%); HPLC: Rₜ = 2.91 min. (>92%); ¹H-NMR (D₂O, 250 MHz) δ (rotamerism observed) 7.51-7.31 (m, 5H), 4.90-4.80 (m, 1H), 4.25-4.14 (m, 1H), 1.61-1.55 (d, *J* = 7.2, 3H), 1.51-1.45 (d, *J* = 6.91, 3H); ES-MS: mass calcd for C₁₂H₁₈N₄O₂ 251.1 (MH⁺). Found *m*/*z* 251.2.

### EXAMPLE 46

### 1-(2S-2-Aminobutanoyl)-4-(o-chlorobenzyl)semicarbazide

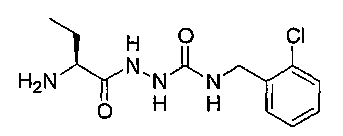

Scheme 2 without the isolation of compound II: CDI (45 mg, 0.28 mmol, 1.1 eq.) was added slowly to a solution of *N*^{α}-Boc-L-aminobutyric acid (51 mg, 0.25 mmol, 1.0 eq.) in 1.0 mL dry THF. After addition the solution was stirred for 2 h. Then, hydrazine monohydrate (13.4 µL, 0.28 mmol, 1.1 eq.) was added and the resulting solution was stirred overnight. CDI (45 mg, 0.28 mmol, 1.1 eq.) was added and the solution was stirred for 6 h. 2-Chlorobenzylamine (27 µL, 0.23 mmol, 0.9 eq.) was added and the reaction mixture was stirred overnight, and concentrated. The residue was treated with 1 mL 95% TFA/H₂O for 2 h, and concentrated followed by purification on preparative HPLC. Yield: 9.4 mg (13%); HPLC: Rₜ = 3.39 min. (>99%); ¹H-NMR (CD₃OD, 250 MHz) δ (rotamerism observed) 7.41-7.28 (m, 2H), 7.26-7.13 (m, 2H), 4.44-4.37 (s, 2H), 3.80-3.71 (t, *J* = 6.59, 1H), 1.96-1.79 (m, 2H), 1.07-0.95 (t, *J* = 7.54, 3H); ES-MS: mass calcd for C₁₂H₁₇ClN₄O₂ 285.1 (MH⁺). Found *m*/*z* 285.1.

### EXAMPLE 47

### 1-(2S-2-Aminovaleryl)-4-(o-chlorobenzyl)semicarbazide

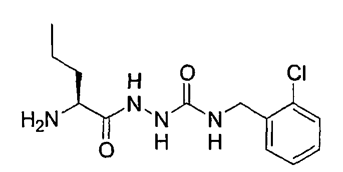

Scheme 2: Following the general procedure of example 46, except substituting *N*^{α}-Boc-L-aminobutyric acid for *N*^{α}-Boc-L-aminovaleric acid. Yield: 11.7 mg (16%); HPLC: Rₜ = 3.36 min. (>99%); ¹H-NMR (CD₃OD, 250 MHz) δ (rotamerism observed) 7.41-7.28 (m, 2H), 7.26-7.13 (m, 2H), 4.44-4.37 (s, 2H), 3.80-3.71 (t, *J* = 6.59, 1H), 1.93-1.66 (m, 2H), 1.49-1.34 (m, 2H), 0.98-0.86 (t, *J* = 7.22, 3H); ES-MS: mass calcd for C₁₃H₁₉ClN₄O₂ 299.1 (MH⁺). Found *m*/*z* 299.1.

### EXAMPLE 48

### 1-(2S-2-Aminohexanoyl)-4-(o-chlorobenzyl)semicarbazide

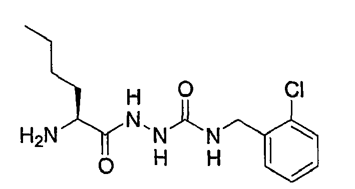

Scheme 2: Following the general procedure of example 46, except substituting *N*^{α}-Boc-L-aminobutyric acid for *N*^{α}-Boc-L-aminocaproic acid. Yield: 30 mg (38%); HPLC: Rₜ = 3.46 min. (>99%); ¹H-NMR (CD₃OD, 250 MHz) δ (rotamerism observed) 7.41-7.28 (m, 2H), 7.26-7.13 (m, 2H), 4.44-4.37 (s, 2H), 3.80-3.71 (t, *J* = 6.59, 1H), 1.89-1.68 (m, 2H), 1.43-1.21 (m, 4H), 0.94-0.82 (t, *J* = 7.22, 3H); ES-MS: mass calcd for C₁₄H₂₁ClN₄O₂ 313.1 (MH⁺). Found *m*/*z* 313.1.

### EXAMPLE 49

### 1-(2S-2-Aminoisocaproyl)-4-(o-chlorobenzyl)semicarbazide

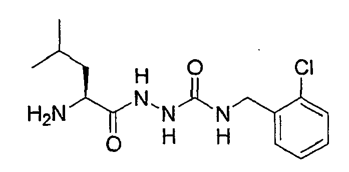

Scheme 2: Following the general procedure of example 46, except substituting *N*^{α}-Boc-L-aminobutyric acid for *N*^{α}-Boc-L-aminoisocaproic acid. Yield: 17.8 mg (23%); HPLC: Rₜ = 3.45 min. (>99%); ¹H-NMR (CD₃OD, 250 MHz) δ (rotamerism observed) 7.41-7.28 (m, 2H), 7.26-7.13 (m, 2H), 4.44-4.37 (s, 2H), 3.80-3.71 (t, *J* = 6.59, 1H), 1.77-1.57 (m, 3H), 0.98-0.90 (m, 6H); ES-MS: mass calcd for C₁₄H₂₁ClN₄O₂ 313.1 (MH⁺). Found *m*/*z* 313.1.

### EXAMPLE 50

### 1-(1-Aminocyclohexane-1-carbonyl)-4-(o-chlorobenzyl)semicarbazide

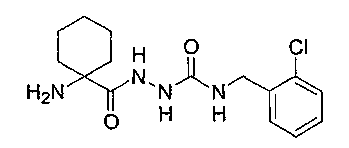

Scheme 2: Following the general procedure of example 46, except substituting *N*^{α}-Boc-L-aminobutyric acid for Boc-1-amino-1-cyclohexanoic acid. Yield: 3.8 mg (5%); HPLC: Rₜ = 3.42 min. (>98%); ¹H-NMR (CD₃OD, 250 MHz) δ (rotamerism observed) 7.41-7.28 (m, 2H), 7.26-7.13 (m, 2H), 4.44-4.37 (s, 2H), 2.19-1.20 (m, 10H); ES-MS: mass calcd for C₁₅H₂₁ClN₄O₂325.1 (MH⁺). Found *m*/*z* 325.1.

### EXAMPLE 51

### 1-(2S-2-Amino-3-phenyl-propionyl)-4-(o-chlorobenzyl)semicarbazide

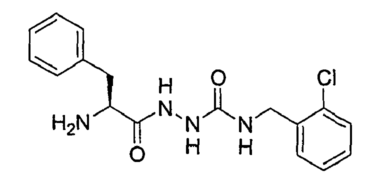

Scheme 2: Following the general procedure of example 46, except substituting *N*^{α}-Boc-L-aminobutyric acid for *N*^{α}-Boc-L-phenylalanine. Yield: 15.6 mg (18%); HPLC: Rₜ = 3.71 min. (>97%); ¹H-NMR (CD₃OD, 250 MHz) δ (rotamerism observed) 7.38-7.14 (m, 9H), 4.44-4.37 (s, 2H), 3.80-3.61 (m, 3H); ES-MS: mass calcd for C₁₇H₁₉ClN₄O₂ 347.1 (MH⁺). Found *m*/*z* 347.1.

### EXAMPLE 52

### 1,1-(2S-1,4-cyclo-Aminobutanoyl)-4-(o-chlorobenzyl)semicarbazide

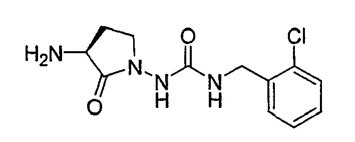

Scheme 3:
Step a) Preparation of Cbz-L-Met-NHNHBoc:
   Cbz-L-methionine (5 g, 17.65 mmol) was dissolved in dry THF (20 mL) and cooled in an ice-bath. CDI (3.15 g, 19.42 mmol) was added in one portion, resulting in vigorous evolution of gas. After 20 min. BocNHNH₂ (2.57 g, 19.42 mmol) was added and the resulting solution was stirred for a period of 20 h, at which time it was poured into water (150 mL) and extracted with EA (x3). The combined organics were washed once with sat. aq. NaHCO₃ 10% aq. citric acid and brine. Drying over MgSO₄ followed by evaporation gave an oil which was dried *in vacuo* to give a white solid. Yield: 6.75 g (96%); HPLC: Rₜ = 5.00 (>99%); ¹H-NMR (CDCl₃, 250 MHz) δ 9.00 (br, 1H), 7.29 (m, 5H), 7.27 (br, 1H), 6.15-6.11 (d, *J* = 8.4, 1H), 5.12-4.98 (m, 2H), 4.45-4.40 (m, 1H), 2.57-2.52 (t, *J* = 7.1, 2H), 2.13-1.89 (m, 5H), 1.42 (s, 9H); ¹³C-NMR (CDCl₃, 250 MHz) δ 171.7, 156.7, 155.7, 136.5, 128.9, 128.5, 128.4, 81.9, 67.5, 52.8, 32.3, 30.3, 28.5, 15.6; ES-MS: mass calcd for C₁₈H₂₈N₃O₅S 398.2 (MH⁺). Found *m*/*z* 398.1.
Step b) Preparation of Cbz-L-Met-NHNHBoc methyl sulfonium iodide:
   Cbz-L-Met-NHNHBoc (6.65 g, 16.73 mmol) was stirred in methyl iodide (25 mL) at rt for a period of 72 h. DCM (50 mL) was added and the yellow solution was concentrated to give a yellow oil, which was dried *in vacuo* to give the sulfonium salt as a yellow solid. Yield: 9.1 g (100%); HPLC: Rₜ = 3.86 (>99%); ¹H-NMR (CDCl₃, 250 MHz) δ 9.33 (br, 1H), 7.27-7.19 (m, 5H), 6.93 (br, 1H), 6.38-6.36 (d, *J* = 7. 1, 1H), 5.01 (s, 2H), 4.61-4.59 (m, 1H), 3.75-3.58 (m, 2H), 3.08 (s, 3H), 2.99 (s, 3H), 2.45-2.30 (m, 2H), 1.34 (s, 9H); ¹³C-NMR (CDCl₃, 250 MHz) δ 171.1, 156.9, 155.8, 136.5, 129.0, 128.6, 128.4, 82.0, 67.7, 52.3, 40.3, 28.7, 26.8, 26.4; ES-MS: mass calcd for C₁₉H₃₀N₃O₅S 412.2 (M⁺). Found *m*/*z* 412.1.
Step c) Preparation of 3(*S*)-benzyloxycarbonylamino-1-*tert-*butoxycarbonylaminopyrrolidin-2-one:
   Cbz-L-Met-NHNHBoc methyl sulfonium iodide (9 g, 16.68 mmol) was dissolved in dry DCM:DMF 1:1 (338 mL). The resulting yellow solution was cooled in an ice-bath under Ar, and NaH (60 % suspension in mineral oil; 1344 mg, 33.6 mmol) was added in one portion. The solution was stirred at 0 °C for 3 h, during which time the color changed from yellow to various shades of red. The reaction was quenched by addition of EA (115 mL) and water (27 mL), and then stirred overnight at room temperature. The volume was concentrated to ca. 100 mL, diluted with water (300 mL) and extracted with EA (x3). The combined organics were washed with brine, dried over MgSO₄ and concentrated to give the crude product as a semi-solid residue (6 g after drying). This was dissolved in hot EA:DE 1:2 (60 mL), then cooled slowly to rt and finally to -18 °C. After 1 h a further 10 mL DE and 20 mL n-heptane were added portionwise to the cold solution. Upon storage at -18 °C o.n. a white solid was isolated, washed with n-heptane (x3) and dried. Yield: 2.9 g (50%); HPLC: Rₜ = 4.68 (>86%)/4.97 (>12%); isobaric peaks; ¹H-NMR (CDCl₃, 250 MHz) δ 7.28-7.19 (m, 5H), 6.81 (s, 1H), 5.51-5.49 (d, *J* = 7.1, 1H), 5.03 (s, 2H), 4.30-4.20 (m, 1H), 3.58-3.37 (m, 2H), 2.58-2.47 (m, 1H), 2.02-1.70 (m, 1H), 1.39 (s, 9H); ¹³C-NMR (CDCl₃, 250 MHz) δ 172.1, 156.8, 154.7, 136.7, 128.9, 128.5, 128.4, 82.2, 67.3, 51.2, 46.3, 28.5, 26.4; ES-MS: mass calcd for C₁₇H₂₄N₃O₅ 350.2 (MH⁺). Found *m*/*z* 350.1.
Step d) Preparation of 3(*S*)-benzyloxycarbonylamino-1-aminopyrrolidin-2-one:
   3(*S*)-benzyloxycarbonylamino-1-*tert*-butoxycarbonylaminopyrrolidin-2-one (100 mg, 0.286 mmol) was dissolved in HCl in dioxane (4M, 2 mL) and left at rt for 18 h. The solution was evaporated to dryness and the crude residue was purified by preparative HPLC to give a white solid. Yield: 35 mg (49%); HPLC: Rₜ = 3.61 (>99%); ES-MS: mass calcd for C₁₂H₁₆N₃O₃ 250.1 (MH⁺). Found *m*/*z* 250.1.
Step e) Preparation of 1,1-(2*S*-1,4-*cyclo*-benzyloxycarbonylaminobutanoyl)-4-(*o-*chlorobenzyl)semicarbazide:
   3(*S*)-Benzyloxycarbonylamino-1-aminopyrrolidin-2-one (35 mg, 0.14 mmol) and NEM (35 µL, 0.28 mmol) were dissolved in dry THF (1.5 mL), and CDI (25 mg, 0.154 mmol) was added. After a period of 21 h at rt *o*-chlorobenzylamine (19 µL, 0.154 mmol) was added. After 4.5 h the crude product was purified by preparative HPLC to give a white solid. Yield: 20 mg (34%); HPLC: Rₜ = 4.84 (>99%); ES-MS: mass calcd for C₂₀H₂₂N₄O₄ 417.1 (MH⁺). Found *m*/*z* 417.0.
Step f) Preparation of 1,1-(2*S*-1,4-cyclo-aminobutanoyl)-4-(*o-*chlorobenzyl)semicarbazide:
   1,1-(2*S*-1,4-*cyclo*-benzyloxycarbonylaminobutanoyl)-4-(*o*-chlorobenzyl)semicarbazide (19 mg, 46 µmol) was dissolved in glacial acetic acid (300 µL). HBr in acetic acid (5.7 M, 20 µL, 114 µmol) was added. After 22 h a further 20 µL HBr in acetic acid was added, and yet another 20 µL after a further 3.5 h. Three hours later the crude product was purified by preparative HPLC to give a white solid. Yield: 10.7 mg (83%); HPLC: Rₜ = 3.34 (>99%); ¹H-NMR (CDCl₃, 250 MHz) δ 7.56-7.34 (m, 4H), 4.59 (s, 2H), 4.29-4.21 (t, *J* = 9.1, 1H), 3.85-3.66 (m, 2H), 2.78-2.66 (m, 1H), 2.34-2.21 (m, 1H); ES-MS: mass calcd for C₁₂H₁₆ClN₄O₂ 283.1 (MH⁺). Found *m*/*z* 283.1.

### EXAMPLE 53

### 1,1-(2R-1,4-cyclo-Aminobutanoyl)-4-(o-chlorobenzyl)semicarbazide

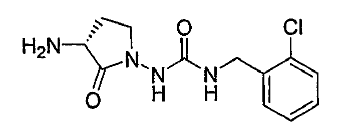

Scheme 3:
Step a) Preparation of Cbz-D-Met-NHNHBoc:
   Starting from Cbz-D-methionine (2 g, 7.06 mmol), following the procedure for Example 52 a) gave a white solid. Yield: 2.7 g (71%); HPLC: Rₜ = 4.03 (shorter gradient; >99%); R_{f} (PE:EA 1:1) = 0.62; ¹H-NMR (CDCl₃, 250 MHz) δ 8.92 (br, 1H), 7.32 (m, 5H), 7.09 (br, 1H), 6.09-6.05 (d, *J* = 8.2, 1H), 5.15-5.00 (m, 2H), 4.54-4.45 (m, 1H), 2.60-2.55 (t, *J* = 7.1, 2H), 2.19-1.89 (m, 5H), 1.45 (s, 9H); ¹³C-NMR (CDCl₃, 250 MHz) δ 173.2, 156.8, 155.8, 136.4, 128.9, 128.6, 128.5, 82.2, 67.6, 52.8, 32.2, 30.3, 28.5, 15.6; ES-MS: mass calcd for C₁₈H₂₈N₃O₅S 398.2 (MH⁺). Found *m*/*z* 398.2.
Step b) Preparation of Cbz-D-Met-NHNHBoc methyl sulfonium iodide:
   Starting from Cbz-D-Met-NHNHBoc (2.6 g, 6.55 mmol), following the procedure for Example 52 b) gave a yellow solid. Yield: 3.5 g (100%); HPLC: Rₜ = 3.71 (>99%); ¹H-NMR (CDCl₃, 250 MHz) δ 9.55 (br, 1H), 7.58 (br, 1H), 7.28-7.19 (m, 5H), 6.73-6.70 (d, *J* = 7.5, 1H), 5.00 (s, 2H), 4.50-4.47 (m, 1H), 3.68-3.59 (m, 2H), 3.08 (s, 3H), 3.03 (s, 3H), 2.33-2.21 (m, 2H), 1.35 (s, 9H); ¹³C-NMR (CDCl₃, 250 MHz) δ 171.8, 156.9, 155.8, 136.5, 128.8, 128.5, 128.3, 81.4, 67.3, 52.0, 40.0, 28.6, 26.3, 26.0; ES-MS: mass calcd for C₁₉H₃₀N₃O₅S 412.2 (M⁺). Found *m*/*z* 412.2.
Step c) Preparation of 3(*R*)-benzyloxycarbonylamino-1-*tert-*butoxycarbonylaminopyrrolidin-2-one:
   Starting from Cbz-D-Met-NHNHBoc methyl sulfonium iodide (1.7 g, 3.15 mmol), following the procedure for Example 52 c) gave the crude product as a clear oil (1.1 g after drying). This was purified on silica, using EA:n-heptane 2:1 to give a white solid. Yield: 660 mg (60%); HPLC: Rₜ = 4.47 (>66%)/4.80 (>28%); isobaric peaks; R_{f} (PE:EA 1:1) = 0.3; ¹H-NMR (CDCl₃, 250 MHz) δ 7.18-7.10 (m, 5H), 6.65 (br, 1H), 5.36 (br, 1H), 4.93 (s, 2H), 4.19-4.06 (m, 1H), 3.44-3.27 (m, 2H), 2.49-2.38 (m, 1H), 1.90-1.77 (m, 1H), 1.29 (s, 9H); ES-MS: mass calcd for C₁₇H₂₄N₃O₅ 350.2 (MH⁺). Found *m*/*z* 350.1.
Step d) Preparation of 3(*R*)-benzyloxycarbonylamino-1-aminopyrrolidin-2-one:
   3(*R*)-Benzyloxycarbonylamino-1-*tert*-butoxycarbonylaminopyrrolidin-2-one (650 mg, 1.86 mmol) was dissolved in DCM (10 mL). TFA:H₂O 95:5 (12 mL) was added and the resulting solution was left at -18 °C for 3 days, at which point it was taken to rt for 6 h and finally concentrated *in vacuo.* The crude, residual oil was purified by preparative HPLC to give a white solid. Yield: 56 mg (12%); HPLC: Rₜ = 3.36 (>95%); ES-MS: mass calcd for C₁₂H₁₆N₃O₃ 250.1 (MH⁺). Found *m*/*z* 250.1.
Step e) Preparation of 1,1-(2*R*-1,4-*cyclo*-benzyloxycarbonylaminobutanoyl)-4-(*o-*chlorobenzyl)semicarbazide:
   Starting from 3(*R*)-benzyloxycarbonylamino-1-aminopyrrolidin-2-one (28 mg, 0.113 mmol), following the procedure for Example 52 e) gave a white solid. Yield: 13 mg (28%); HPLC: Rₜ = 4.90 (>95%); ES-MS: mass calcd for C₂₀H₂₂N₄O₄ 417.1 (MH⁺). Found *m*/*z* 417.1.
Step f) Preparation of 1,1-(2*R*-1,4-*cyclo*-aminobutanoyl)-4-(*o-*chlorobenzyl)semicarbazide:
   1,1-(2*R*-1,4-*cyclo*-benzyloxycarbonylaminobutanoyl)-4-(*o*-chlorobenzyl)semicarbazide (13 mg, 31 µmol) was dissolved in neat TFA (625 µL, 8 mmol). Thioanisole (185 µL , 1.56 mmol) was added, followed by TMSBr (41 µL , 312 µmol). After 3 h at rt the solution was concentrated, and the crude product was purified by preparative HPLC to give a white solid. Yield: 3 mg (34%); HPLC: Rₜ = 3.15 (>99%); ¹H-NMR (CDCl₃, 250 MHz) δ 7.57-7.35 (m, 4H), 4.60 (s, 2H), 4.30-4.22 (t, *J* = 9.1, 1H), 3.86-3.67 (m, 2H), 2.79-2.67 (m, 1H), 2.34-2.18 (m, 1H); ES-MS: mass calcd for C₁₂H₁₆ClN₄O₂ 283.1 (MH⁺). Found *m*/*z* 283.1.

### EXAMPLE 54

### 1-(2S-2-Aminobutanoyl)-4-[o-(o-hydroxymethylphenylthio)benzyl]semicarbazide

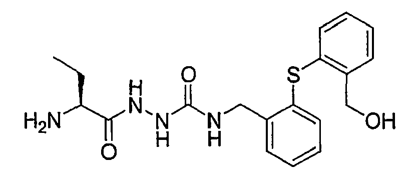

Scheme 2: a) Preparation of *N*^{α}-Boc-L-aminobutyric acid hydrazide: CDI (1.76 g, 10.8 mmol) was added slowly to a solution of *N*^{α}-Boc-L-aminobutyric acid (2.0 g, 9.84 mmol) in 20 mL dry THF. After addition the solution was stirred for 1 h. Then, hydrazine monohydrate (1.44 mL, 29.5 mmol) was added and the resulting solution was stirred o.n. The reaction mixture was concentrated and the white solid re-dissolved in 1,4-dioxane and lyophilized overnight yielding 3.85 g (180%) of crude product. The solid was a mixture of imidazole and desired product (imidazole:product 2:1, determined by ¹H-NMR) and was used without further purification. ¹H-NMR (DMSO-*d₆*, 250 MHz) δ 9.08-8.89 (s, 1H), 6.75-6.10 (d, *J* = 8.2, 1H), 3.79-3.64 (q, *J* = 7.91, 14.4, 1H), 1.61-1.34 (m, 2H), 1.34-1.18 (s, 9H), 0.81-0.63 (t, *J* = 7.54, 3H)
b) CDI (41 mg, 0.26 mmol) was added to a solution of *N*^{α}-Boc-L-aminobutyric acid hydrazide (50 mg, 0.23 mmol) in 1.0 mL dry THF. The resulting solution was stirred overnight and 2-[2-(aminomethyl)phenylthio]-benzyl alcohol (0.26 mmol) dissolved in 1.0 mL dry THF was added. The reaction mixture was stirred o.n., and then concentrated. The residue was treated with 1 mL 95% TFA/H₂O for 5 min, and concentrated followed by purification by preparative HPLC. The title compound was obtained as a white solid. Yield: 25 mg (28%); HPLC: Rₜ = 3.66 min. (>98%); ES-MS: mass calcd for C₁₉H₂₄N₄O₂S 389.2 (MH⁺). Found *m*/*z* 389.1.

### EXAMPLE 55

### 1-(2S-2-Aminobutanoyl)-4-(o-bromobenzyl)semicarbazide

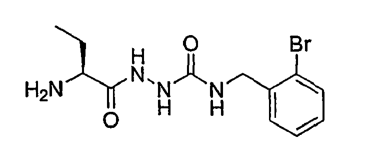

Scheme 2: Following the general procedure of example 54, except substituting 2-[2-(aminomethyl)phenylthio]-benzyl alcohol for *o*-bromobenzylamine. Yield: 49 mg (65%); HPLC: Rₜ = 3.12 min. (>99%); ES-MS: mass calcd for C₁₂H₁₇BrN₄O₂ 329.1 (MH⁺). Found *m*/*z* 329.1

### EXAMPLE 56

### 1-(2S-2-Aminobutanoyl)-4-(2-chloro-6-fluorobenzyl)semicarbazide

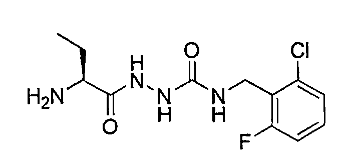

Scheme 2: Following the general procedure of example 54, except substituting 2-[2-(aminomethyl)phenylthio]-benzyl alcohol for 2-chloro-6-fluorobenzylamine. Yield: 14 mg (20%); HPLC: Rₜ = 3.03 min. (>99%); ES-MS: mass calcd for C₁₂H₁₇ClFN₄O₂ 303.1 (MH⁺). Found *m*/*z* 303.0.

### EXAMPLE 57

### 1-(2S-2-Aminobutanoyl)-4-(o-ethoxybenzyl)semicarbazide

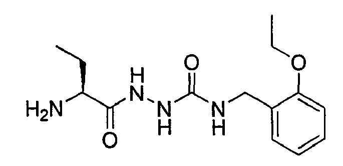

Scheme 2: Following the general procedure of example 54, except substituting 2-[2-(aminomethyl)phenylthio]-benzyl alcohol for o-ethoxybenzylamine. Yield: 28 mg (41%); HPLC: Rₜ = 3.27 min. (>99%); ES-MS: mass calcd for C₁₄H₂₃N₄O₃ 295.2 (MH⁺). Found *m*/*z* 295.2

### EXAMPLE 58

### 1-(2S-2-Aminobutanoyl)-4-([1,1'-biphenyl-4-yl]methyl)semicarbazide

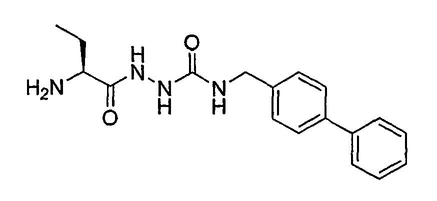

Scheme 2: Following the general procedure of example 54, except substituting 2-[2-(aminomethyl)phenylthio]-benzyl alcohol for (1,1'-biphenyl-4-yl)methylamine. Yield: 34 mg (45%); HPLC: Rₜ = 3.86 min. (>99%); ES-MS: mass calcd for C₁₈H₂₃N₄O₂ 327.2 (MH⁺). Found *m*/*z* 327.2.

### EXAMPLE 59

### 1-(2S-2-Aminobutanoyl)-4-([1,1'-biphenyl-2-yl]methyl)semicarbazide

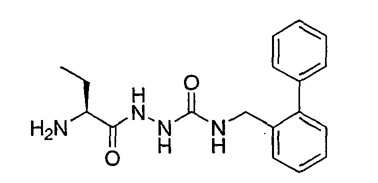

Scheme 2: Following the general procedure of example 54, except substituting 2-[2-(aminomethyl)phenylthio]-benzyl alcohol for (1,1'-biphenyl-2-yl)methylamine. Yield: 63 mg (83%); HPLC: Rₜ = 3.97 min. (>97%); ES-MS: mass calcd for C₁₈H₂₃N₄O₂ 327.2 (MH⁺). Found *m*/*z* 327.4.

### EXAMPLE 60

### 1-(2S-2-Aminobutanoyl)-4-(p-[thiophen-2-yl]benzyl)semicarbazide

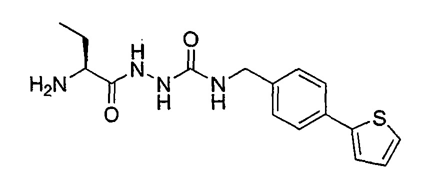

Scheme 2: Following the general procedure of example 54, except substituting 2-[2-(aminomethyl)phenylthio]-benzyl alcohol for *p*-(thiophen-2-yl)benzylamine. Yield: 29 mg (37%); HPLC: Rₜ = 3.92 min. (>99%); ES-MS: mass calcd for C₁₆H₂₁N₄O₂S 333.1 (MH⁺). Found *m*/*z* 333.2.

### EXAMPLE 61

### 1-(2S-2-Aminobutanoyl)-4-(o-[N-morpholinyl]benzyl)semicarbazide

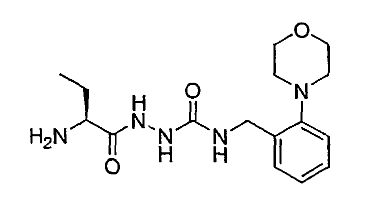

Scheme 2: Following the general procedure of example 54, except substituting 2-[2-(aminomethyl)phenylthio]-benzyl alcohol for *o*-(*N*-morpholinyl)benzylamine. Yield: 32 mg (41%); HPLC: Rₜ = 2.55 min. (>99%); ES-MS: mass calcd for C₁₆H₂₆N₅O₃ 336.2 (MH⁺). Found *m*/*z* 336.3.

### EXAMPLE 62

### 1-(2S-2-Aminobutanoyl)-4-(1-naphthylmethyl)semicarbazide

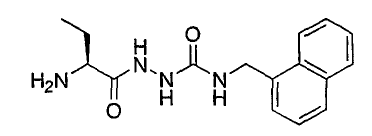

Scheme 2: Following the general procedure of example 54, except substituting 2-[2-(aminomethyl)phenylthio]-benzyl alcohol for 1-naphthylmethylamine. Yield: 32 mg (46%); HPLC: Rₜ = 3.53 min. (>99%); ES-MS: mass calcd for C₁₆H₂₁N₄O₂ 301.2 (MH⁺). Found *m*/*z* 301.3.

### EXAMPLE 63

### 1-(2S-2-Aminobutanoyl)-4-(o-[N-piperidinyl]benzyl)semicarbazide

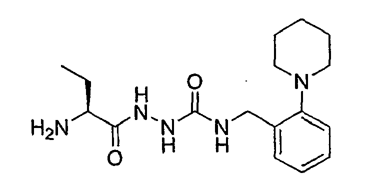

Scheme 2: Following the general procedure of example 54, except substituting 2-[2-(aminomethyl)phenylthio]-benzyl alcohol for *o*-(*N*-piperidinyl)benzylamine. Yield: 7 mg (9%); HPLC: Rₜ = 2.71 min. (>96%); ES-MS: mass calcd for C₁₇H₂₈N₅O₂ 334.2 (MH⁺). Found *m*/*z* 334.2.

### EXAMPLE 64

### 1-(2S-2-Aminobutanoyl)-4-(o-methylthiobenzyl)semicarbazide

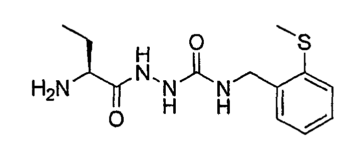

Scheme 2: Following the general procedure of example 54, except substituting 2-[2-(aminomethyl)phenylthio]-benzyl alcohol for o-methylthiobenzylamine. Yield: 20 mg (29%); HPLC: Rₜ = 3.27 min. (99>%); ES-MS: mass calcd for C₁₃H₂₁N₄O₂S 297.1 (MH⁺). Found *m*/*z* 297.2.

### Biological Assays

The compounds of this invention may be tested in one of several biological assays to determine their pharmacological properties.

### Human dipeptidyl peptidase I (DPP-I) assay

Using this assay, the IC₅₀ value of a compound of the invention as a DPP-I inhibitor was determined using an AFC substrate.

### Assay buffer (pH 6.0):

100 mM sodium phosphate (8.9 g Na₂HPO₄; M = 177.99), 150 mM KCI (5.6 g KCl; M = 74.6) and 1.5 mM EDTA (279 mg EDTA; M = 372.2) was dissolved in 500 mL H₂O, and pH was adjusted to 6.0. Cysteine*HCl (Sigma C-1276; M = 157.6), 1 mg/mL assay buffer, corresponding to 6 mM, was added to the solution for activation of the DPP-I enzyme.

### Substrate:

Gly-Phe-AFC*TFA (Enzyme Systems Products AFC-033) was used as the substrate for determination of IC₅₀ values. Kₘ was 270 µM. The substrate was solubilized in DMSO to give a 7.5 mM stock solution (2.2 mg of substrate was added to 0.5 mL DMSO).

### DPP-I:

Human DPP-I (hDDP-I; obtained from UniZyme A/S, DK-2970 Hørsholm, Denmark) was stored at -20 °C in a buffer containing 2.5 mM Na-phosphate, 150 mM NaCl, 2 mM cysteamine, 50% glycerol, pH 7.0 at a concentration of 2.5 mg/mL. This stock solution was diluted 200 times in the assay buffer.

### Assay conditions:

The assay was performed in 96-well plates. Assay buffer (230 µL) was added to the well, followed by 10 µL of diluted DPP-I, corresponding to 9.1 nM in the assay. Then 5 µL of either DMSO (control) or test substance in varying concentrations was added, and the solution was mixed. The plate was incubated at 37 °C for 10 minutes, followed by addition of 5 µL of 7.5 mM substrate (corresponding to 150 µM in the assay). The excitation wavelength was 400 nm, and the emission was measured at 505 nm for 10 minutes at 37 °C. Each measurement was made in duplicate. In the software (SOFTmax Pro) used for data collection from the fluorometer (Molecular Devices: Gemini XS), it was ensured that the measured slopes were linear (R² >0.99). Data were exported to GraphPad Prism and nonlinear regression was performed using the option Sigmoidal dose-response (variable slope).

### Human liver cathepsin B assay

Using this assay, the selectivity of a compound of the invention for hDPP-1 over human cathepsin B was determined, using a fluorogenic substrate.

### Assay buffer (pH 6. 1):

0.1 M MES buffer (1.95 g; M = 195.2) and 1 mM EDTA (37 mg; M = 372.2) were mixed in 100 mL H₂O and pH adjusted to 6.1. DTT (10 µL, 0.5 M) was added for activation of the enzyme (corresponding to 5 mM).

### Cathepsin B:

Human liver cathepsin B (Enzyme System Products, CAT-B; stock 25 µg / 54 µL = 463 ng/µL). One aliquot was diluted to a concentration of 40 ng/µL by adding 53 µL assay buffer (without DTT). Just prior to experiment, two more dilution steps were performed:
4 ng/µL: 5 µL (40 ng/µL) + 45 µL buffer (without DTT)
0.1 ng/µL: 5 µL (4 ng/µL) + 285 µL buffer (without DTT)

### Substrate:

Boc-Leu-Arg-Arg-AFC•2TFA (Enzyme System Products AFC113). Stock solution made (20 mM; 15.1 mg dissolved in 1 ml DMSO). Diluted further in H₂O to 10 mM. Kₘ for this substrate has been determined to be 600 µM.

### Assay conditions:

The assay was performed in 96-well plates. 84 µL assay buffer was added to the well followed by 10 µL 1 % DMSO in assay buffer (control) or a compound of the invention (10 µM in the assay). Then 10 µL (0.1 ng/µL, corresponding to 1 ng in assay) enzyme was added, and 5 min. later 6 µL substrate (10 mM, corresponding to 600 µM in the assay) was added. The excitation wavelength was 400 nm, and the emission was measured at 505 nm for 10-20 minutes at 37 °C. Each measurement was made in duplicate. In the software (SOFTmax Pro) used for data collection from the fluorometer (Molecular Devices: Gemini XS), it was ensured that the measured slopes were linear (R² >0.99). Data were exported to GraphPad Prism and nonlinear regression was performed using the option Sigmoidal dose-response (variable slope).

### Human cathepsin G assay

Using this assay, the selectivity of a compound of the invention for hDPP-I over human cathepsin G was determined, using a chromogenic substrate.

### Assay buffer (pH 8.3):

Buffer containing 100 mM Tris/HCl pH 8.3 (25 °C), 500 mM NaCl, and 20 mM CaCl2 prepared. DTNB (5,5'-dithio-bis(2-nitrobenzoic acid)) was added just prior to experiment (10 µL 1.25 mM/well).

### Cathepsin G:

Human cathepsin G (Calbiochem Cat # 219373, 50 ng/µL = 0.1 mU/µL stock) was reconstituted in 50 mM sodium acetate and 150 mM NaCl (pH 5.5), and stored at -20 °C. Diluted to 2 ng/µL in buffer just prior to experiment.

### Substrate:

Suc-Ala-Ala-Pro-Phel-SBzl (Bachem Cat # 4009924.0050) diluted to 1 mM in buffer from 10 mM stock in buffer. Kₘ determined to 100-200 µM.

### Assay conditions:

The assay was performed in 96-well plates. 60 µL assay buffer was added to the well followed by 10 µL DTNB (1.25 mM), and 10 µL of a compound of the invention (100µM, diluted with assay buffer to give 10 µM in the assay). Then 10 µL (2 ng/µL) enzyme was added, and 10 min. later (at 37 °C) 10 µL substrate (100 µM final concentration) was added. The absorption was measured at 410 nm for 10 minutes at 37 °C. Each measurement was made in duplicate. In the software (SOFTmax Pro) used for data collection from the UV-spectrometer it was ensured that the measured slopes were linear (R² >0.99). Data were exported to GraphPad Prism and nonlinear regression was performed using the option Sigmoidal dose-response (variable slope).

### Human liver cathepsin H assay

Using this assay, the selectivity of a compound of the invention for hDPP-1 over human cathepsin H was determined, using a fluorogenic substrate.

### Assay buffer (pH 6.0):

50 mM sodium phosphate (0.89 g Na₂HPO₄; M = 177.99), 2.0 mM EDTA (74 mg EDTA; M = 372.2), 0.012 % Triton-X (390 µL 3 %) dissolved in 100 mL H₂O and pH adjusted to 6.0.

### Cathepsin H:

Human liver cathepsin H (Enzyme System Products, Cath-1; 25 µg) was solubilized in 60 µL enzyme buffer (giving a stock with a concentration of 417 ng/µL). Enzyme stock (5 µL) was diluted with 1245 µL enzyme buffer. Prior to running experiment 1 µL 0.5 M DTT/100 µL enzyme solution was added. Incubated for 5 minutes on ice, then added to reaction mixture.

### Substrate:

ARG-AFC*2HBR (Enzyme System Products AF002; 10.6 mg) was dissolved in 1 mL DMSO, giving a 20 mM solution. Km for this substrate has been determined to be 27 µM

### Assay conditions:

The assay was performed in 96-well plates. 50 µL assay buffer was added to the well followed by 25 µL reference inhibitor (cystatin; stock 1 mg/mL, diluted with assay buffer to give 10 nM in assay) or a compound of the invention (diluted with assay buffer to give 10 µM in the assay). Then 25 µL (40 ng) enzyme was added, and 1 min. later (at 37 °C), 100 µL ARG-AFC substrate (15 µM in the assay) was added. The excitation wavelength was 400 nm, and the emission was measured at 505 nm for 10-20 minutes at 37 °C. Each measurement was made in duplicate. In the software (SOFTmax Pro) used for data collection from the fluorometer (Molecular Devices: Gemini XS), it was ensured that the measured slopes were linear (R² >0.99). Data were exported to GraphPad Prism and nonlinear regression was performed using the option Sigmoidal dose-response (variable slope).

### Human liver cathepsin L assay

Using this assay, the selectivity of a compound of the invention for hDPP-I over human cathepsin L was determined, using a fluorogenic substrate.

### Assay buffer (pH 5.5):

20 mM sodium acetate (M = 82.04; 164 mg), 4.0 mM EDTA (M = 372.2; 149 mg), 0.012 % Triton-X (390 µL 3 %) dissolved in 100 mL H₂O and pH adjusted to 5.5.

### Cathepsin L:

Human liver cathepsin L (Enzyme System Products, Catl-1; 5 µL of a1.61 µU/µL stock) was solubilized in 2500 µL enzyme buffer. Prior to running experiment, 1 µL 0.5 M DTT/100 µL enzyme solution was added. Incubated for 5 minutes on ice, then added to reaction mixture.

### Substrate:

Z-Phe-ARG-AFC*TFA (Enzyme System Products AF052; 15.6 mg) was dissolved in 1 mL DMSO, giving a 20 mM solution.

### Assay conditions:

The assay was performed in 96-well plates. 50 µL assay buffer was added to the well followed by 25 µL reference inhibitor (cystatin, stock 1 mg/mL, diluted with assay buffer to give 25 nM in assay) or a compound of the invention (diluted with assay buffer to give 10 µM in the assay). Then 25 µL (80 nU) enzyme was added, and 1 min. later (at 37 °C) 100 µL substrate (10 µM in the assay) was added. The excitation wavelength was 400 nm, and the emission was measured at 505 nm for 10-20 minutes at 37°C. Each measurement was made in duplicate. In the software (SOFTmax Pro) used for data collection from the fluorometer (Molecular Devices: Gemini XS), it was ensured that the measured slopes were linear (R² >0.99). Data were exported to GraphPad Prism and nonlinear regression was performed using the option Sigmoidal dose-response (variable slope).

### Aspergillus DPP-IV assay

Using this assay, the selectivity of a compound of the invention for hDPP-I over Aspergillus DPP-IV was determined, using a fluorogenic substrate.

### Assay buffer (pH 7.4):

50 mM Tris, 0.1 % Triton.

### Aspergillus DPP-IV:

*Aspergillus* DPP-IV (Enzyme System Products, SPE01; 5 mU dissolved in 25 µL assay buffer, giving a stock with a concentration of a 0.2 mU/µL). Prior to running experiment, 5 µL of enzyme stock solution was diluted to 0.004 mU/µL with 245 µL buffer.

### Substrate:

Gly-Pro-AFC*TFA (Enzyme System Products AF039; 10 mg) was dissolved in 1 mL DMSO, giving a 20 mM solution. Diluted further in buffer to 1 mM.

### Assay conditions:

The assay was performed in 96-well plates. 60 µL assay buffer was added to the well followed by 10 µL 1% DMSO in assay buffer (control) or a compound of the invention (100 µM, corresponding to 10 µM in the assay). Incubated at 37 °C, then 10 µL enzyme (corresponding to 0.04 mU in assay) was added, followed by 10 µL substrate (100 µM in the assay). The excitation wavelength was 400 nm, and the emission was measured at 505 nm for 10-20 minutes at 30 °C. Each measurement was made in duplicate. In the software (SOFTmax Pro) used for data collection from the fluorometer (Molecular Devices: Gemini XS), it was ensured that the measured slopes were linear (R² >0.99). Data were exported to GraphPad Prism and nonlinear regression was performed using the option Sigmoidal dose-response (variable slope).

### Human neutrophil elastase assay

Using this assay, the selectivity of a compound of the invention for hDPP-1 over human neutrophil elastase was determined, using a fluorogenic substrate.

### Assay buffer (pH 7.5):

Buffer containing 100 mM Tris/HCl pH 7.5 (25 °C), and 500 mM NaCl.

### Neutrophil elastase:

Human neutrophil elastase (Calbiochem Cat # 324681, 50 ng/□L stock) was reconstituted in 50 mM sodium acetate pH 5.5 and 200 mM NaCl, then stored at -20 °C. Diluted to 1 ng/µL in buffer just prior to experiment.

### Substrate:

MeOSuc-Ala-Ala-Pro-Val-AMC (Calbiochem Cat # 324740) was dissolved in buffer to 4 mM (398 µL/mg) just prior to experiment. Kₘ was determined to be 560 µM.

### Assay conditions:

The assay was performed in 96-well plates. 80 µL assay buffer was added to the well followed by 10 µL enzyme (1 ng/µL). Incubated for 10 min. at 37 °C, then 10 µL substrate was added, and subsequently a compound of the invention (10 µM in the assay) The excitation wavelength was 380 nm, and the emission was measured at 460 nm for 10-20 minutes at 37 °C. Each measurement was made in duplicate. In the software (SOFTmax Pro) used for data collection from the fluorometer (Molecular Devices: Gemini XS), it was ensured that the measured slopes were linear (R² >0.99). Data was exported to GraphPad Prism and nonlinear regression was performed using the option sigmoidal dose-response (variable slope).

### Tryptase assay

Using this assay, the selectivity of a compound of the invention for hDPP-I over β-tryptase was determined, using a fluorogenic substrate.

### Assay buffer (pH 7.5):

0.1 M HEPES buffer (pH 7.5) with 0.5 M NaCl.

### Tryptase:

Rh-Skin β-tryptase (Promega # G7061) was diluted to a final stock solution of 0.08 µg/µL in assay buffer.

Rh-lung β-tryptase (Promega # G5631) was diluted to a final stock solution of 0.08 µg/µL in assay buffer.

### Substrate:

Z- ARG-AMC*HCl (BACHEM 11130; 10 mg) was dissolved in 1 mL DMSO, giving a 20 mM solution. Kₘ was determined to be > 400 µM.

### Assay conditions:

The assay was performed in 96-well plates. 85 µl assay buffer was added to the well followed by 5 µL enzyme stock. Then 5 µL substrate was added, and subsequently 5 µL 4% DMSO in assay buffer (control) or a compound of the invention (10 µM in the assay) The excitation wavelength was 380 nm, and the emission was measured at 460 nm for 10-20 minutes at 37°C. Each measurement was made in duplicate. In the software (SOFTmax Pro) used for data collection from the fluorometer (Molecular Devices: Gemini XS), it was ensured that the measured slopes were linear (R² >0.99).

Data were exported to GraphPad Prism and nonlinear regression was performed using the option Sigmoidal dose-response (variable slope).

### Human CYP1A2 assay

This assay was used to determine the effect of a compound of the invention on the CYP1A2 liver metabolising enzyme.

Vivid^{®} CYP1A2 Blue Screening Kit from PanVera was used.

### Kit components:

Vivid^{®}CYP450 Reaction Buffer I: 200 mM potassium phosphate buffer, pH 8.
CYP1A2 baculosomes^{®} reagent: CYP1A2 and NADH-P450 reductase (P450-specific content: 1.1 µM). Microsomes prepared from insect cells that were infected with baculovirus containing the cDNA for human CYP1A2 and rabbit cytochrome P450 reductase.
Regenerating System: 333 mM glucose-6-phosphate and 40 U/mL glucose-6-phosphate dehydrogenase in 100 mM potassium phosphate buffer, pH 8.
Vivid^{®} CYP1A2 Blue Substrate.
Vivid^{®} Blue Standard: 3-cyano-7-hydroxycoumarin.
10 mM NADP⁺ in 100 mM potassium phosphate buffer, pH 8.

### Procedure

Tubes thawed on ice. 2x reaction buffer dispensed (room temperature) in two eppendorf tubes (for Mix A and Mix B).

Controls or compounds of the invention: Diluted to a concentration of 50 µM. Positive control compound: α-naphthoflavone.
Negative controls: DMSO and H₂O.

Mix A: 485 µL reaction buffer, 10 µL regeneration system, 5 µL Baculosome reagent. Mixed gently and placed on ice.

Mix B: 88.5 µL reaction buffer, 1.5 µL substrate, 10 µL 10 mM NADP⁺.

In a 96-well plate (Costar c-3904), 40 µL of 50 µM sample dispensed. 50 µl Mix A added. Incubated 20 min. at rt. Then, 10 µl of Mix B added. Fluorescence determined (Molecular Devices, SPECTRAmax Gemini EM): Ex: 409 nm, Em: 460 nm, at 37 °C for 20 min. (Automix: 5 s, top read.).
Following the procedure for CYP1A2, using the appropriate kits below, the effect of a compound of the invention on the other CYP enzymes was determined:
Vivid^{®} CYP2C9 Red Screening Kit from PanVera.
Vivid^{®} CYP2C19 Red Screening Kit from PanVera.
Vivid^{®} CYP2D6 Cyan Screening Kit from PanVera.
Vivid^{®} CYP3A4 Red Screening Kit from PanVera.

### Cellular DPP-I assay

The following assay was used to the determine the inhibitory effect of compounds of the invention on cellular rat DPP-I.

### Cell culture:

The RBL-2H3 cells (basophilic leukaemia cell line), were obtained from the American Type Culture Collection. Cells were cultured at 37 °C in humidified air atmosphere with 5% CO₂. Cells were grown in complete media (CM) consisting of RPMI (GIBCO) supplemented with 10% heat-inactivated foetal bovine serum (FBS; GIBCO), 100 U/mL penicillin and 100 ug/mL streptomycin (GIBCO).

### Treatment of cells:

All cell-based experiments employing inhibitors of DPP-I were performed by addition of the compound (kept below -20 °C as a 10 mM stock solution in DMSO (Sigma)) for indicated time and concentrations to sub-confluent cell cultures growing on culture dishes. All experiments were set up in triplicates. Following the treatment, cells were washed twice in ice-cold PBS (Sigma) and scraped from the culture dish. After another wash in PBS cells were lysed in ice-cold H₂O containing 0.3% Triton × 100. An additional volume of buffer containing 40 mM MOPS, 2M NaCl, 2mM EDTA and 20% of glycerol was added. The cell lysate was clarified by centrifugation at 3000 *rpm* for 10 min. The resulting supernatant was transferred to eppendorf tubes. The Coomassie Plus Protein Assay (Pierece) was used to determine protein levels of the samples.

### In vitro measurement of DPP-I activity:

The activity of DPP-I in cell lysates was performed in 100 mM sodium phosphate, 150 mM KCl, 1.5 mM EDTA (pH = 6) using 50 uM of H-G(Y(3NO2))GPP(K(Abz))G-OH
(Schafer-N, Denmark) as a substrate. For each measurement, cell lysate corresponding to approximately 50 µg of protein was used. All samples were prepared in duplicates.

### Assay for determination of metabolic stability is rat liver microsomes

The assay was performed at Cerep.(Seattle) according to the protocol described by Kuhnz et. al. (Kuhnz, W., Gieschan, H. Drug Metab. Dispos., 26,1120-1127), using rat liver microsomes (0.3 mg/mL), test compound (1 µM), NADP (1 mM), and G6P (5 mM), and G6PDHase (1 U/mL) with 0.6 % methanol/0.6 % acetonitrile in water. Incubation was performed at 37 °C in phosphate buffer (50 mM, pH = 7.4), and the samples were analyzed for remaining product ion (selected reaction monitoring) by HPLC-MS/MS (Thermo Finnigan) at 0, 15, 30, 45 and 60 min. intervals.

### Results:

| **Assay** | **Enzyme/ species** | **Example 11** |
|---|---|---|
| Primary enzymatic screening (IC₅₀) | DPP-I | 40 ± 6 nM |
| Secondary enzymatic screening (10 µM cutoff or IC₅₀) | Cathepsin B | 4500 nM |
| | Cathepsin G | > 10 µM |
| | Cathepsin H | 3200 nM |
| | Cathepsin L | > 10 µM |
| | DPP-IV | > 10 µM |
| | Neutrophil elastase | > 10 µM |
| | Tryptase | > 10µM |
| Metabolism: CYP enzymes (20 µM cutoff or IC₅₀) | CYP1A2 | > 20 µM |
| | CYP2C9 | > 20 µM |
| | CYP2C19 | >20µM |
| | CYP2D6 | >20µM |
| | CYP3A4 | 600 nM |
| Cellular (% inhibition) | DPP-I (RBL-2H3 cells) | 54% |
| Metabolic stability (t_{1/2} in min.) | Rat liver microsomes | 23 |

The results show that the compound tested is a selective inhibitor of DPP-I.

## Claims

1. A compound of formula (I) for use in medicine 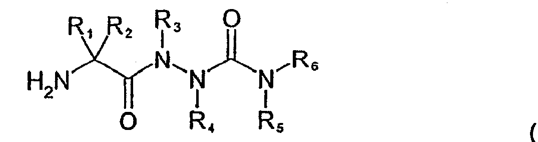 or a pharmaceutically acceptable salt or ester thereof,
wherein
R₁ and R₂ independently of each other are hydrogen, C₁₋₆alkyl, C₂₋₆alkenyl, C₂₋₆alkynyl, an unsubstituted or substituted C₃₋₁₀cycloalkyl group, an unsubstituted or substituted C₅₋₁₀cycloalkenyl group, an unsubstituted or substituted C₃₋₇heterocycloalkyl group, an unsubstituted or substituted C₁₋₆alkylaryl group but not benzyl when R₆ are phenyl, an unsubstituted or substituted C₁₋₆alkylheteroaryl group, an unsubstituted or substituted aryl group, an unsubstituted or substituted heteroaryl group, an unsubstituted or substituted aryl-C₁₋₅alkyl group, an unsubstituted or substituted heteroaryl-C₁₋₅alkyl group, an unsubstituted or substituted C₃₋₇cycloalkyl-C₁₋₅alkyl group, an unsubstituted or substituted C₃₋₇heterocycloalkyl-C₁₋₅alkyl group, or R₁ and R₂ together form an unsubstituted or substituted C₃₋₁₀cycloalkyl group or an unsubstituted or substituted C₃₋₇ heterocycloalkyl group;
R₃ is hydrogen, C₁₋₆alkyl, or R₃ and R₁ together form an unsubstituted or substituted C₃₋₁₀ cycloalkyl group or an unsubstituted or substituted C₃₋₇heterocycloalkyl group, or R₃ and R₂ together form an unsubstituted or substituted C₃₋₁₀cycloalkyl group or an unsubstituted or substituted C₃₋₇heterocycloalkyl group;
R₄ and R₅ independently of each other are hydrogen or C₁₋₆alkyl;
R₆ is C₁₋₆alkyl, -CH(R₇)CONH(R₈), C₂₋₆alkenyl, C₂₋₆alkynyl, an unsubstituted or substituted C₃₋₁₀cycloalkyl group, an unsubstituted or substituted C₅₋₁₀cycloalkenyl group, an unsubstituted or substituted C₃₋₇heterocycloalkyl group, an unsubstituted or substituted C₃₋₇cycloalkyl-C₁₋₅alkyl group, an unsubstituted or substituted C₃₋₇heterocycloalkyl-C₁₋₅alkyl group, an unsubstituted or substituted C₁₋₆alkylaryl group, an unsubstituted or substituted C₁₋₆alkyl-heteroaryl group, an unsubstituted or substituted aryl group, an unsubstituted or substituted heteroaryl group, an unsubstituted or substituted aryl-C₁₋₅alkyl group, an unsubstituted or substituted heteroaryl-C₁₋₅alkyl group;
R₇ is hydrogen, an unsubstituted or substituted C₁₋₆alkyl group, an unsubstituted or substituted C₃₋₁₀cycloalkyl group, an unsubstituted or substituted C₁₋₆alkylaryl group, an unsubstituted or substituted C₁₋₆alkylheteroaryl group or a side chain of a natural or non-natural amino acid residue;
R₈ is hydrogen, an unsubstituted or substituted C₁₋₆alkyl group, an unsubstituted or substituted C₃₋₁₀cycloalkyl group, an unsubstituted or substituted C₁₋₆alkylaryl group, an unsubstituted or substituted C₁₋₆alkylheteroaryl group, a side chain of a natural or non-natural amino acid residue or a group -CH(R₇)CONH(R₉);
R₉ is hydrogen, an unsubstituted or substituted C₁₋₆alkyl group, an unsubstituted or substituted C₃₋₁₀cycloalkyl group, an unsubstituted or substituted C₁₋₆alkylaryl group or an unsubstituted or substituted C₁₋₆alkylheteroaryl group, wherein a substituted group is substituted with one, two or three substituents independently selected from the group consisting of C₁₋₆alkyl, C₁₋₆alkylthio, C₁₋₆alkylcarbonyl, C₁₋₆-N-alkylamide, C₁₋₆alkoxy, dialkylamino-C₁₋₆alkyl, amide, hydroxy, carboxy, amino, halogen, trifluoromethyl, trifluoromethoxy, trifluoromethylthio and cyano;
with the proviso that the compound is not Ile-Gly^{a}-Leu-Met-NH₂ ,
wherein Gly^{a} is an α-aza-amino acid residue.

2. A compound according to claim 1, wherein R₁ and R₂, independently of each other, are hydrogen, C₁₋₆alkyl, an unsubstituted or substituted phenyl group, an unsubstituted or substituted C₁₋₆alkylaryl group or an unsubstituted or substituted C₁₋₆alkylheteroaryl group.

3. A compound according to claim 1 or 2, wherein R₁ and R₂, independently of each other, are selected from methyl, ethyl, propyl, butyl, iso-butyl, benzyl, 1-naphthylmethyl, 2-naphthylmethyl and (4-pyridyl)methyl.

4. A compound according to claim 1 or 2, wherein R₁ is C₁₋₆alkyl and R₂ is H.

5. A compound according to claim 1, wherein R₁ and R₂ together form an unsubstituted or substituted C₃₋₁₀cycloalkyl group or an unsubstituted or substituted C₃₋₇-heterocycloalkyl group.

6. A compound according to claim 5, wherein R₁ and R₂ together form an unsubstituted or substituted cyclohexyl group.

7. A compound according to any of the preceding claims, wherein R₃ is hydrogen or methyl.

8. A compound according to any of the claims 1-6, wherein R₁ and R₃ together form an unsubstituted or substituted C₃₋₁₀cycloalkyl group or an unsubstituted or substituted C₃₋₇heterocycloalkyl group.

9. A compound according to claim 1, wherein R₂ and R₃ together form an unsubstituted or substituted C₃₋₁₀cycloalkyl group or an unsubstituted or substituted C₃₋₇-heterocycloalkyl group.

10. A compound according to any of the preceding claims, wherein R₄ is hydrogen or methyl.

11. A compound according to any of the preceding claims, wherein R₅ is hydrogen or methyl.

12. A compound according to any of claims 1-7, 10-11, wherein at least one of R₃ and R₄ and R₅ is hydrogen.

13. A compound according to claim 12, wherein R₃ and R₄ and R₅ is hydrogen.

14. A compound according to any of the preceding claims, wherein R₆ is-CH(R₇)CONH(R₈), C₁₋₆alkyl, an unsubstituted or substituted C₁₋₆alkylaryl group or an unsubstituted or substituted C₁₋₆alkylheteroaryl group.

15. A compound according to any of the preceding claims, wherein R₆ is an unsubstituted or substituted benzyl group.

16. A compound according to any of claims 1-14, wherein R₆ is the group-CH(R₇)CONH(R₈).

17. A compound according to claim 1, wherein R₆ is selected from 2S-3-phenylpropan-2-yl-amide, 2S-N-[2S-3-(m-fluorophenyl)propan-2-yl-amide]-3-cyclohexylpropan-2-yl-amide, 2S-N-[2S-3-(m-fluorophenyl)propan-2-yl-amide]-4-phenylbutan-2-yl-amide, 2S-N-[2S-3-(indol-3-yl)propan-2-yl-amide]-3-phenylpropan-2-yl-amide, 2S-N-[2S-3-(m-fluorophenyl)propan-2-yl-amide]-3-methylbutan-2-yl-amide, 2S-N-[2S-3-(indol-3-yl)propan-2-yl-amide]-butan-2-yl-amide, 2S-N-[2S-3-(indol-3-yl)propan-2-yl-amide]-2-phenylethan-2-yl-amide, 2S-N-[2S-3-(indol-3-yl)propan-2-yl-amide]-4-phenylbutan-2-yl-amide, 2S-N-[2S-3-(m-fluorophenyl)propan-2-yl-amide]-3-(indol-3-yl)propan-2-yl-amide, 2S-N-[2S-3-(m-methoxypheny)propan-2-yl-amide]-3-(*p*-chtorophenyl)propan-2-yl-amide, 2S-N-[2S-3-(1,1'-biphenyl-4-yl)propan-2-yl-amide]-3-phenylpropan-2-yl-amide, 25-N-[2S-3-(m-methoxyphenyl)propan-2-yl-amide]-3-(1,1'-biphenyl-4-yl)propan2-yl-amide, 2S-N-[2S-3-(1,1'-biphenyl-4-yl)propan-2-yl-amide]-3-(p-hydroxyphenyl)propan-2-yl-amide, 2S-N-[2S-4-methylpentan-2-yl-amide]-3, (benzo[b]thiophen-3-yl)propan-2-yl-amide, 2S-N-[2S-3-(2-naphthyl)propan-2-yl-amide]-4-phenylbutan-2-yl-amide, 2S-N-[2S-3-(m-methoxyphenyl)propan-2-yl-amide]-4-phenylbutan-2-yl-amide, 2S-N-[2S-3-(benzo[b]thiophen-3-yl)propan-2-yl-amide]-4-phenylbutan-2-yl-amide, 2S-N-[2S-3-(p-chlorophenyl)propan-2-yl-amide]-4-phenylbutan-2-yl-amide, 2S-N-[2S-3-(m-fluorophenyl)propan-2-yl-amide]-3-(2-naphthyl)propan-2-yl-amide, 2S-N-[2S-3-(m-fluorophenyl)propan-2-yl-amide]-3-(1,1'-biphenyl-4-yl)propan-2-yl-amide, 2-phenylethyl, 2-(p-hydroxyphenyl)ethyl, benzyl, o-chlorobenzyl, 2-furfuryl, 2-(3-indolyl)ethyl, 1-naphthylmethyl, 4-piperonyl, p-chlorobenzyl, 2-(thiophen-2-yl)ethyl, m-fluorobenzyl, o-fluorobenzyl, p-fluorobenzyl, o-trifluoromethylbenzyl, m-chlorobenzyl, o-methoxybenzyl, o-methylbenzyl, alpha-methylbenzyl, o-(o-hydroxymethylphenylthio)benzyl, o-bromobenzyl, 2-chloro-6-fluorobenzyl, o-ethoxybenzyl, [1,1'-biphenyl-4-yl]methyl, [1,1'-biphenyl-2-yl]methyl, p-[thiophen-2-yl]benzyl, o-[N-morpholinyl]benzyl, o-[N-piperidinyl]benzyl and o-methylthiobenzyl.

18. A compound according to any of claims 1-14, wherein R₆ is an unsubstituted or substituted [1,1'-biphenyl-2-yl]methyl group or an unsubstituted or substituted [1,1'-biphenyl-4-yl]methyl group.

19. A compound according to any of claims 1-14, 16, wherein R₇ is hydrogen, an unsubstituted or substituted C₁₋₆alkyl group, an unsubstituted or substituted C₃₋₁₀ cycloalkyl group, an unsubstituted or substituted C₁₋₆alkylaryl group, an unsubstituted or substituted C₁₋₆alkylheteroaryl group.

20. A compound according to claim 19, wherein R₇ is an unsubstituted or substituted C₁₋₆alkylaryl group or an unsubstituted or substituted C₁₋₆alkylheteroaryl group.

21. A compound according to claim 20, wherein R₇ is 2-phenylethyl.

22. A compound according to any of claims 1-14, 16-21, wherein R₈ is hydrogen,-CH(R₇)CONH(R₉) or an unsubstituted or substituted C₁₋₆alkyl group.

23. A compound according to claim 22, wherein R₈ is -CH(R₇)CONH(R₉) and R₇ is an unsubstituted or substituted C₁₋₆alkylaryl group or an unsubstituted or substituted C₁₋₆ alkyl-heteroaryl group.

24. A compound of claim 23, wherein R₇ is (indol-3-yl)methyl or (m-fluorophenyl)methyl.

25. A compound according to claim 23, wherein R₉ is hydrogen or an unsubstituted or substituted C₁₋₆alkyl group.

26. A compound according to claim 25, wherein R₉ is hydrogen.

27. A compound according to claim 1 selected from the group consisting of
1-(2*S*-2-Aminopropionyl)-4-(2*S*-3-phenylpropan-2-yl-amide)semicarbazide;
1-(aminoacetyl)-4-(2*S*-3-phenylpropan-2-yl-amide)semicarbazide;
1-(2*S*-2-aminopropionyl)-1-methyl-4-(2*S*-3-phenylpropan-2-yl-amide)semicarbazide;
1-(1-aminocyclohexane-1-carbonyl)-4-[2*S*-*N*-[2*S*-3-(*m*-fluorophenyl)propan-2-yl-amide]-3-cyclohexylpropan-2-yl-amide]semicarbazide;
1-(1-aminocyclohexane-1-carbonyl)-4-[2*S*-*N*-[2*S*-3-(*m*-fluorophenyl)propan-2-yl-amide]-4-phenylbutan-2-yl-amide]semicarbazide;
1-(2*S*-2-aminobutanoyl)-4-[2*S*-*N*-[2*S*-3-(indol-3-yl)propan-2-yl-amide]-3-phenylpropan-2-yl-amide]semicarbazide;
1-(2*S*-2-aminopropanoyl)-4-[2*S*-*N*-[2*S*-3-(indol-3-yl)propan-2-yl-amide]-3-phenylpropan-2-yl-amide]semicarbazide;
1-(2*S*-3-naphthyl-2-propanoyl)-4-[2*S*-*N*-[2*S*-3-(*m*-fluorophenyl)propan-2-yl-amide]-3-methylbutan-2-yl-amide]semicarbazide;
1-[2*S*-2-amino-3-(4-pyridyl)propanoyl]-4-[2*S*-*N*(2*S*-3-(indol-3-yl)propan-2-yl-amide)-butan-2-yl-amide]semicarbazide;
1-[2*S*-2-amino-3-(4-pyridyl)propanoyl]-4-[2*S*-*N*-(2*S*-3-(indol-3-yl)propan-2-yl-amide)-2-phenylethan-2-yl-amide]semicarbazide;
1-(2*S*-2-Aminobutanoyl)-4-[2*S*-*N*-[2*S*-3-(indol-3-yl)propan-2-yl-amide]-4-phenylbutan-2-yl-amide]semicarbazide;
1-(2*S*-2-Aminobutanoyl)-4-[2*S*-*N*-[2*S*-3-(*m*-fluorophenyl)propan-2-yl-amide]-3-(indol-3-yl)propan-2-yl-amide]semicarbazide;
1-(2*S*-2-Aminobutanoyl)-4-[2*S*-*N*-[2*S*-3-(*m*-methoxyphenyl)propan-2-yl-amide]-3-(*p-*chlorophenyl)propan-2-yl-amide]semicarbazide;
1-(2*S*-2-Aminopentanoyl)-4-[2*S*-*N*-[2*S*-3-(*m*-methoxyphenyl)propan-2-yl-amide]-3-(*p-*chlorophenyl)propan-2-yl-amide]semicarbazide;
1-(2*S*-2-amino-4-phenylbutanoyl)-4-[2*S*-*N*-[2*S*-3-(1,1'-biphenyl-4-yl)propan-2-yl-amide]-3-phenylpropan-2-yl-amide]semicarbazide;
1-(2*S*-2-aminopentanoyl)-4-[2*S*-*N*-[2*S*-3-(*m*-methoxyphenyl)propan-2-yl-amide]-3-(1,1'-biphenyl-4-yl)propan2-yl-amide]semicarbazide;
1-(2*S*-2-aminobutanoyl)-4-[2*S*-*N*-[2*S*-3-(1,1'-biphenyl-4-yl)propan-2-yl-amide]-3-(*p-*hydroxyphenyl)propan-2-yl-amide]semicarbazide;
1-(2*S*-2-aminobutanoyl)-4-[2*S*-*N*-[2*S*-4-methylpentan-2-yl-amide]-3-(benzo[b]thiophen-3-yl)propan-2-yl-amide]semicarbazide;
1-(2*S*-2-Aminobutanoyl)-4-[2*S*-*N*-[2*S*-3-(2-naphthyl)propan-2-yl-amide]-4-phenylbutan-2-yl-amide]semicarbazide;
1-(2*S*-2-Aminobutanoyl)-4-[2*S*-*N*-[2*S*-3-(*m*-methoxyphenyl)propan-2-yl-amide]4-phenylbutan-2-yl-amide]semicarbazide;
1-(2*S*-2-Aminobutanoyl)-4-[2*S*-*N*-[2*S*-3-(benzo[b]thiophen-3-yl)propan-2-yl-amide]-4-phenylbutan-2-yl-amide]semicarbazide;
1-(2*S*-2-Aminobutanoyl)-4-[2*S*-*N*-[2*S*-3-(*p*-chlorophenyl)propan-2-yl-amide]-4-phenylbutan-2-yl-amide]semicarbazide;
1-(2*S*-2-Aminopentanoyl)-4-[2*S-N*-[2*S*-3-(2-naphthyl)propan-2-yl-amide]-4-phenylbutan-2-yl-amide]semicarbazide;
1-(2*S*-2-Aminobutanoyl)-4-[2*S-N*-[2*S*-3-(*m*-fluorophenyl)propan-2-yl-amide]-3-(2-naphthyl)propan-2-yl-amide]semicarbazide;
1-(2*S*-2-Aminobutanoyl)-4-[2*S-N*-[2*S-*3-(*m*-fluorophenyl)propan-2-yl-amide]-3-(1,1'-biphenyl-4-yl)propan-2-yl-amide]semicarbazide;
1-(2*S*-2-aminopropionyl)-4-(2-phenylethyl)semicarbazide;
1-(2*S*-2-aminopropionyl)-4-(2-phenylethyl)-4-methylsemicarbazide;
1-(2*S*-2-aminopropionyl)-4-[2-(*p*-hydroxyphenyl)ethyl]semicarbazide;
1-(2*S*-2-aminopropionyl)-4-benzylsemicarbazide;
1-(2*S*-2-aminopropionyl)-4-(*o*-chlorobenzyl)semicarbazide;
1-(2*S*-2-aminopropionyl)-4-(2-furfuryl)semicarbazide;
1-(2*S*-2-aminopropionyl)-4-[2-(3-indolyl)ethyl]semicarbazide;
1-(2*S*-2-aminopropionyl)-4-(1-naphthylmethyl)semicarbazide;
1-(2*S*-2-aminopropionyl)-4-piperonylsemicarbazide;
1-(2*S*-2-aminopropionyl)-4-(*p*-chlorobenzyl)semicarbazide;
1-(2*S*-2-aminopropionyl)-4-[2-(thiophen-2-yl)ethyl]semicarbazide;
1-(2*S*-2-aminopropionyl)-4-(*m*-fluorobenzyl)semicarbazide;
1-(2*S*-2-aminopropionyl)-4-(*o*-fluorobenzyl)semicarbazide;
1-(2*S*-2-aminopropionyl)-4-(*p*-fluorobenzyl)semicarbazide;
1-(2*S*-2-aminopropionyl)-4-(*o*-trifluoromethylbenzyl)semicarbazide;
1-(2*S*-2-aminopropionyl)4-(*m*-chlorobenzyl)semicarbazide;
1-(2*S*-2-aminopropionyl)-4-(*o-*methoxybenzyl)semicarbazide;
1-(2*S*-2-aminopropionyl)-4-(*o*-methylbenzyi)semicarbazide;
1-(2*S*-2-aminopropionyl)-4-(alpha-methylbenzyl)semicarbazide;
1-(2*S*-2-aminobutanoyl)-4-(*o*-chlorobenzyl)semicarbazide;
1-(2*S*-2-aminovaleryl)-4-(*o*-chlorobenzyl)semicarbazide;
1-(2*S*-2-aminohexanoyl)-4-(*o*-chlorobenzyl)semicarbazide;
1-(2*S*-2-aminoisocaproyl)-4-(*o*-chlorobenzyl)semicarbazide;
1-(1-aminocyclohexane-1-carbonyl)-4-(*o*-chlorobenzyl)semicarbazide;
1-(2*S*-2-amino-3-phenyl-propionyl)-4-(*o*-chlorobenzyl)semicarbazide;
1,1-(2*S*-1,4-*cyclo*-aminobutanoyl)-4-(*o*-chlorobenzyl)semicarbazide;
1,1-(2*R*-1,4-*cyclo*-aminobutanoyl)-4-(*o*-chlorobenzyl)semicarbazide;
1-(2*S*-2-Aminobutanoyl)-4-[2*S*-N[2*S*-3-(*m*-fluorophenyl)propan-2-yl-amide]-4-phenylbutan-2-yl-amide]semicarbazide;
1-(2*S*-2-Aminobutanoyl)-4-[*o*-(*o*-hydroxymethylphenylthio)benzyl]semicarbazide;
1-(2*S*-2-Aminobutanoyl)-4-(*o*-bromobenzyl)semicarbazide;
1-(2*S*-2-Aminobutanoyl)-4-(2-chloro-6-fluorobenzyl)semicarbazide;
1-(2*S*-2-Aminobutanoyl)-4-(*o*-ethoxybenzyl)semicarbazide;
1-(2*S*-2-Aminobutanoyl)-4-([1,1'-biphenyl-4-yl]methyl)semicarbazide;
1-(2*S*-2-Aminobutanoyl)-4-([1,1'-biphenyl-2-yl]methyl)semicarbazide;
1-(2*S*-2-Aminobutanoyl)-4-(*p*-[thiophen-2-yl]benzyl)semicarbazide;
1-(2*S*-2-Aminobutanoyl)-4-(*o*-[*N*-morpholinyl]benzyl)semicarbazide;
1-(2*S*-2-Aminobutanoyl)-4-(1-naphthylmethyl)semicarbazide;
1-(2*S*-2-Aminobutanoyl)-4-(*o*-[*N*-piperidinyl]benzyl)semicarbazide; and
1-(2*S*-2-Aminobutanoyl)-4-(*o*-methylthiobenzyl)semicarbazide.

28. A compound according to claim 1 selected from the group consisting of
1-(2*R*-2-aminopropionyl)-4-(2*S*-3-phenylpropan-2-yl-amide)semicarbazide;
1-(2*R*-2-aminopropionyl)-1-methyl-4-(2*S*-3-phenylpropan-2-yl-amide)semicarbazide;
1-(2*R*-2-aminobutanoyl)-4-[2*S*-*N*[2S 3-(indol-3-yl)propan-2-yl-amide]-3-phenylpropan-2-yl-amide]semicarbazide;
1-(2*R*-2-aminopropanoyl)-4-[2*S*-*N*-[2*S*-3-(indol-3-yl)propan-2-yl-amide]-3-phenylpropan-2-yl-amide]semicarbazide;
1-(2*R*-3-naphthyl-2-propanoyl)-4-[2*S*-*N*[2*S*-3-(*m*-fluorophenyl)propan-2-yl-amide]-3-methylbutan-2-yl-amide]semicarbazide;
1-[2*R*-2-amino-3-(4-pyridyl)propanoyl]-4-[2*S*-*N*-(2*S*-3-(indol-3-yl)propan-2-yl-amide)-butan-2-yl-amide]semicarbazide;
1-[2*R*-2-amino-3-(4-pyridyl)propanoyl]-4-[2*S*-*N*-(2*S*-3-(indol-3-yl)propan-2-yl-amide)2-phenylethan-2-yl-amide]semicarbazide;
1-(2*R*-2-Aminobutanoyl)-4-[2*S*-*N*-[2*S*-3-(indol-3-yl)propan-2-yl-amide]-4-phenylbutan-2-yl-amide]semicarbazide;
1-(2*R*-2-Aminobutanoyl)-4-[2*S*-*N*-[2*S*-3-(*m*-fluorophenyl)propan-2-yl-amide]-3-(indol-3-yl)propan-2-yl-amide]semicarbazide;
1-(2*R*-2-Aminobutanoyl)-4-[2*S*-*N*-[2*S*-3-(*m*-methoxyphenyl)propan-2-yl-amide]-3-(*p-*chlorophenyl)propan-2-yl-amide]semicarbazide;
1-(2*R*-2-Aminopentanoyl)-4-[2*S*-*N*-[2*S*-3-(*m*-methoxyphenyl)propan-2-yl-amide]-3-(*p-*chlorophenyl)propan-2-yl-amide]semicarbazide;
1-(2*R*-2-amino-4-phenylbutanoyl)-4-[2*S*-*N*-[2*S*-3-(1,1'-biphenyl-4-yl)propan-2-yl-amide]-3-phenylpropan-2-yl-amide]semicarbazide;
1-(2*R*-2-aminopentanoyl)-4-[2*S*-*N*-[2*S*-3-(*m*-methoxyphenyl)propan-2-yl-amide]-3-(1,1'-biphenyl-4-yl)propan2-yl-amide]semicarbazide;
1-(2*R*-2-aminobutanoyl)-4-[2*S*-*N*-[2*S*-3-(1,1'-biphenyl-4-yl)propan-2-yl-amide]-3-(*p-*hydroxyphenyl)propan-2-yl-amide]semicarbazide;
1-(2*R*-2-aminobutanoyl)-4-[2*S*-*N*-[2*S*-4-methylpentan-2-yl-amide]-3-(benzo[b]thiophen-3-yl)propan-2-yl-amide]semicarbazide;
1-(2*R*-2-Aminobutanoyl)-4-[2*S*-*N*-[2*S*-3-(2-naphthyl)propan-2-yl-amide]-4-phenylbutan-2-yl-amide]semicarbazide;
1-(2*R*-2-Aminobutanoyl)-4-[2*S*-*N*-[2*S*-3-(*m*-methoxyphenyl)propan-2-yl-amide]-4-phenylbutan-2-yl-amide]semicarbazide;
1-(2*R*-2-Aminobutanoyl)-4-[2*S*-*N*-[2*S*-*N*-(benzo[b]thiophen-3-yl)propan-2-yl-amide]-4-phenylbutan-2-yl-amide]semicarbazide;
1-(2*R*-2-Aminobutanoyl)-4-[2*S*-*N*-[2*S*-3-(*p*-chlorophenyl)propan-2-yl-amide]-4-phenylbutan-2-yl-amide]semicarbazide;
1-(2*R*-2-Aminopentanoyl)-4-[2*S*-*N*-[2*S*-3-(2-naphthyl)propan-2-yl-amide]-4-phenylbutan-2-yl-amide]semicarbazide;
1-(2*R*-2-Aminobutanoyl)-4-[2*S*-*N*-[2*S*-3-(*m*-fluorophenyl)propan-2-yl-amide]-3-(2-naphthyl)propan-2-yl-amide]semicarbazide;
1-(2*R*-2-Aminobutanoyl)-4-[25-*N*-[25-3-(*m*-fluorophenyl)propan-2-yl-amide]-3-(1,1'-biphenyl-4-yl)propan-2-yl-amide]semicarbazide;
1-(2*R*-2-aminopropionyl)-4-(2-phenylethyl)semicarbazide;
1-(2*R*-2-aminopropionyl)-4-(2-phenylethyl)-methylsemicarbazide;
1-(2*R*-2-aminopropionyl)-4-[2-(*p*-hydroxyphenyl)ethyl]semicarbazide;
1-(2*R*-2-aminopropionyl)-4-benzylsemicarbazide;
1-(2*R*-2-aminopropionyl)-4-(*o*-chlorobenzyl)semicarbazide;
1-(2*R*-2-aminopropionyl)-4-(2-furfuryl)semicarbazide;
1-(2*R*-2-aminopropionyl)-4-[2-(3-indolyl)ethyl]semicarbazide;
1-(2*R*-2-aminopropionyl)-4-(1-naphthylmethyl)semicarbazide;
1-(2*R*-2-aminopropionyl)-4-piperonylsemicarbazide;
1-(2*R*-2-aminopropionyl)-4-(*p*-chlorobenzyl)semicarbazide;
1-(2*R*-2-aminopropionyl)-4-[2-(thiophen-2-yl)ethyl]semicarbazide;
1-(2*R*-2-aminopropionyl)-4-(*m*-fluorobenzyl)semicarbazide;
1-(2*R*-2-aminopropionyl)-4-(*o*-fluorobenzyl)semicarbazide;
1-(2*R*-2-aminopropionyl)-4-(*p*-fluorobenzyl)semicarbazide;
1-(2*R*-2-aminopropionyl)-4-(*o*-trifluoromethylbenzyl)semicarbazide;
1-(2*R*-2-aminopropionyl)-4-(*m*-chlorobenzyl)semicarbazide;
1-(2*R*-2-aminopropionyl)-4-(*o*-methoxybenzyl)semicarbazide;
1-(2*R*-2-aminopropionyl)-4-(*o*-methylbenzyl)semicarbazide;
1-(2*R* 2-aminopropionyl)-4-(alpha-methylbenzyl)semicarbazide;
1-(2*R*-2-aminobutanoyl)-4-(*o*-chlorobenzyl)semicarbazide;
1-(2*R*-2-aminovaleryl)-4-(*o*-chlorobenzyl)semicarbazide;
1-(2*R*-2-aminohexanoyl)-4-(*o*-chlorobenzyl)semicarbazide;
1-(2*R*-2-aminoisocaproyl)-4-(*o*-chlorobenzyl)semicarbazide;
1-(2*R*-2-amino-3-phenyl-propionyl)-4-(*o*-chlorobenzyl)semicarbazide;
1-(2*R*-2-Aminobutanoyl)-4-[2*S*-*N*-[2*S*-3-(*m*-fluorophenyl)propan-2-yl-amide]-4-phenylbutan-2-yl-amide]semicarbazide;
1-(2*R*-2-Aminobutanoyl)-4-[*o*-(*o*-hydroxymethylphenylthio)benzyl]semicarbazide;
1-(2*R*-2-Aminobutanoyl)-4-(*o*-bromobenzyl)semicarbazide;
1-(2*R*-2-Aminobutanoyl)-4-(2-chloro-6-fluorobenzyl)semicarbazide;
1-(2*R*-2-Aminobutanoyl)-4-(*o*-ethoxybenzyl)semicarbazide;
1-(2*R*-2-Aminobutanoyl)-4-([1,1'-biphenyl-4-yl]methyl)semicarbazide;
1-(2*R*-2-Aminobutanoyl)-4-([1,1'-biphenyl-2-yl]methyl)semicarbazide;
1-(2*R*-2-Aminobutanoyl)-4-(*p*-[thiophen-2-yl]benzyl)semicarbazide;
1-(2*R*-2-Aminobutanoyl)-4-(*o*-[*N*-morpholinyl]benzyl)semicarbazide;
1-(2*R*-2-Aminobutanoyl)-4-(1-naphthylmethyl)semicarbazide;
1-(2*R*-2-Aminobutanoyl)-4-(*o*-[*N*-piperidinyl]benzyl)semicarbazide; and
1-(2*R*-2-Aminobutanoyl)-4-(*o*-methylthiobenzyl)semicarbazide.

29. A compound according to any of the preceding claims, which in the DPP-I assay exhibits an IC₅₀ value of less than 500 *µ*M such as, e.g., less than 100 *µ*M, less than 50 *µ*M, less than 1 *µ*M, less than 500 nM, less than 100 nM or less than 50 nM.

30. A compound according to any one of claims 1-29 for use as a protease inhibitor.

31. A compound according to claim 30 for use as a cysteine protease inhibitor.

32. A compound according to any of claims 1-31 for use in the treatment, prophylaxis and/or diagnosis of inflammation, type2 diabetes, asthma, severe influenza, respiratory syncytial virus infection, CD8 T cell inhibition, inflammatory bowel diseases, psoriasis, atopic dermatitis, Papillon Lefevre syndrome, Haim Munk syndrome, gum disease, periodontitis, rheumatoid arthritis, Huntington's disease, Chagas' disease, Alzheimer's disease, sepsis or for application in target cell apoptosis.

33. A pharmaceutical composition comprising, as an active substance, a compound as defined in any of claims 1-32 or a pharmaceutically acceptable salt thereof together with a pharmaceutically acceptable carrier or diluent.

34. A pharmaceutical composition according to claim 33 in unit dosage form, comprising from about 1 µg to about 1000 mg such as, e.g., from about 10 µg to about 500 mg, from about 0.05 to about 100 mg or from about 0.1 to about 50 mg, of the active substance.

35. A pharmaceutical composition according to claim 33 or 34 for oral, nasal, transdermal, pulmonal or parenteral administration.

36. Use of a compound as defined in any of claims 1-32 for the preparation of a medicament.

37. The use of a compound of formula (I) 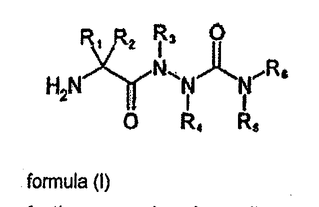 for the preparation of a medicament for modulating DPP-1 levels in a subject, or for the the treatment, prophylaxis and/or diagnosis of inflammation, type2 diabetes, asthmea, severe influenza, respiratory syncytial virus infection, CD8 T cell inhibition, inflammatory bowel diseases, psoriasis, atopic dermatitis, Papillon Lefevre syndrome, Haim Munk syndrome, gum disease, periodontitis, rheumatoid arthritis, Huntingtin's disease, Chagas' disease, Alzheimer's disease, sepsis or for application in target cell apoptosis.
wherein
R₁ and R₂ independently of each other are hydrogen, C₁₋₆alkyl, C₂₋₆alkenyl, C₂₋₆alkynyl, an unsubstituted or substituted C₃₋₁₀cycloalkyl group, an unsubstituted or substituted C₅₋₁₀cycloalkenyl group, an unsubstituted or substituted C₃₋₇heterocycloalkyl group, an unsubstituted or substituted C₁₋₆alkylaryl group but not benzyl when R₆ are phenyl, an unsubstituted or substituted C₁₋₆alkylheteroaryl group, an unsubstituted or substituted aryl group, an unsubstituted or substituted heteroaryl group, an unsubstituted or substituted aryl-C₁₋₅alkyl group, an unsubstituted or substituted heteroaryl-C₁₋₅alkyl group, an unsubstituted or substituted C₃₋₇cycloalkyl-C₁₋₅alkyl group, an unsubstituted or substituted C₃₋₇heterocycloalkyl-C₁₋₅alkyl group, or R₁ and R₂ together form an unsubstituted or substituted C₃₋₁₀cycloalkyl group or an unsubstituted or substituted C₃₋₇ heterocycloalkyl group;
R₃ is hydrogen, C₁₋₆alkyl, or R₃ and R₁ together form an unsubstituted or substituted C₃₋₁₀ cycloalkyl group or an unsubstituted or substituted C₃₋₇heterocycloalkyl group, or R₃ and R₂ together form an unsubstituted or substituted C₃₋₁₀cycloalkyl group or an unsubstituted or substituted C₃₋₇heterocycloalkyl group;
R₄ and R₅ independently of each other are hydrogen or C₁₋₆alkyl;
R₆ is C₁₋₆alkyl, -CH(R₇)CONH(R₈), C₂₋₆alkenyl, C₂₋₆alkynyl, an unsubstituted or substituted C₃₋₁₀cycloalkyl group, an unsubstituted or substituted C₅₋₁₀cycloalkenyl group, an unsubstituted or substituted C₃₋₇heterocycloalkyl group, an unsubstituted or substituted C₃₋₇cycloalkyl-C₁₋₅alkyl group, an unsubstituted or substituted C₃₋₇ heterocycloalkyl-C₁₋₅alkyl group, an unsubstituted or substituted C₁₋₆alkylaryl group, an unsubstituted or substituted C₁₋₆alkyl-heteroaryl group, an unsubstituted or substituted aryl group, an unsubstituted or substituted heteroaryl group, an unsubstituted or substituted aryl-C₁₋₅alkyl group, an unsubstituted or substituted heteroaryl-C₁₋₅alkyl group;
R₇ is hydrogen, an unsubstituted or substituted C₁₋₆alkyl group, an unsubstituted or substituted C₃₋₁₀cycloalkyl group, an unsubstituted or substituted C₁₋₆alkylaryl group, an unsubstituted or substituted C₁₋₆alkylheteroaryl group or a side chain of a natural or non-natural amino acid residue;
R₈ is hydrogen, an unsubstituted or substituted C₁₋₆alkyl group, an unsubstituted or substituted C₃₋₁₀cycloalkyl group, an unsubstituted or substituted C₁₋₆alkylaryl group, an unsubstituted or substituted C₁₋₆alkylheteroaryl group, a side chain of a natural or non-natural amino acid residue or a group -CH(R₇)CONH(R₉);
R₉ is hydrogen, an unsubstituted or substituted C₁₋₆alkyl group, an unsubstituted or substituted C₃₋₁₀cycloalkyl group, an unsubstituted or substituted C₁₋₆alkylaryl group or an unsubstituted or substituted C₁₋₆alkylheteroaryl group, wherein a substituted group is substituted with one, two or three substituents independently selected from the group consisting of C₁₋₆alkyl, C₁₋₆alkylthio, C₁₋₆alkylcarbonyl, C₁₋₆-N-alkylamide, C₁₋₆alkoxy, dialkylamino-C₁₋₆alkyl, amide, hydroxy, carboxy, amino, halogen, trifluoromethyl, trifluoromethoxy, trifluoromethylthio and cyano.

38. A compound of formula (I) 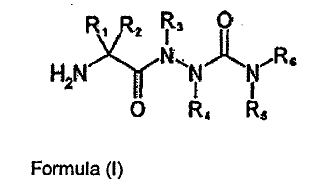 Wherein R₁, R₂, R₃, R₄, R₅ and R₆ are as defined in any one of claims 1 - 28,
with the proviso that the compound is not Ile-Gly^{a}-Leu-Met-NH₂, or Tyr-AzAla-Gly-Phe-Leu,
wherein Gly^{a} is an α-aza-amino acid residue.

## Patentansprüche

1. Verbindung der Formel (I) zur Verwendung in der Medizin
oder ein pharmazeutisch verträgliches/verträglicher Salz oder Ester davon, worin
R₁ und R₂ unabhängig voneinander Wasserstoff, C₁₋₆-Alkyl, C₂₋₆-Alkenyl, C₂₋₆-Alkinyl, eine unsubstituierte oder substituierte C₃₋₁₀-Cycloalkylgruppe, eine unsubstituierte oder substituierte C₅₋₁₀-Cycloalkenylgruppe, eine unsubstituierte oder substituierte C₃₋₇-Heterocycloalkylgruppe, eine unsubstituierte oder substituierte C₁₋₆-Alkylarylgruppe, aber nicht Benzyl sind, wenn R₆ Phenyl ist, eine unsubstituierte oder substituierte C₁₋₆-Alkylheteroarylgruppe, eine unsubstituierte oder substituierte Arylgruppe, eine unsubstituierte oder substituierte Heteroarylgruppe, eine unsubstituierte oder substituierte Aryl-C₁₋₅-alkylgruppe, eine unsubstituierte oder substituierte Heteroaryl-C₁₋₅-alkylgruppe, eine unsubstituierte oder substituierte C₃₋₇-Cycloalkyl-C₁₋₅-alkylgruppe, eine unsubstituierte oder substituierte C₃₋₇-Heterocycloalkyl-C₁₋₅-alkylgruppe sind, oder R₁ und R₂ bilden zusammen eine unsubstituierte oder substituierte C₃₋₁₀-Cycloalkylgruppe oder eine unsubstituierte oder substituierte C₃₋₇-Heterocycloalkylgruppe;
R₃ Wasserstoff oder C₁₋₆-Alkyl ist, oder R₃ und R₁ bilden zusammen eine unsubstituierte oder substituierte C₃₋₁₀-Cycloalkylgruppe oder eine unsubstituierte oder substituierte C₃₋₇-Heterocycloalkylgruppe, oder R₃ und R₂ bilden zusammen eine unsubstituierte oder substituierte C₃₋₁₀-Cycloalkylgruppe oder eine unsubstituierte oder substituierte C₃₋₇-Heterocycloalkylgruppe;
R₄ und R₅ unabhängig voneinander Wasserstoff oder C₁₋₆-Alkyl sind;
R₆ C₁₋₆-Alkyl, -CH(R₇) CONH(R₈), C₂₋₆-Alkenyl, C₂₋₆-Alkinyl, eine unsubstituierte oder substituierte C₃₋₁₀-Cycloalkylgruppe, eine unsubstituierte oder substituierte C₅₋₁₀-Cycloalkenylgruppe, eine unsubstituierte oder substituierte C₃₋₇-Heterocycloalkylgruppe, eine unsubstituierte oder substituierte C₃₋₇-Cycloalkyl-C₁₋₅-alkylgruppe, eine unsubstituierte oder substituierte C₃₋₇-Heterocycloalkyl-C₁₋₅-alkylgruppe, eine unsubstituierte oder substituierte C₁₋₆-Alkylarylgruppe, eine unsubstituierte oder substituierte C₁₋₆-Alkyl-Heteroarylgruppe, eine unsubstituierte oder substituierte Arylgruppe, eine unsubstituierte oder substituierte Heteroarylgruppe, eine unsubstituierte oder substituierte Aryl-C₁₋₅-alkylgruppe, eine unsubstituierte oder substituierte Heteroaryl-C₁₋₅-alkylgruppe ist;
R₇ Wasserstoff, eine unsubstituierte oder substituierte C₁₋₆-Alkylgruppe, eine unsubstituierte oder substituierte C₃₋₁₀-Cycloalkylgruppe, eine unsubstituierte oder substituierte C₁₋₆-Alkylarylgruppe, eine unsubstituierte oder substituierte C₁₋₆-Alkylheteroarylgruppe oder eine Seitenkette eines natürlichen oder nicht-natürlichen Aminosäurerests ist;
R₈ Wasserstoff, eine unsubstituierte oder substituierte C₁₋₆-Alkylgruppe, eine unsubstituierte oder substituierte C₃₋₁₀-Cycloalkylgruppe, eine unsubstituierte oder substituierte C₁₋₆-Alkylarylgruppe, eine unsubstituierte oder substituierte C₁₋₆-Alkylheteroarylgruppe, eine Seitenkette eines natürlichen oder nicht-natürlichen Aminosäurerests oder eine Gruppe -CH (R₇) CONH (R₉) ist;
R₉ Wasserstoff, eine unsubstituierte oder substituierte C₁₋₆-Alkylgruppe, eine unsubstituierte oder substituierte C₃₋₁₀-Cycloalkylgruppe, eine unsubstituierte oder substituierte C₁₋₆-Alkylarylgruppe oder eine unsubstituierte oder substituierte C₁₋₆-Alkylheteroarylgruppe ist;
wobei eine substituierte Gruppe mit einem, zwei oder drei Substituenten substituiert ist, unabhängig ausgewählt aus der Gruppe, bestehend aus C₁₋₆-Alkyl, C₁₋₆-Alkylthio, C₁₋₆-Alkylcarbonyl, C₁₋₆-N-Alkylamid, C₁₋₆-Alkoxy, Dialkylamino-C₁₋₆-alkyl, Amid, Hydroxy, Carboxy, Amino, Halogen, Trifluormethyl, Trifluormethoxy, Trifluormethylthio und Cyano; mit der Maßgabe, dass die Verbindung nicht Ile-Gly^{a}-Leu-Met-NH₂ ist, worin Gly^{a} ein α-Aza-aminosäurerest ist.

2. Verbindung nach Anspruch 1, worin R₁ und R₂ unabhängig voneinander Wasserstoff, C₁₋₆-Alkyl, eine unsubstituierte oder substituierte Phenylgruppe, eine unsubstituierte oder substituierte C₁₋₆-Alkylarylgruppe oder eine unsubstituierte oder substituierte C₁₋₆-Alkylheteroarylgruppe sind.

3. Verbindung nach Anspruch 1 oder 2, worin R₁ und R₂ unabhängig voneinander ausgewählt sind aus Methyl, Ethyl, Propyl, Butyl, Iso-Butyl, Benzyl, 1-Naphthylmethyl, 2-Naphthylmethyl und (4-Pyridyl)methyl.

4. Verbindung nach Anspruch 1 oder 2, worin R₁ C₁₋₆-Alkyl und R₂ H ist.

5. Verbindung nach Anspruch 1, worin R₁ und R₂ zusammen eine unsubstituierte oder substituierte C₃₋₁₀-Cycloalkylgruppe oder eine unsubstituierte oder substituierte C₃₋₇-Heterocycloalkylgruppe bilden.

6. Verbindung nach Anspruch 5, worin R₁ und R₂ zusammen eine unsubstituierte oder substituierte Cyclohexylgruppe bilden.

7. Verbindung nach einem der vorstehenden Ansprüche, worin R₃ Wasserstoff oder Methyl ist.

8. Verbindung nach einem der Ansprüche 1-6, worin R₁ und R₃ zusammen eine unsubstituierte oder substituierte C₃₋₁₀-Cycloalkylgruppe oder eine unsubstituierte oder substituierte C₃₋₇-Heterocycloalkylgruppe bilden.

9. Verbindung nach Anspruch 1, worin R₂ und R₃ zusammen eine unsubstituierte oder substituierte C₃₋₁₀-Cycloalkylgruppe oder eine unsubstituierte oder substituierte C₃₋₇-Heterocycloalkylgruppe bilden.

10. Verbindung nach einem der vorstehenden Ansprüche, worin R₄ Wasserstoff oder Methyl ist.

11. Verbindung nach einem der vorstehenden Ansprüche, worin R₅ Wasserstoff oder Methyl ist.

12. Verbindung nach einem der Ansprüche 1-7, 10-11, worin mindestens einer von R₃ und R₄ und R₅ Wasserstoff ist.

13. Verbindung nach Anspruch 12, worin R₃ und R₄ und R₅ Wasserstoff sind.

14. Verbindung nach einem der vorstehenden Ansprüche, worin R₆ -CH(R₇) CONH(R₈), C₁₋₆-Alkyl, eine unsubstituierte oder substituierte C₁₋₆-Alkylarylgruppe oder eine unsubstituierte oder substituierte C₁₋₆-Alkylheteroarylgruppe ist.

15. Verbindung nach einem der vorstehenden Ansprüche, worin R₆ eine unsubstituierte oder substituierte Benzylgruppe ist.

16. Verbindung nach einem der Ansprüche 1-14, worin R₆ die Gruppe -CH(R₇)CONH(R₈) ist.

17. Verbindung nach Anspruch 1, worin R₆ ausgewählt ist aus 2S-3-Phenylpropan-2-yl-amid, 2S-N-[2S-3-(m-Fluorphenyl)propan-2-yl-amid]-3-cyclohexylpropan-2-yl-amid, 2S-N-[2S-3-(m-Fluorphenyl)propan-2-yl-amid]-4-phenylbutan-2-yl-amid, 2S-N-[2S-3-(Indol-3-yl)propan-2-yl-amid]-3-phenylpropan-2-yl-amid, 2S-N-[2S-3-(m-Fluorphenyl)propan-2-yl-amid]-3-methylbutan-2-yl-amid, 2S-N-[2S-3-(Indol-3-yl)propan-2-yl-amid]-butan-2-yl-amid, 2S-N-[2S-3-(Indol-3-yl)propan-2-yl-amid]-2-phenylethan-2-yl-amid, 2S-N-[2S-3-(Indol-3-yl)propan-2-yl-amid]-4-phenylbutan-2-yl-amid, 2S-N-[2S-3-(m-Fluorphenyl)propan-2-yl-amid]-3-(indol-3-yl)propan-2-yl-amid, 2S-N-[2S-3-(m-Methoxyphenyl)propan-2-yl-amid]-3-(p-chlorphenyl)propan+2-yl-amid, 2S-N-[2S-3-(1,1'-Biphenyl-4-yl)propan-2-yl-amid]-3-phenylpropan-2-yl-amid, 2S-N-[2S-3-(m-Methoxyphenyl)propan-2-yl-amid]-3-(1,1'-biphenyl-4-yl)propan-2-yl-amid, 2S-N-[2S-3-(1,1'-Biphenyl-4-yl)propan-2-yl-amid]-3-(p-hydroxyphenyl)propan-2-yl-amid, 2S-N-[2S-4-Methylpentan-2-yl-amid]-3, (Benzo[b]thiophen-3-yl)propan-2-yl-amid, 2S-N-[2S-3-(2-Naphthyl)propan-2-yl-amid]-4-phenylbutan-2-yl-amid, 2S-N-[2S-3-(m-Methoxyphenyl)propan-2-yl-amid]-4-phenylbutan-2-yl-amid, 2S-N-[2S-3-(Benzo[b]thiophen-3-yl)propan-2-yl-amid]-4-phenylbutan-2-yl-amid, 2S-N-[2S-3-(p-chlorphenyl)propan-2-yl-amid]-4-phenylbutan-2-yl-amid, 2S-N-[2S-3-(m-Fluorphenyl)propan-2-yl-amid]-3-(2-naphthyl)propan-2-yl-amid, 2S-N-[2S-3-(m-Fluorphenyl)propan-2-yl-amid]-3-(1,1'-biphenyl-4-yl)propan-2-yl-amid, 2-Phenylethyl, 2-(p-Hydroxyphenyl)ethyl, Benzyl, o-Chlorbenzyl, 2-Furfuryl, 2-(3-Indolyl)ethyl, 1-Naphthylmethyl, 4-Piperonyl, p-Chlorbenzyl, 2-(Thiophen-2-yl)ethyl, m-Fluorbenzyl, o-Fluorbenzyl, p-Fluorbenzyl, o-Trifluormethylbenzyl, m-Chlorbenzyl, o-Methoxybenzyl, o-Methylbenzyl, alpha-Methylbenzyl, o-(o-Hydroxymethylphenylthio)benzyl, o-Brombenzyl, 2-Chlor-6-fluorbenzyl, o-Ethoxybenzyl, [1,1'-Biphenyl-4-yl]methyl, [1,1'-Biphenyl-2-yl]methyl, p-[Thiophen-2-yl]benzyl, o-[N-Morpholinyl]benzyl, o-[N-piperidinyl]benzyl und o-Methylthiobenzyl.

18. Verbindung nach einem der Ansprüche 1-14, worin R₆ eine unsubstituierte oder substituierte [1,1'-Biphenyl-2-yl]methylgruppe oder eine unsubstituierte oder substituierte [1,1'-Biphenyl-4-yl]methylgruppe ist.

19. Verbindung nach einem der Ansprüche 1-14, 16, worin R₇ Wasserstoff, eine unsubstituierte oder substituierte C₁₋₆-Alkylgruppe, eine unsubstituierte oder substituierte C₃₋₁₀-Cycloalkylgruppe, eine unsubstituierte oder substituierte C₁₋₆-Alkylarylgruppe, eine unsubstituierte oder substituierte C₁₋₆-Alkylheteroarylgruppe ist.

20. Verbindung nach Anspruch 19, worin R₇ eine unsubstituierte oder substituierte C₁₋₆-Alkylarylgruppe oder eine unsubstituierte oder substituierte C₁₋₆-Alkylheteroarylgruppe ist.

21. Verbindung nach Anspruch 20, worin R₇ 2-Phenylethyl ist.

22. Verbindung nach einem der Ansprüche 1-14, 16-21, worin R₈ Wasserstoff, -CH(R₇)CONH(R₉) oder eine unsubstituierte oder substituierte C₁₋₆-Alkylgruppe ist.

23. Verbindung nach Anspruch 22, worin R₈ -CH(R₇)CONH(R₉) und R₇ eine unsubstituierte oder substituierte C₁₋₆-Alkylarylgruppe oder eine unsubstituierte oder substituierte C₁₋₆-Alkyl-heteroarylgruppe ist.

24. Verbindung nach Anspruch 23, worin R₇ (Indol-3-yl)methyl oder (m-Fluorphenyl)methyl ist.

25. Verbindung nach Anspruch 23, worin R₉ Wasserstoff oder eine unsubstituierte oder substituierte C₁₋₆-Alkylgruppe ist.

26. Verbindung nach Anspruch 25, worin R₉ Wasserstoff ist.

27. Verbindung nach Anspruch 1, ausgewählt aus der Gruppe, bestehend aus
1-(2S-2-Aminopropionyl)-4-(2S-3-phenylpropan-2-yl-amid)semicarbazid;
1-(Aminoacetyl)-4-(2S-3-Phenylpropan-2-yl-amid)semicarbazid;
1-(2S-2-Aminopropionyl)-1-methyl-4-(2S-3-phenylpropan-2-yl-amid)semicarbazid;
1-(1-Aminocyclohexan-1-carbonyl)-4-[2S-N-[2S-3-(m-fluorphenyl)propan-2-yl-amid]-3-cyclohexylpropan-2-yl-amid]semicarbazid;
1-(1-Aminocyclohexan-1-carbonyl)-4-[2S-N-[2S-3-(m-fluorphenyl)propan-2-yl-amid]-4-phenylbutan-2-yl-amid]semicarbazid;
1-(2S-2-Aminobutanoyl)-4-[2S-N-[2S-3-(indol-3-yl)propan-2-yl-amide]-3-phenylpropan-2-yl-amid]semicarbazid;
1-(2S-2-Aminopropanoyl)-4-[2S-N-[2S-3-(indol-3-yl)propan-2-yl-amid]-3-phenylpropan-2-yl-amid]semicarbazid;
1-(2S-3-Naphthyl-2-propanoyl)-4-[2S-N-[2S-3-(m-fluorphenyl)propan-2-yl-amid]-3-methylbutan-2-yl-amid]semicarbazid;
1-[2S-2-Amino-3-(4-pyridyl)propanoyl]-4-[2S-N-(2S-3-(indol-3-yl)propan-2-yl-amid)-butan-2-yl-amid]semicarbazid;
1-[2S-2-Amino-3-(4-pyridyl)propanoyl]-4-[2S-N-(2S-3-(indol-3-yl)propan-2-yl-amid)-2-phenylethan-2-yl-amid]semicarbazid;
1-(2S-2-Aminobutanoyl)-4-[2S-N-[2S-3-(indol-3-yl)propan-2-yl-amid]-4-phenylbutan-2-yl-amid]semicarbazid;
1-(2S-2-Aminobutanoyl)-4-[2S-N-[2S-3-(m-fluorphenyl)propan-2-yl-amid]-3-(indol-3-yl)propan-2-yl-amid]semicarbazid;
1-(2S-2-Aminobutanoyl),-4-[2S-N-[2S-3-(m-methoxyphenyl)propan-2-yl-amid]-3-(p-chlorphenyl)propan-2-yl-amid]semicarbazid;
1-(2S-2-Aminopentanoyl)-4-[2S-N-[2S-3-(m-methoxyphenyl)propan-2-yl-amid]-3-(p-chlorphenyl)propan-2-yl-amid]semicarbazid;
1-(2S-2-Amino-4-phenylbutanoyl)-4-[2S-N-[2S-3-(1,1'-biphenyl-4-yl)propan-2-yl-amid]-3-phenylpropan-2-yl-amid]semicarbazid;
1-(2S-2-Aminopentanoyl)-4-[2S-N-[2S-3-(m-methoxyphenyl)propan-2-yl-amid]-3-(1,1'-biphenyl-4-yl)propan-2-yl-amid]semicarbazid;
1-(2S-2-Aminobutanoyl)-4-[2S-N-[2S-3-(1,1'-biphenyl-4-yl)propan-2-yl-amid]-3-(p-hydroxyphenyl)propan-2-yl-amid]semicarbazid;
1-(2S-2-Aminobutanoyl)-4-[2S-N-[2S-4-methylpentan-2-yl-amid]-3-(benzo[b]thiophen-3-yl)-propan-2-yl-amid]semicarbazid;
1-(2S-2-Aminobutanoyl)-4-[2S-N-[2S-3-(2-naphthyl)propan-2-yl-amid]-4-phenylbutan-2-yl-amid]semicarbazid;
1-(2S-2-Aminobutanoyl)-4-[2S-N-[2S-3-(m-methoxyphenyl)propan-2-yl-amid]-4-phenylbutan-2-yl-amid]semicarbazid;
1-(2S-2-Aminobutanoyl)-4-[2S-N-[2S-3-(benzo[b]thiophen-3-yl)propan-2-yl-amid]-4-phenylbutan-2-yl-amid]semicarbazid;
1-(2S-2-Aminobutanoyl)-4-[2S-N-[2S-3-(p-chlorphenyl)propan-2-yl-amid]-4-phenylbutan-2-yl-amid]semicarbazid;
1-(2S-2-Aminopentanoyl)-4-[2S-N-[2S-3-(2-naphthyl)propan-2-yl-amid]-4-phenylbutan-2-yl-amid]semicarbazid;
1-(2S-2-Aminobutanoyl)-4-[2S-N-[2S-3-(m-fluorphenyl)propan-2-yl-amid]-3-(2-naphthyl)propan-2-yl-amid]semicarbazid;
1-(2S-2-Aminobutanoyl)-4-[2S-N-[2S-3-(m-fluorophenyl)propan-2-yl-amid]-3-(1,1'-biphenyl-4-yl)propan-2-yl-amid]semicarbazid;
1-(2S-2-Aminopropionyl)-4-(2-phenylethyl)semicarbazid;
1-(2S-2-Aminopropionyl)-4-(2-phenylethyl)-4-methylsemicarbazid;
1-(2S-2-Aminopropionyl)-4-[2-(p-hydroxyphenyl)ethyl]semicarbazid;
1-(2S-2-Aminopropionyl)-4-benzylsemicarbazid;
1-(2S-2-Aminopropionyl)-4-(o-chlorbenzyl)semicarbazid;
1-(2S-2-Aminopropionyl)-4-(2-furfuryl)semicarbazid;
1-(2S-2-Aminopropionyl)-4-[2-(3-indolyl)ethyl]semicarbazid;
1-(2S-2-aminopropionyl)-4-(1-naphthylmethyl)semicarbazid;
1-(2S-2-Aminopropionyl)-4-piperonylsemicarbazid;
1-(2S-2-Aminopropionyl)-4-(p-chlorbenzyl)semicarbazid;
1-(2S-2-aminopropionyl)-4-[2-(thiophen-2-yl)ethyl]semicarbazid,
1-(2S-2-Aminopropionyl)-4-(m-fluorbenzyl)semicarbazid;
1-(2S-2-aminopropionyl)-4-(o-fluorbenzyl)semicarbazid;
1-(2S-2-Aminopropionyl)-4-(p-fluorbenzyl)semicarbazid;
1-(2S-2-Aminopropionyl)-4-(o-trifluormethylbenzyl)semicarbazid;
1-(2S-2-Aminopropionyl)-4-(m-chlorbenzyl)semicarbazid;
1-(2S-2-Aminopropionyl)-4-(o-methoxybenzyl)semicarbazid;
1-(2S-2-Aminopropionyl)-4-(o-methylbenzyl)semicarbazid;
1-(2S-2-Aminopropionyl)-4-(alpha-methylbenzyl)semicarbazid;
1-(2S-2-Aminobutanoyl)-4-(o-chlorbenzyl)semicarbazid;
1-(2S-2-Aminovaleryl)-4-(o-chlorbenzyl)semicarbazid;
1-(2S-2-Aminohexanoyl)-4-(o-chlorbenzyl)semicarbazid;
1-(2S-2-Aminoisocaproyl)-4-(o-chlorbenzyl)semicarbazid;
1-(1-Aminocyclohexan-1-carbonyl)-4-(o-chlorbenzyl)semicarbazid;
1-(2S-2-Amino-3-phenyl-propionyl)-4-(o-chlorbenzyl)semicarbazid;
1,1-(2S-1,4-Cyclo-aminobutanoyl)-4-(o-chlorbenzyl)semicarbazid;
1,1-(2R-1,4-Cyclo-aminobutanoyl)-4-(o-chlorbenzyl)semicarbazid;
1-(2S-2-Aminobutanoyl)-4-[2S-N-[2S-3-(m-fluorphenyl)propan-2-yl-amid]-4-phenylbutan-2-yl-amid]semicarbazid;
1-(2S-2-Aminobutanoyl)-4-[o-(o-hydroxymethylphenylthio)benzyl]semicarbazid;
1-(2S-2-Aminobutanoyl)-4-(o-brombenzyl)semicarbazid;
1-(2S-2-Aminobutanoyl)-4-(2-chlor-6-fluorbenzyl)semicarbazid;
1-(2S-2-Aminobutanoyl)-4-(o-ethoxybenzyl)semicarbazid;
1-(2S-2-Aminobutanoyl)-4-([1,1'-biphenyl-4-yl]methyl)semicarbazid;
1-(2S-2-Aminobutanoyl)-4-([1,1'-biphenyl-2-yl]methyl)semicarbazid;
1-(2S-2-Aminobutanoyl)-4-(p-[thiophen-2-yl]benzyl)semicarbazid;
1-(2S-2-Aminobutanoyl)-4-(o-[N-morpholinyl]benzyl)semicarbazid;
1-(2S-2-Aminobutanoyl)-4-(1-naphthylmethyl)semicarbazid;
1-(2S-2-Aminobutanoyl)-4-(o-[N-piperidinyl]benzyl)semicarbazid; und
1-(2S-2-Aminobutanoyl)-4-(o-methylthiobenzyl)semicarbazid.

28. Verbindung nach Anspruch 1, ausgewählt aus der Gruppe, bestehend aus
1-(2R-2-Aminopropionyl)-4-(2S-3-phenylpropan-2-yl-amid)semicarbazid;
1-(2R-2-Aminopropionyl)-1-methyl-4-(2S-3--phenylpropan-2-yl-amid)semicarbazid;
1-(2R-2-Aminobutanoyl)-4-[2S-N-[2S-3-(indol-3-yl)propan-2-yl-amid]-3-phenylpropan-2-yl-amid]semicarbazid;
1-(2R-2-Aminopropanoyl)-4-[2S-N-[2S-3-(indol-3-yl)propan-2-yl-amid]-3-phenylpropan-2-yl-amid]semicarbazid;
1-(2R-3-Naphthyl-2-propanoyl)-4-[2S-N-[2S-3-(m-fluorphenyl)propan-2-yl-amid]-3-methylbutan-2-yl-amid]semicarbazid;
1-[2R-2-Amino-3-(4-pyridyl)propanoyl]-4-[2S-N-(2S-3-(indol-3-yl)propan-2-yl-amid)-butan-2-yl-amid]semicarbazid;
1-[2R-2-Amino-3-(4-pyridyl)propanoyl]-4-[2S-N-(2S-3-(indol-3-yl)propan-2-yl-amid)-2-phenylethan-2-yl-amid]semicarbazid;
1-(2R-2-Aminobutanoyl)-4-[2S-N-[2S-3-(indol-3-yl)propan-2-yl-amid]-4-phenylbutan-2-yl-amid]semicarbazid;
1-(2R-2-Aminobutanoyl)-4-[2S-N-[2S-3-(m-fluorphenyl)propan-2-yl-amid]-3-(indol-3-yl)propan-2-yl-amid]semicarbazid;
1-(2R-2-Aminobutanoyl)-4-[2S-N-[2S-3-(m-methoxyphenyl)propan-2-yl-amid]-3-(p-chlorphenyl)propan-2-yl-amid]semicarbazid;
1-(2R-2-Aminopentanoyl)-4-[2S-N-[2S-3-(m-methoxyphenyl)propan-2-yl-amid]-3-(p-chlorphenyl)propan-2-yl-amid]semicarbazid;
1-(2R-2-Amino-4-phenylbutanoyl)-4-[2S-N-[2S-3-(1,1'-biphenyl-4-yl)propan-2-yl-amid]-3-phenylpropan-2-yl-amid]semicarbazid;
1-(2R-2-Aminopentanoyl)-4-[2S-N-[2S-3-(m-methoxyphenyl)propan-2-yl-amid]-3-(1,1'-biphenyl-4-yl)propan-2-yl-amid]semicarbazid;
1-(2R-2-Aminobutanoyl)-4-[2S-N-[2S-3-(1,1'-biphenyl-4-yl)propan-2-yl-amid]-3-(p-hydroxyphenyl)propan-2-yl-amid]semicarbazid;
1-(2R-2-Aminobutanoyl),-4-[2S-N-[2S-4-methylpentan-2-yl-amid]-3-(benzo[b]thiophen-3'-yl)propan-2-yl-amid]semicarbazid;
1-(2R-2-Aminobutanoyl)-4-[2S-N-[2S-3-(2-naphthyl)propan-2-yl-amid]-4-phenylbutan-2-yl-amid]semicarbazid;
1-(2R-2-Aminobutanoyl)-4-[2S-N-[2S-3-(m-methoxyphenyl)propan-2-yl-amid]-4-phenylbutan-2-yl-amid]semicarbazid;
1-(2R-2-Aminobutanoyl)-4-[2S-N-[2S-3-(benzo[b]thiophen-3-yl)propan-2-yl-amid]-4-phenylbutan-2-yl-amid]semicarbazid;
1-(2R-2-Aminobutanoyl)-4-[2S-N-[2S-3-(p-chlorphenyl)propan-2-yl-amid]-4-phenylbutari-2-yl-amid]semicarbazid;
1-(2R-2-Aminopentanoyl)-4-,[2S-N-[2S-3-(2-naphthyl)propan-2-yl-amid]-4-phenylbutan-2-yl-amid]semicarbazid;
1-(2R-2-Aminobutanoyl)-4-[2S-N-[2S-3-(m-fluorphenyl)propan-2-yl-amid]-3-(2-naphthyl)propan-2-yl-amid]semicarbazid;
1-(2R-2-Aminobutanoyl),-4-[2S-N-[2S-3-(m-fluorphenyl)propan-2-yl-amid]-3-(1,1'-biphenyl-4-yl)propan-2-yl-amid]semicarbazid;
1-(2R-2-Aminopropionyl)-4-(2-phenylethyl)semicarbazid;
1-(2R-2-Aminopropionyl)-4-(2-phenylethyl)-4-methylsemicarbazid;
1-(2R-2-Aminopropionyl)-4-[2-(p-hydroxyphenyl)ethyl]semicarbazid;
1-(2R-2-Aminopropionyl)-4-benzylsemicarbazid;
1-(2R-2-Aminopropionyl)-4-(o-chlorbenzyl)semicarbazid;
1-(2R-2-Aminopropionyl)-4-(2-furfuryl)semicarbazid;
1-(2R-2-Aminopropionyl)-4-[2-(3-indolyl)ethyl]semicarbazid;
1-(2R-2-Aminopropionyl)-4-(1-naphthylmethyl)semicarbazid;
1-(2R-2-Aminopropionyl)-4-piperonylsemicarbazid;
1-(2R-2-Aminopropionyl)-4-(p-chlorbenzyl)semicarbazid;
1-(2R-2-Aminopropionyl)-4-[2-(thiophen-2-yl)ethyl]semicarbazid;
1-(2R-2-Aminopropionyl)-4-(m-fluorbenzyl)semicarbazid;
1-(2R-2-Aminopropionyl)-4-(o-fluorbenzyl)semicarbazid;
1-(2R-2-Aminopropionyl)-4-(p-fluorbenzyl)semicarbazid;
1-(2R-2-Aminopropionyl)-4-(o-trifluormethylbenzyl)semicarbazid;
1-(2R-2-Aminopropionyl)-4-(m-chlorbenzyl)semicarbazid;
1-(2R-2-Aminopropionyl)-4-(o-methoxybenzyl)semicarbazid;
1-(2R-2-Aminopropionyl)-4-(o-methylbenzyl)semicarbazid;
1-(2R-2-Aminopropionyl)-4-(alpha-methylbenzyl)semicarbazid;
1-(2R-2-Aminobutanoyl)-4-(o-chlorbenzyl)semicarbazid;
1-(2R-2-Aminovaleryl)-4-(o-chlorbenzyl)semicarbazid;
1-(2R-2-Aminohexanoyl)-4-(o-chlorbenzyl)semicarbazid;
1-(2R-2-Aminoisocaproyl)-4-(o-chlorbenzyl)semicarbazid;
1-(2R-2-Amino-3-phenyl-propionyl)-4-(o-chlorbenzyl)semicarbazid;
1-(2R-2-Aminobutanoyl)-4-[2S-N-[2S-3-(m-fluorphenyl)propan-2-yl-amid]-4-phenylbutan-2-yl-amid]semicarbazid;
1-(2R-2-Aminobutanoyl)-4-[o-(o-hydroxymethylphenylthio)benzyl]semicarbazid;
1-(2R-2-Aminobutanoyl)-4-(o-brombenzyl)semicarbazid;
1-(2R-2-Aminobutanoyl)-4-(2-chlor-6-fluorbenzyl)semicarbazid;
1-(2R-2-Aminobutanoyl)-4-(o-ethoxybenzyl)semicarbazid;
1-(2R-2-Aminobutanoyl)-4-([1,1'-biphenyl-4-yl]methyl)semicarbaziel;
1-(2R-2-Aminobutanoyl)-4-([1,1'-biphenyl-2-yl]methyl)semicarbazid;
1-(2R-2-Aminobutanoyl)-4-(p-[thiophen-2-yl]benzyl)semicarbazid;
1-(2R-2-Aminobutanoyl)-4-(o-[N-morpholinyl]benzyl)semicarbazid;
1-(2R-2-Aminobutanoyl)-4-(1-naphthylmethyl)semicarbazid;
1-(2R-2-Aminobutanoyl)-4-(o-[N-piperidinyl]benzyl)semicarbazid; und
1-(2R-2-Aminobutanoyl)-4-(o-methylthiobenzyl)semicarbazid.

29. Verbindung nach einem der vorstehenden Ansprüche, die in dem DPP-I-Assay einen IC₅₀-Wert von weniger als 500 µM aufweist, wie zum Beispiel weniger als 100 µM, weniger als 50 µM, weniger als 1 µM, weniger als 500 nM, weniger als 100 nM oder weniger als 50 nM.

30. Verbindung nach einem der Ansprüche 1-29 zur Verwendung als Proteaseinhibitor.

31. Verbindung nach Anspruch 30 zur Verwendung als Cysteinproteaseinhibitor.

32. Verbindung nach einem der Ansprüche 1-31 zur Verwendung bei der Behandlung, Prophylaxe und/oder Diagnose von Entzündungen, Typ-2-Diabetes, Asthma, schwerer Influenza, respiratorischer Synzytium-Virusinfektion, CD8-T-Zellen-Hemmung, entzündlichen Darmerkrankungen, Psoriasis, atopischer Dermatitis, Papillon-Lefèvre-Syndrom, Haim-Munk-Syndrom, Zahnfleischerkrankung ("gum disease"), Periodontitis, rheumatoider Arthritis, Huntington-Krankheit, Chagas-Krankheit, Alzheimer-Krankheit, Sepsis oder zur Anwendung bei der Targetzellen-Apoptose.

33. Pharmazeutische Zusammensetzung, umfassend als Wirksubstanz eine Verbindung wie in einem der Ansprüche 1-32 definiert oder ein pharmazeutisch verträgliches Salz davon zusammen mit einem pharmazeutisch verträglichen Trägerstoff oder Verdünnungsmittel.

34. Pharmazeutische Zusammensetzung nach Anspruch 33 in Einheitsdosisform, umfassend von etwa 1 µg bis etwa 1000 mg, wie zum Beispiel von etwa 10 µg bis etwa 500 mg, von etwa 0,05 bis etwa 100 mg oder von etwa 0,1 bis etwa 50 mg der Wirksubstanz.

35. Pharmazeutische Zusammensetzung nach Anspruch 33 oder 34 für die orale, nasale, transdermale, pulmonale oder parenterale Verabreichung.

36. Verwendung einer Verbindung wie in einem der Ansprüche 1-32 definiert zur Herstellung eines Medikaments.

37. Verwendung einer Verbindung der Formel (I) 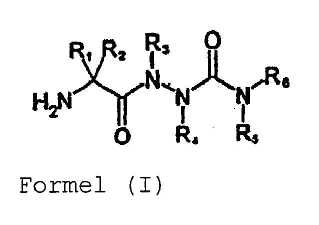 zur Herstellung eines Medikaments zur Modulierung der DPP-1-Spiegels in einem Patienten oder zur Behandlung, Prophylaxe und/oder Diagnose von Entzündungen, Typ-2-Diabetes, Asthma, schwerer Influenza, respiratorischer Synzytium-Virusinfektion, CD8-T-Zellen-Hemmung, entzündlichen Darmerkrankungen, Psoriasis, atopischer Dermatitis, Papillon-Lefevre-Syndrom, Haim-Munk-Syndrom, Zahnfleischerkrankung, Periodontitis, rheumatoider Arthritis, Huntington-Krankheit, Chagas-Krankheit, Alzheimer-Krankheit, Sepsis oder zur Anwendung bei der Targetzellen-Apoptose,
worin
R₁ und R₂ unabhängig voneinander Wasserstoff, C₁₋₆-Alkyl, C₂₋₆-Alkenyl, C₂₋₆-Alkinyl, eine unsubstituierte oder substituierte C₃₋₁₀-Cycloalkylgruppe, eine unsubstituierte oder substituierte C₅₋₁₀-Cycloalkenylgruppe, eine unsubstituierte oder substituierte C₃₋₇-Heterocycloalkylgruppe, eine unsubstituierte oder substituierte C₁₋₆-Alkylarylgruppe, aber nicht Benzyl sind, wenn R₆ Phenyl ist, eine unsubstituierte oder substituierte C₁₋₆-Alkylheteroarylgruppe, eine unsubstituierte oder substituierte Arylgruppe, eine unsubstituierte oder substituierte Heteroarylgruppe, eine unsubstituierte oder substituierte Aryl-C₁₋₅-alkylgruppe, eine unsubstituierte oder substituierte Heteroaryl-C₁₋₅-alkylgruppe, eine unsubstituierte oder substituierte C₃₋₇-Cycloalkyl-C₁₋₅-alkylgruppe, eine unsubstituierte oder substituierte C₃₋₇-Heterocycloalkyl-C₁₋₅-alkylgruppe sind, oder R₁ und R₂ bilden zusammen eine unsubstituierte oder substituierte C₃₋₁₀-Cycloalkylgruppe oder eine unsubstituierte oder substituierte C₃₋₇-Heterocycloalkylgruppe;
R₃ Wasserstoff oder C₁₋₆-Alkyl ist, oder R₃ und R₁ bilden zusammen eine unsubstituierte oder substituierte C₃₋₁₀-Cycloalkylgruppe oder eine unsubstituierte oder substituierte C₃₋₇-Heterocycloalkylgruppe, oder R₃ und R₂ bilden zusammen eine unsubstituierte oder substituierte C₃₋₁₀-Cycloalkylgruppe oder eine unsubstituierte oder substituierte C₃₋₇-Heterocycloalkylgruppe;
R₄ und R₅ unabhängig voneinander Wasserstoff oder C₁₋₆-Alkyl sind;
R₆ C₁₋₆-Alkyl, -CH(R₇)CONH(R₈), C₂₋₆-Alkenyl, C₂₋₆-Alkinyl, eine unsubstituierte oder substituierte C₃₋₁₀-Cycloalkylgruppe, eine unsubstituierte oder substituierte C₅₋₁₀-Cycloalkenylgruppe, eine unsubstituierte oder substituierte C₃₋₇-Heterocycloalkylgruppe, eine unsubstituierte oder substituierte C₃₋₇-Cycloalkyl-C₁₋₅-alkylgruppe, eine unsubstituierte oder substituierte C₃₋₇-Heterocycloalkyl-C₁₋₅-alkylgruppe, eine unsubstituierte oder substituierte C₁₋₆-Alkylarylgruppe, eine unsubstituierte oder substituierte C₁₋₆-Alkyl-Heteroarylgruppe, eine unsubstituierte oder substituierte Arylgruppe, eine unsubstituierte oder substituierte Heteroarylgruppe, eine unsubstituierte oder substituierte Aryl-C₁₋₅-alkylgruppe, eine unsubstituierte oder substituierte Heteroaryl-C₁₋₅-alkylgruppe ist;
R₇ Wasserstoff, eine unsubstituierte oder substituierte C₁₋₆-Alkylgruppe, eine unsubstituierte oder substituierte C₃₋₁₀-Cycloalkylgruppe, eine unsubstituierte oder substituierte C₁₋₆-Alkylarylgruppe, eine unsubstituierte oder substituierte C₁₋₆-Alkylheteroarylgruppe oder eine Seitenkette eines natürlichen oder nicht-natürlichen Aminosäurerests ist;
R₈ Wasserstoff, eine unsubstituierte oder substituierte C₁₋₆-Alkylgruppe, eine unsubstituierte oder substituierte C₃₋₁₀-Cycloalkylgruppe, eine unsubstituierte oder substituierte C₁₋₆-Alkylarylgruppe, eine unsubstituierte oder substituierte C₁₋₆-Alkylheteroarylgruppe, eine Seitenkette eines natürlichen oder nicht-natürlichen Aminosäurerests oder eine Gruppe -CH(R₇)CONH(R₉) ist;
R₉ Wasserstoff, eine unsubstituierte oder substituierte C₁₋₆-Alkylgruppe, eine unsubstituierte oder substituierte C₃₋₁₀-Cycloalkylgruppe, eine unsubstituierte oder substituierte C₁₋₆-Alkylarylgruppe oder eine unsubstituierte oder substituierte C₁₋₆-Alkylheteroarylgruppe ist;
wobei eine substituierte Gruppe mit einem, zwei oder drei Substituenten substituiert ist, unabhängig ausgewählt aus der Gruppe, bestehend aus C₁₋₆-Alkyl, C₁₋₆-Alkylthio, C₁₋₆-Alkylcarbonyl, C₁₋₆-N-Alkylamid, C₁₋₆-Alkoxy, Dialkylamino-C₁₋₆-alkyl, Amid, Hydroxy, Carboxy, Amino, Halogen, Trifluormethyl, Trifluormethoxy, Trifluormethylthio und Cyano.

38. Verbindung der Formel (I) 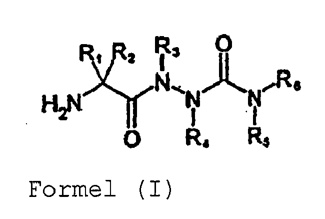 worin R₁, R₂, R₃, R₄, R₅ und R₆ wie in einem der Ansprüche 1-28 definiert sind, mit der Maßgabe, dass die Verbindung nicht Ile-Gly^{a}-Leu-Met-NH₂ oder Tyr-AzAla-Gly-Phe-Leu ist, worin Gly^{a} ein α-Aza-aminosäurerest ist.

## Revendications

1. Composé de formule (I) pour une utilisation en médecine 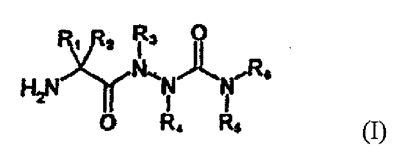 ou un sel ou un ester de celui-ci acceptable du point de vue pharmaceutique, formule dans laquelle
R₁ et R₂ sont indépendamment l'un de l'autre l'hydrogène, un alkyle en C₁ à C₆, un alcényle en C₂ à C₆, un alcynyle en C₂ à C₆, un groupe cycloalkyle en C₃ à C₁₀ substitué ou non substitué, un groupe cycloalcényle en C₅ à C₁₀ substitué ou non substitué, un groupe hétérocycloalkyle en C₃ à C₇ substitué ou non substitué, un groupe alkyl(C₁ à C₆)-aryle substitué ou non substitué mais pas benzyle lorsque R₆ est phényle, un groupe alkyl(C₁ à C₆)-hétéroaryle substitué ou non substitué, un groupe aryle substitué ou non substitué, un groupe hétéroaryle substitué ou non substitué, un groupe aryl-alkyle en C₁ à C₆ substitué ou non substitué, un groupe hétéroaryl-alkyle en C₁ à C₆ substitué ou non substitué, un groupe cycloalkyl(C₃ à C₇)-alkyle en C₁ à C₆ substitué ou non substitué, un groupe hétérocyloalkyl(C₃ à C₇)-alkyle en C₁ à C₆ substitué ou non substitué, ou R₁ et R₂ forment ensemble un groupe cycloalkyle en C₃ à C₁₀ substitué ou non substitué ou un groupe hétérocycloalkyle en C₃ à C₇ substitué ou non substitué;
R₃ est l'hydrogène, un alkyle en C₁ à C₆, ou R₃ et R₁ forment ensemble un groupe cycloalkyle en C₃ à C₁₀ substitué ou non substitué ou un groupe hétérocycloalkyle en C₃ à C₇ substitué ou non substitué, ou R₃ et R₂ forment ensemble un groupe cycloalkyle en C₃ à C₁₀ substitué ou non substitué ou un groupe hétérocycloalkyle en C₃ à C₇ substitué ou non substitué ;
R₄ et R₅ sont indépendamment l'un de l'autre l'hydrogène ou un alkyle en C₁ à C₆ ;
R₆ est alkyle en C₁ à C₆, -CH(R₇)CONH(R₈), un alcényle en C₂ à C₆, un alcynyle en C₂ à C₆, un groupe cycloalkyle en C₃ à C₁₀ substitué ou non substitué, un groupe cycloalcényle en C₅ à C₁₀ substitué ou non substitué, un groupe hétérocycloalkyle en C₃ à C₇ substitué ou non substitué, un groupe cycloalkyl(C₃ à C₇)-alkyle en C₁ à C₆ substitué ou non substitué, un groupe hétérocycloalkyl(C₃ à C₇)-alkyle en C₁ à C₆ substitué ou non substitué, un groupe alkyl(C₁ à C₆)-aryle substitué ou non substitué, un groupe alkyl(C₁ à C₆)-hétéroaryle substitué ou non substitué, un groupe aryle substitué ou non substitué, un groupe hétéroaryle substitué ou non substitué, un groupe aryl-alkyle en C₁ à C₆ substitué ou non substitué, un groupe hétéroaryl-alkyle en C₁ à C₆ substitué ou non substitué ;
R₇ est l'hydrogène, un groupe alkyle en C₁ à C₆ substitué ou non substitué, un groupe cycloalkyle en C₃ à C₁₀ substitué ou non substitué, un groupe alkyl(C₁ à C₆)-aryle substitué ou non substitué, un groupe alkyl(C₁ à C₆)-hétéroaryle substitué ou non substitué ou une chaîne latérale d'un résidu acide aminé naturel ou non naturel ;
R₈ est l'hydrogène, un groupe alkyle en C₁ à C₆ substitué ou non substitué, un groupe cycloalkyle en C₃ à C₁₀ substitué ou non substitué, un groupe alkyl(C₁ à C₆)-aryle substitué ou non substitué, un groupe alkyl(C₁ à C₆)-hétéroaryle substitué ou non substitué, une chaîne latérale d'un résidu acide aminé naturel ou non naturel ou un groupe -CH(R₇)CONH(R₉) ;
R₉ est l'hydrogène, un groupe alkyle en C₁ à C₆ substitué ou non substitué, un groupe cycloalkyle en C₃ à C₁₀ substitué ou non substitué, un groupe alkyl(C₁ à C₆)-aryle substitué ou non substitué ou un groupe alkyl(C₁à C₆)-hétéroaryle substitué ou non substitué ;
dans lesquels un groupe substitué est substitué par un, deux ou trois substituants choisis indépendamment dans le groupe consistant en un alkyle en C₁ à C₆, un alkylthio en C₁ à C₆, un alkylcarbonyle en C₁ à C₆, un N-alkylamide en C₁ à C₆, un alcoxy en C₁ à C₆, un dialkylamino-alkyle en C₁ à C₆, amide, hydroxy, carboxy, amino, halogène, trifluorométhyle, trifluorométhoxy, trifluorométhylthio et cyano ;
avec la restriction que le composé n'est pas Ile-Gly^{a}-Leu-Met-NH₂, Gly^{a} étant un résidu α-aza-acide aminé.

2. Composé selon la revendication 1, dans lequel R₁ et R₂, indépendamment l'un de l'autre, sont l'hydrogène, un alkyle en C1 à C6, un groupe phényle substitué ou non substitué, un groupe alkyl(C₁ à C₆)-aryle substitué ou non substitué ou un groupe alkyl(C₁ à C₆)-hétéroaryle substitué ou non substitué.

3. Composé selon la revendication 1 ou 2, dans lequel R₁ et R₂, indépendamment l'un de l'autre, sont choisis parmi méthyle, éthyle, propyle, butyle, iso-butyle, benzyle, 1-naphtylméthyle, 2-naphtylméthyle et (4-pyridyl)méthyle.

4. Composé selon la revendication 1 ou 2, dans lequel R₁ est un alkyle en C₁ à C₆ et R₂ est H.

5. Composé selon la revendication 1, dans lequel R₁ et R₂ forment ensemble un groupe cycloalkyle en C₃ à C₁₀ substitué ou non substitué ou un groupe hétérocycloalkyle en C₃ à C₇ substitué ou non substitué.

6. Composé selon la revendication 5, dans lequel R₁ et R₂ forment ensemble un groupe cyclohexyle substitué ou non substitué.

7. Composé selon l'une quelconque des revendications précédentes, dans lequel R₃ est l'hydrogène ou le méthyle.

8. Composé selon l'une quelconque des revendications 1 à 6, dans lequel R₁ et R₃ forment ensemble un groupe cycloalkyle en C₃ à C₁₀ substitué ou non substitué ou un groupe hétérocycloalkyle en C₃ à C₇ substitué ou non substitué.

9. Composé selon la revendication 1, dans lequel dans lequel R₂ et R₃ forment ensemble un groupe cycloalkyle en C₃ à C₁₀ substitué ou non substitué ou un groupe hétérocycloalkyle en C₃ à C₇ substitué ou non substitué.

10. Composé selon l'une quelconque des revendications précédentes, dans lequel R₄ est l'hydrogène ou le méthyle.

11. Composé selon l'une quelconque des revendications précédentes, dans lequel R₅ est l'hydrogène ou le méthyle.

12. Composé selon l'une quelconque des revendications 1 à 7, 10 et 11, dans lequel au moins l'un de R₃ et R₄ et R₅ est l'hydrogène.

13. Composé selon la revendication 12, dans lequel R₃ et R₄ et R₅ sont l'hydrogène.

14. Composé selon l'une quelconque des revendications précédentes, dans lequel R₆ est -CH(R₇)CONH(R₈), un alkyle en C1 à C6, un groupe alkyl(C1 à C6)-aryle substitué ou non substitué ou un groupe alkyl(C1 à C6)-hétéroaryle substitué ou non substitué.

15. Composé selon l'une quelconque des revendications précédentes, dans lequel R₆ est un groupe benzyle substitué ou non substitué.

16. Composé selon l'une quelconque des revendications 1 à 14, dans lequel R₆ est le groupe CH(R₇)CONH(R₈).

17. Composé selon la revendication 1, dans lequel R₆ est choisi parmi 2*S*-3-phénylpropan-2-yl-amide, 2S-N-[2S-3-(m-fluorophényl)propan-2-yl-amide]-3-cyclohexylpropan-2-yl-amide, 2S-N-[2S-3-(m-fluorophényl)propan-2-yl-amide]-4-phénylbutan-2-yl-amide, 2S-N-[2S-3-(indol-3-yl)propan-2-yl-amide]-3-phénylpropan-2-yl-amide, 2S-N-[2S-3-(m-fluorophényl)propan-2-yl-amide]-3-méthylbutan-2-yl-amide, 2S-N-[2S-3-(indol-3-yl)propan-2-yl-amide]-butan-2-yl-amide, 2S-N-[2S-3-(indol-3-yl)propan-2-yl-amide]-2-phényléthan-2-yl-amide, 2S-N-[2S-3-(indol-3-yl)propan-2-yl-amide]-4-phénylbutan-2-yl-amide, 2S-N-[2S-3-(m-fluorophényl)propan-2-yl-amide]-3-(indol-3-yl)propan-2-yl-amide, 2S-N-[2S-3-(m-méthoxyphényl)propan-2--yl-amide]-3-(p-chlorophényl)propan-2-yl-amide, 2S-N-[2S-3-(1,1'-biphényl-4-yl)propan-2-yl-amide]-3-phénylpropan-2-yl-amide, 2S-N-[2S-3-(m-méthoxyphényl)propan-2-yl-amide]-3-(1,1'-biphényl-4-yl)propan-2-yl-amide, 2S-N-[2S-3-(1,1'-biphényl-4-yl)propan-2-yl-amide]-3-(p-hydroxyphényl)propan-2-yl-amide, 2S-N-[2S-4-méthylpentan-2-yl-amide]-3-(benzo[b]thiophén-3-yl)propan-2-yl-amide, 2S-N-[2S-3-(2-naphtyl)propan-2-yl-amide]-4-phénylbutan-2-yl-amide, 2S-N-[2S-3-(m-méthoxyphényl)propan-2-yl-amide]-4-phénylbutan-2-yl -amide, 2S-N-[2S-3-(benzo[b]thiophén-3-yl)propan-2-yl-amide] -4-phénylbutan-2-yl-amide, 2S-N-[2S-3-(p-chlorophényl)propan-2-yl-amide]-4-phénylbutan-2-yl-amide, 2S-N-[2S-3-(m-fluorophényl)propan-2-yl-amide]-3-(2-naphtyl)propan-2-yl-amide, 2S-N-[2S-3-(m-fluorophényl)propan-2-yl-amide]-3-(1,1'-biphényl-4-yl)propan-2-yl-amide, 2-phényléthyle, 2-(p-hydroxyphényl)éthyle, benzyle, o-chlorobenzyle, 2-furfuryle, 2-(3-indolyl)éthyle, 1-naphtylméthyle, 4-pipéronyle, p-chlorobenzyle, 2-(thiophén-2-yl)éthyle, m-fluorobenzyle, o-fluorobenzyle, p-fluorobenzyle, o-trifluorométhylbenzyle, m-chlorobenzyle, o-méthoxybenzyle, o-méthylbenzyle, alpha-méthylbenzyle, o-(o-hydroxyméthylphénylthio)benzyle, o-bromobenzyle, 2-chloro-6-fluorobenzyle, o-éthoxybenzyle, [1,1'-biphényl-4-yl]méthyle, [1,1'-biphényl-2-yl]méthyle, p-[thiophén-2-yl]benzyle, o-[N-morpholinyl]benzyle, o-[N-pipéridinyl]benzyle et o-méthylthiobenzyle.

18. Composé selon l'une quelconque des revendications 1 à 14, dans lequel R₆ est un groupe [1,1'-biphényl-2-yl]méthyle substitué ou non substitué ou un groupe [1,1'-biphényl-4-yl]méthyle substitué ou non substitué.

19. Composé selon l'une quelconque des revendications 1 à 14, 16, dans lequel R₇ est l'hydrogène, un groupe alkyle en C₁ à C₆ substitué ou non substitué, un groupe cycloalkyle en C₃ à C₁₀ substitué ou non substitué, un groupe alkyl(C₁ à C₆)-aryle substitué ou non substitué, un groupe alkyl(C₁ à C₆)-hétéroaryle substitué ou non substitué.

20. Composé selon la revendication 19, dans lequel R₇ est un groupe alkyl(C₁ à C₆)-aryle substitué ou non substitué ou un groupe alkyl(C₁ à C₆)-hétéroaryle substitué ou non substitué.

21. Composé selon la revendication 20, dans lequel R₇ est 2-phényléthyle.

22. Composé selon l'une quelconque des revendications 1 à 14, 16 à 21, dans lequel R₈ est l'hydrogène, -CH(R₇)CONH(R₉) ou un groupe alkyle en C₁ à C₆ substitué ou non substitué.

23. Composé selon la revendication 22, dans lequel R₈ est -CH(R₇)CONH(R₉) et R₇ est un groupe alkyl(C₁ à C₆)-aryle substitué ou non substitué ou un groupe alkyl(C₁ à C₆)-hétéroaryle substitué ou non substitué.

24. Composé selon la revendication 23, dans lequel R₇ est (indol-3-yl)méthyle ou (m-fluorophényl)méthyle.

25. Composé selon la revendication 23, dans lequel R₉ est l'hydrogène ou un groupe alkyl(C₁ à C₆)aryle substitué ou non substitué.

26. Composé selon la revendication 25, dans lequel R₉ est l'hydrogène.

27. Composé selon la revendication 1 choisi dans le groupe consistant en
1-(2*S*-2-aminopropionyl)-4-(2*S*-3-phénylpropan-2-yl-amide) semicarbazide ;
1-(aminoacétyl)-4-(2*S*-3-phénylpropan-2-yl-amide) semicarbazide ;
1-(2*S*-2-aminopropionyl)-1-méthyl-4-(2*S*-3-phénylpropan-2-yl-amide) semicarbazide ;
1-(1-aminocyclohexane-1-carbonyl)-4-[2*S*-*N*-[2*S*-3-(*m*-fluorophényl)propan-2-yl-amide]-3-cyclohexylpropan-2-yl-amide]semicarbazide ;
1-(1-aminocyclohexane-1-carbonyl)-4-[2*S*-*N*-[2*S*-3-(*m*-fluorophényl)propan-2-yl-amide]-4-phénylbutan-2-yl-amide]semicarbazide ;
1-(2*S*-2-aminobutanoyl)-4-[2*S*-*N-*[2*S-*3-(indol-3-yl)propan-2-yl-amide]-3-phénylpropan-2-yl-amide]semicarbazide ;
1-(2*S*-2-aminopropanoyl)-4-[2*S*-*N*-[2*S*-3-(indol-3-yl)propan-2-yl-amide]-3-phénylpropan-2-yl-amide]semicarbazide ;
1-(2*S*-3-naphtyl-2-propanoyl)-4-[2*S*-*N*-[2*S*-3-(*m*-fluorophényl)propan-2-yl-amide]-3-méthylbutan-2-yl-amide]semicarbazide ;
1-[2*S*-2-amino-3-(4-pyridyl)propanoyl]-4-[2*S*-*N*-(2*S*-3-(indol-3-yl)propan-2-yl-amide)-butan-2-yl-amide]semicarbazide ;
1-[2*S*-2-amino-3-(4-pyridyl)propanoyl]-4-[2*S*-*N*-(2*S*-3-(indol-3-yl)propan-2-yl-amide)-2-phényléthan-2-yl-amide]semicarbazide ;
1-(2*S*-2-aminobutanoyl)-4-[2*S*-*N*-[2*S*-3-(indol-3-yl)propan-2-yl-amide]-4-phénylbutan-2-yl-amide]semicarbazide ;
1-(2*S*-2-aminobutanoyl)-4-[2S-*N*-[2*S*-3-(*m*-fluorophényl)propan-2-yl-amide]-3-(indol-3-yl)propan-2-yl-amide]semicarbazide ;
1-(2*S*-2-aminobutanoyl)-4-[2*S*-*N*-[2*S*-3-(*m*-méthoxyphényl)propan-2-yl-amide]-3-(p-chlorophényl)propan-2-yl-amide]semicarbazide ;
1-(2*S*-2-aminopentanoyl)-4-[2*S*-*N*-[2*S*-3-(*m*-méthoxyphényl)propan-2-yl-amide]-3-(*p*-chlorophényl)propan-2-yl-amide]semicarbazide ;
1-(2*S*-2-amino-4-phénylbutanoyl)-4-[2*S*-*N*-[2*S*-3-(1,1'-biphényl-4-yl)propan-2-yl-amide]-3-phénylpropan-2-yl-amide]semicarbazide ;
1-(2*S*-2-aminopentanoyl)-4-[2*S*-*N*-[2*S*-3*-*(*m*-méthoxyphényl)propan-2-yl-amide]-3-(1,1'-biphényl-4-yl)propan-2-yl-amide]semicarbazide ;
1-(2*S*-2-aminobutanoyl)-4-[2*S*-*N*-[2*S*-3-(1,1'-biphényl-4-yl)propan-2-yl-amide]-3-(*p-*hydroxyphényl)propan-2-yl-amide]semicarbazide ;
1-(2*S*-2-aminobutanoyl)-4-[2*S*-*N*-[2*S*-4-méthylpentan-2-yl-amide]-3-(benzo[b]thiophén-3-yl)propan-2-yl-amide]semicarbazide ;
1-(2*S*-2-aminobutanoyl)-4-[2*S*-*N*-[2*S*-3-(2-naphtyl)propan-2-yl-amide]-4-phénylbutan-2-yl-amide]semicarbazide ;
1-(2*S*-2-aminobutanoyl)-4-[2*S*-*N*-[2*S*-3-(*m*-méthoxyphényl)propan-2-yl-amide]-4-phénylbutan-2-yl-amide]semicarbazide ;
1-(2*S*-2-aminobutanoyl)-4-[2*S*-*N*-[2*S*-3-(benzo[b]thiophén-3-yl)propan-2-yl-amide]-4-phénylbutan-2-yl-amide]semicarbazide ;
1-(2*S*-2-aminobutanoyl)-4-[2*S*-*N*-[2*S*-3-(*p*-chlorophényl)propan-2-yl-amide]-4-phénylbutan-2-yl-amide]semicarbazide ;
1-(2*S*-2-aminopentanoyl)-4-[2*S*-*N*-[2*S*-3-(2-naphtyl)propan-2-yl-amide]-4-phénylbutan-2-yl-amide]semicarbazide ;
1-(2*S*-2-aminobutanoyl)-4-[2*S*-*N*-[2*S*-3-(*m*-fluorophényl)propan-2-yl-amide]-3-(2-naphtyl)propan-2-yl-amide]semicarbazide ;
1-(2*S*-2-aminobutanoyl)-4-[2*S*-*N*-[2*S*-3-(*m-*fluorophényl)propan-2-yl-amide]-3-(1,1'-biphényl-4-yl)propan-2-yl-amide]semicarbazide ;
1-{2*S*-2-aminopropionyl}-4-(2-phényléthyl) semicarbazide ;
1-(2*S*-2-aminopropionyl)-4-(2-phényléthyl)-4-méthylsemicarbazide ;
1-(2*S*-2-aininopropionyl)-4-[2-(*p*-hydroxyphényl)éthyl]semicarbazide ;
1-(2*S*-2-aminopropionyl)-4-benzylsemicarbazide;
1-(2*S*-2-aminopropionyl)-4-(*o*-chlorobenzyl)semicarbazide ;
1-(2*S*-2-aminopropionyl)-4-(2-furfuryl)semicarbazide ;
1-(2*S*-2-aminopropionyl)-4-[2-(3-indolyl)éthyl]semicarbazide ;
1-(2*S*-2-aminopropionyl)-4-(1-naphlylméthyl)semicarbazide ;
1-(2*S*-2-aminopropionyl)-4-pipéronylsemicarbazide ;
1-(2*S*-2-aminopropionyl)-4-(*p*-chlorobenzyl)semicarbazide ;
1-(2*S*-2-atninopropionyl)-4-[2-(thiophén-2-yl)éthyl]semicarbazide ;
1-(2*S*-2-aminopropionyl)-4-(*m*-fluorobenzyl)semicarbazide ;
1-(2*S*-2-aminopropionyl)-4-(*o*-fluorobenzyl)semicarbazide ;
1-(2*S*-2-aminopropionyl)-4-(*p*-fluorobenzyl)semicarbazide ;
1-(2*S*-2-aminopropionyl)-4-(*o*-trifluorométhylbenzyl)semicarbazide ;
1-(2*S*-2-aminopropionyl)-4-(*m*-chlorobenzyl)semicarbazide ;
1-(2*S*-2-aminopropionyl)-4-(*o*-méthoxybenzyl)semicarbazide ;
1-(2*S*-2-aminopropionyl)-4-(*o*-méthylbenzyl)semicarbazide ;
1-(2*S*-2-aminopropionyl)-4-(alpha-méthylbenzyl)semicarbazide ;
1-(2*S*-2-aminobutanoyl)-4-(*o*-chlorobenzyl)semicarbazide;
1-(2*S*-2-aminovaléryl)-4-(*o*-chlorobenzyl)semicarbazide ;
1-(2*S*-2-aminohexanoyl)-4-(*o*-chlorobenzyl)semicarbazide ;
1-(2*S*-2-aminoisocaproyl)-4-(*o*-chlorobenzyl)semicarbazide ;
1-(1-aminocyclohexane-1-carbonyl)-4-(*o*-chlorobenzyl)semicarbazide ;
1-(2*S*-2-amino-3-phényl-propionyl)-4-(*o*-chlorobenzyl)semicarbazide ;
1,1-(2*S*-1 ,4-cyclo-aminobutanoyl)-4-(*o*-chlorobenzyl)semicarbazide ;
1,1-(2*R*-1,4-cyclo-aminobutanoyl)-4-(*o*-chlorobenzyl)semicarbazide ;
1-(2*S*-2-aminobutanoyl)-4-[2*S*-*N*-[2*S*-3-(*m*-fluorophényl)propan-2-yl-amide]-4-phénylbutan-2-yl-amide]semicarbazide ;
1-(2*S*-2-aminobutanoyl)-4-[*o*-(*o*-hydroxyméthylphénylthio)benzyl]semicarbazide ;
1-(2*S*-2-aminobutanoyl)-4-(*o*-bromobenzyl)semicarbazide ;
1-(2*S*-2-aminobutanoyl)-4-(2-chloro-6-fluorobenzyl)semicarbazide ;
1-(2*S*-2-aminobutanoyl)-4-(*o*-éthoxybenzyl)semicarbazide ;
1-(2*S*-2-aminobutanoyl)-4-([1,1'-biphényl-4-yl]méthyl)semicarbazide ;
1-(2*S*-2-aminobutanoyl)-4-([1,1'-biphényl-2-yl]méthyl)semicarbazide ;
1-(2*S*-2-aminobutanoyl)-4-(*p*-[thiophén-2-yl]benzyl)semicarbazide ;
1-(2*S*-2-aminobutanoyl)-4-(*o*-[*N*-morpholinyl]benzyl)semicarbazide ;
1-(2*S*-2-aminobutanoyl)-4-(1-naphtylméthyl)semicarbazide ;
1-(2*S*-2-aminobutanoyl)-4-(*o*-[*N*-pipéridinyl]benzyl)semicarbazide ; et
1-(2*S*-2-aminobutanoyl)-4-(*o*-méthylthiobenzyl)semicarbazide.

28. Composé selon la revendication 1 choisi dans le groupe consistant en
1-(2*R*-2-aminopropionyl)-4-(2*S*-3-phénylpropan-2-yl-amide) semicarbazide ;
1-(2*R*-2-aminopropionyl)-1-méthyl-4-(2*S*-3-phénylpropan-2-yl-amide) semicarbazide ;
1-(2*R*-2-aminobutanoyl)-4-[2*S-N* [2*S*-3-(indol-3-yl)propan-2-yl-amide]-3-phénylpropan-2-yl-amide]semicarbazide ;
1-(2*R*-2-aminopropanoyl)-4-[2*S-N*-[2*S-*3-(indol-3-yl)propan-2-yl-amide]-3-phénylpropan-2-yl-amide]semicarbazide ;
1-(2*R*-3-naphtyl-2-propanoyl)-4-[2S-*N*-[2*S*-3-(*m*-fluorophényl)propan-2-yl-amide]-3-méthylbutan-2-yl-amide]semicarbazide ;
1-[2*R*-2-amino-3-(4-pyridyl)propanoyl]-4-[2*S*-*N*-(2*S*-3-(indol-3-yl)propan-2-yl-amide)-butan-2-yl-amide]semicarbazide ;
1-[2*R*-2-amino-3-(4-pyridyl)propanoyl]-4-[2*S*-*N*-(2*S*-3-(indol-3-yl)propan-2-yl-amide)-2-phényléthan-2-yl-amide]semicarbazide ;
1-(2*R*-2-aminobutanoyl)-4-[2*S*-*N*-[2*S*-3-(indol-3-yl)propan-2-yl-amide]-4-phénylbutan-2-yl-amide]semicarbazide ;
1-(2*R*-2-aminobutanoyl)-4-[2*S*-*N*-[2*S*-3-(*m*-fluorophényl)propan-2-yl-amide]-3-(indol-3-yl)propan-2-yl-amide]semicarbazide ;
1-(2*R*-2-aminobutanoyl)-4-[2*S*-*N*-[2*S*-3-(*m*-méthoxyphényl)propan-2-yl-amide]-3-(*p-*chlorophényl)propan-2-yl-amide]semicarbazide ;
1-(2*R*-2-aminopentanoyl)-4-[2*S*-N-[2*S*-3-(*m*-méthoxyphényl)propan-2-yl-amide]-3-(*p*-chlorophényl)propan-2-yl-amide]semicarbazide ;
1-(2*R*-2-amino-4-phénylbutanoyl)-4-[2*S*-*N*-[2*S*-3-(1',1'-biphényl-4-yl)propan-2-yl-amide]-3-phénylpropan-2-yl-amide]semicarbazide ;
1-(2*R*-2-aminopentanoyl)-4-[2*S*-*N*-[2*S*-3-(*m*-méthoxyphényl)propan-2-yl-amide]-3-(1,1'-biphényl-4-yl)propan-2-yl-amide]semicarbazide ;
1-(2*R*-2-aminobutanoyl)-4-[2*S*-*N*-[2*S*-3-(1,1'-biphényl-4-yl)propan-2-yl-amide]-3-(*p-*hydroxyphényl)propan-2-yl-amide]semicarbazide ;
1-(2*R*-2-aminobutanoyl)-4-[2*S*-*N*-[2*S*-4-méthylpentan-2-yl-amide]-3-(benzo[b]thiophén-3-yl)propan-2-yl-amide]semicarbazide ;
1-(2*R*-2-aminobutanoyl)-4-[2*S*-*N*-[2*S*-3-(2-naphtyl)propan-2-yl-amide]-4-phénylbutan-2-yl-amide]semicarbazide ;
1-(2*R*-2-aminobutanoyl)-4-[2*S*-*N*-[2*S*-3-(*m*-méthoxyphényl)propan-2-yl-amide]-4-phénylbutan-2-yl-amide]semicarbazide ;
1-(2*R*-2-aminobutanoyl)-4-[2*S*-*N*-[2*S*-3-(benzo[b]thiophén-3-yl)propan-2-yl-amide]-4-phénylbutan-2-yl-amide]semicarbazide ;
1-(2*R*-2-aminobutanoyl)-4-[2*S*-*N*-[2*S*-3-(*p*-chlorophényl)propan-2-yl-amide]-4-phénylbutan-2-yl-amide]semicarbazide ;
1-(2*R*-2-aminopentanoyl)-4-[2*S*-*N*-[2*S*-3-(2-naphtyl)propan-2-yl-amide]-4-phénylbutan-2-yl-amide]semicarbazide ;
1-(2*R*-2-aminobutanoyl)-4-[2*S*-*N*-[2*S*-3-(*m*-fluorophényl)propan-2-yl-amide]-3-(2-naphtyl)propan-2-yl-amide]semicarbazide ;
1-(2*R*-2-aminobutanoyl)-4-[2*S*-*N*-[2*S*-3-(*m-*fluorophényl)propan-2-yl-amide]-3-(1,1'-biphényl-4-yl)propan-2-yl-amide]semicarbazide ;
1-(2*R*-2-aminopropionyl)-4-(2-phényléthyl)semicarbazide ;
1-(2*R*-2-aminopropionyl)-4-(2-phényléthyl)-4-méthylsemicarbazide ;
1-(2*R*-2-aminopropionyl)-4-[2-(*p*-hydroxyphényl)éthyl]semicarbazide ;
1-(2*R*-2-aminopropionyl)-4-benzylsemicarbazide ;
1-(2*R*-2-aminopropionyl)-4-(*o*-chlorobenzyl)semicarbazide ;
1-(2*R*-2-aminopropionyl)-4-(2-furfuryl)semicarbazide ;
1-(2*R*-2-aminopropionyl)-4-[2-(3-indolyl)éthyl]semicarbazide;
1-(2*R*-2-aminopropionyl)-4-(1-naphtylméthyl)semicarbazide ;
1-(2*R*-2-aminopropionyl)-4-pipéronylsemicarbazide ;
1-(2*R*-2-aminopropionyl)-4-(*p*-chlorobenzyl)semicarbazide ;
1-(2*R*-2-aminopropionyl)-4-[2-(thiophén-2-yl)éthyl]semicarbazide ;
1-(2*R*-2-aminopropionyl)-4-(*m-*fluorobenzyl)semicarbazide ;
1-(2*R*-2-aminopropionyl)-4-(*o*-fluorobenzyl)semicarbazide ;
1-(2*R*-2-aminopropionyl)-4-(*p*-fluorabenzyl)semicarbazide ;
1-(2*R*-2-aminopropionyl)-4-(*o*-trifluorométhylbenzyl)semicarbazide ;
1-(2*R*-2-aminopropionyl)-4-(*m*-chlorobenzyl)semicarbazide ;
1-(2*R*-2-aminopropionyl)-4-(*o*-méthoxybenzyl)semicarbazide ;
1-(2*R*-2-aminopropionyl)-4-(*o*-méthylbenzyl)semicarbazide ;
1-(2*R*-2-aminopropionyl)-4-(alpha-méthylbenzyl)semicarbazide ;
1-(2*R*-2-aminobutanoyl)-4-(*o*-chlorobenzyl)semicarbazide ;
1-(2*R*-2-aminovaléryl)-4-(*o*-chlorobenzyl)semicarbazide ;
1-(2*R*-2-aminohexanoyl)-4-(*o*-chlorobenzyl)semicarbazide ;
1-(2*R*-2-aminoisocaproyl)-4-(*o*-chlorobenzyl)semicarbazide ;
1-(2*R*-2-amino-3-phényl-propionyl)-4-(*o*-chlorobenzyl)semicarbazide ;
1-(2*R*-2-aminobutanoyl)-4-[2*S*-*N-*[2*S-*3-(*m*-fluorophényl)propan-2-yl-amide]-4-phénylbutan-2-yl-amide]semicarbazide ;
1-(2*R*-2-aminobutanoyl)-4-[*o*-(*o*-hydroxyméthylphénylthio)benzyl]semicarbazide ;
1-(2*R*-2-aminobutanoyl)-4-(*o*-bromobenzyl)semicarbazide ;
1-(2*R*-2-aminobutanoyl)-4-(2-chloro-6-fluorobenzyl)semicarbazide ;
1-(2*R*-2-aminobutanoyl)-4-(*o*-éthoxybenzyl)semicarbazide ;
1-(2*R*-2-aminobutanoyl)-4-([1,1'-biphényl-4-yl]méthyl)semicarbazide ;
1-(2*R*-2-aminobutanoyl)-4-([1,1'-biphényl-2-yl]méthyl)semicarbazide ;
1-(2*R*-2-aminobutanoyl)-4-(*p*-[thiophén-2-yl]benzyl)semicarbazide ;
1-(2*R*-2-aminobutanoyl)-4-(*o*-[*N*-morpholinyl]benzyl)semicarbazide ;
1-(2*R*-2-aminobutanoyl)-4-(1-naphtylméthyl)semicarbazide ;
1-(2*R*-2-aminobutanoyl)-4-(*o*-[*N*-pipéridinyl]benzyl)semicarbazide ; et
1-(2*R*-2-aminobutanoyl)-4-(*o*-méthylthiobenzyl)semicarbazide.

29. Composé selon l'une des revendications précédentes, qui, dans l'essai de DPP-I, présente une valeur d'IC₅₀ inférieure à 500 µM comme, par exemple, inférieure à 100 µM, inférieure à 50 µM, inférieure à 1 µM, inférieure à 500 nM, inférieure à 100 nM ou inférieure à 50 nM.

30. Composé selon l'une quelconque des revendications 1 à 29 pour une utilisation en tant qu'inhibiteur de protéase.

31. Composé selon la revendication 30 pour une utilisation en tant qu'inhibiteur de cystéine protéase.

32. Composé selon l'une des revendications 1 à 31 pour une utilisation dans le traitement, la prophylaxie et/ou le diagnostic d'une inflammation, d'un diabète de type 2, de l'asthme, d'une grippe sévère, d'une infection par le virus respiratoire syncytial, d'une inhibition de cellule T CD8, de maladies intestinales inflammatoires, d'un psioriasis, d'une dermatite atopique, du syndrome de Papillon Lefèvre, du syndrome de Haim Munk, de maladies périodontiques (de gomme), d'une périodontite, d'arthrite rhumatoïde, de la maladie de Huntington, de la maladie de Chagas, de la maladie d'Alzheimer, d'une speticémie, ou pour une application dans l'apoptose d'une cellule cible.

33. Composition pharmaceutique comprenant, en tant que substance active, un composé tel que défini dans l'une quelconque des revendications 1 à 32, ou un sel de celui-ci acceptable du point de vue pharmaceutique, en association avec un véhicule ou un diluant acceptable du point de vue pharmaceutique.

34. Composition pharmaceutique selon la revendication 33 sous forme de dosage unitaire, comprenant d'environ 1 µg à environ 1000 mg comme, par exemple, d'environ 10 µg à environ 500 mg, d'environ 0,05 à environ 100 mg ou d'environ 0,1 à environ 50 mg, de la substance active.

35. Composition pharmaceutique selon la revendication 33 ou 34 pour une administration par voie orale, nasale, transdermique, pulmonaire ou parentérale.

36. Utilisation d'un composé tel que défini selon l'une quelconque des revendications 1 à 32 pour la préparation d'un médicament.

37. Utilisation d'un composé de formule (I) 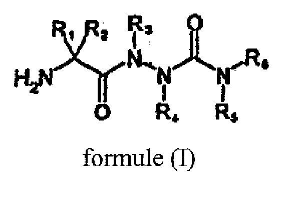 pour la préparation d'un médicament pour moduler les teneurs en DPP-I chez un sujet, ou pour le traitement, la prophylaxie et/ou le diagnostic d'une inflammation, d'un diabète de type 2, de l'asthme, d'une grippe sévère, d'une infection par le virus respiratoire syncytial, d'une inhibition de cellule T CD8, de maladies intestinales inflammatoires, d'un psioriasis, d'une dermatite atopique, du syndrome de Papillon Lefèvre, du syndrome de Haim Munk, de maladies périodontiques (de gomme), d'une périodontite, d'arthrite rhumatoïde, de la maladie de Huntington, de la maladie de Chagas, de la maladie d'Alzheimer, d'une speticémie, ou pour une application dans l'apoptose d'une cellule cible,
formule dans laquelle :
R₁ et R₅ sont indépendamment l'un de l'autre l'hydrogène, un alkyle en C₁ à C₆, un alcényle en C₂ à C₆, un alcynyle en C₂ à C₆, un groupe cycloalkyle en C₃ à C₁₀ substitué ou non substitué, un groupe cycloalcényle en C₅ à C₁₀ substitué ou non substitué, un groupe hétérocycloalkyle en C₃ à C₇ substitué ou non substitué, un groupe alkyl(C₁ à C₆)-aryle substitué ou non substitué mais pas benzyle lorsque R₆ est phényle, un groupe alkyl(C₁ à C₆)-hétéroaryle substitué ou non substitué, un groupe aryle substitué ou non substitué, un groupe hétéroaryle substitué ou non substitué, un groupe aryl-alkyle en C₁ à C₆ substitué ou non substitué, un groupe hétéroaryl-alkyle en C₁ à C₆ substitué ou non substitué, un groupe cycloalkyl(C₃ à C₇)-alkyle en C₁ à C₆ substitué ou non substitué, un groupe hétérocycloalkyl(C₃ à C₇)-alkyle en C₁ à C₆ substitué ou non substitué, ou R₁ et R₂ forment ensemble un groupe cycloalkyle en C₃ à C₁₀ substitué ou non substitué ou un groupe hétérocycloalkyle en C₃ à C₇ substitué ou non substitué ;
R₃ est l'hydrogène, un alkyle en C₁ à C₆, ou R₃ et R₁ forment ensemble un groupe cycloalkyle en C₃ à C₁₀ substitué ou non substitué ou un groupe hétérocycloalkyle en C₃ à C₇ substitué ou non substitué, ou R₃ et R₂ forment ensemble un groupe cycloalkyle en C₃ à C₁₀ substitué ou non substitué ou un groupe hétérocycloalkyle en C₃ à C₇ substitué ou non substitué ;
R₄ et R₅ sont indépendamment l'un de l'autre l'hydrogène ou un alkyle en C₁ à C₆ ;
R₆ est alkyle en C₁ à C₆, -CH(R₇)CONH(R₈), un alcényle en C₂ à C₆, un alcynyle en C₂ à C₆, un groupe cycloalkyle en C₃ à C₁₀ substitué ou non substitué, un groupe cycloalcényle en C₅ à C₁₀ substitué ou non substitué, un groupe hétérocycloalkyle en C₃ à C₇ substitué ou non substitué, un groupe cycloalkyl(C₃ à C₇)-alkyle en C₁ à C₆ substitué ou non substitué, un groupe hétérocycloalkyl(C₃ à C₇)-alkyle en C₁ à C₆ substitué ou non substitué, un groupe alkyl(C₁ à C₆)-aryle substitué ou non substitué, un groupe alkyl(C₁ à C₆)-hétéroaryle substitué ou non substitué, un groupe aryle substitué ou non substitué, un groupe hétéroaryle substitué ou non substitué, un groupe aryl-alkyle en C₁ à C₆ substitué ou non substitué, un groupe hétéroaryl-alkyle en C₁ à C₆ substitué ou non substitué ;
R₇ est l'hydrogène, un groupe alkyle en C₁ à C₆ substitué ou non substitué, un groupe cycloalkyle en C₃ à C₁₀ substitué ou non substitué, un groupe alkyl(C₁ à C₆)-aryle substitué ou non substitué, un groupe alkyl(C₁ à C₆)-hëtéroaryle substitué ou non substitué ou une chaîne latérale d'un résidu acide aminé naturel ou non naturel ;
R₈ est l'hydrogène, un groupe alkyle en C₁ à C₆ substitué ou non substitué, un groupe cycloalkyle en C₃ à C₁₀ substitué ou non substitué, un groupe alkyl(C₁ à C₆)-aryle substitué ou non substitué, un groupe alkyl(C₁à C₆)-hétéroaryle substitué ou non substitué, une chaîne latérale d'un résidu acide aminé naturel ou non naturel ou un groupe -CH(R₇)CONH(R₉) ;
R₉ est l'hydrogène, un groupe alkyle en C₁ à C₆ substitué ou non substitué, un groupe cycloalkyle en C₃ à C₁₀ substitué ou non substitué, un groupe alkyl(C₁ à C₆)-aryle substitué ou non substitué ou un groupe alkyl(C₁ à C₆)-hétéroaryle substitué ou non substitué ;
dans lesquels un groupe substitué est substitué par un, deux ou trois substituants choisis indépendamment dans le groupe consistant en un alkyle en C₁ à C₆, un alkylthio en C₁ à C₆, un alkylcarbonyle en C₁ à C₆, un N-alkylamide en C₁ à C₆, un alcoxy en C₁ à C₆, un dialkylamino-alkyle en C₁ à C₆, amide, hydroxy, carboxy, amino, halogène, trifluorométhyle, trifluorométhoxy, trifluorométhylthio et cyano.

38. Composé de formule (I) 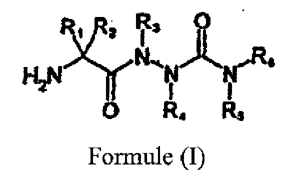 dans laquelle R₁, R₂, R₃, R₄, R₅ et R₆ sont tels que définis dans l'une quelconque des revendications 1 à 28,
avec la restriction que le composé n'est pas Ile-Gly^{a}-Leu-Met-NH₂ ou Tyr-AzAla-Gly-Phe-Leu, Gly^{a} étant un résidu α-aza-acide aminé.
